(19) Europäisches Patentamt
European Patent Office
Office européen des brevets

(11) EP 4 289 830 B1

(12) **EUROPEAN PATENT SPECIFICATION**

(45) Date of publication and mention of the grant of the patent:
**08.04.2026 Bulletin 2026/15**

(21) Application number: **22749988.6**

(22) Date of filing: **28.01.2022**

(51) International Patent Classification (IPC):
***C07D 277/82*** *(2006.01)* ***A61K 31/428*** *(2006.01)*
***A61P 29/00*** *(2006.01)* ***A61P 3/00*** *(2006.01)*
***A61P 25/00*** *(2006.01)* ***A61P 25/28*** *(2006.01)*
***A61P 37/00*** *(2006.01)* ***C07D 417/12*** *(2006.01)*
***A23L 33/10*** *(2016.01)*

(52) Cooperative Patent Classification (CPC):
**C07D 277/82; A23L 33/10; C07D 417/12**

(86) International application number:
**PCT/KR2022/001620**

(87) International publication number:
**WO 2022/169239 (11.08.2022 Gazette 2022/32)**

(54) **NLRP3 INFLAMMASOME SMALL-MOLECULE INHIBITOR FOR TREATMENT OF ALZHEIMER'S DISEASE**

NLRP3-INFLAMMASOM-KLEINMOLEKÜL-INHIBITOR ZUR BEHANDLUNG VON MORBUS ALZHEIMER

INHIBITEUR D'INFLAMMASOME NLRP3 À PETITES MOLÉCULES POUR LE TRAITEMENT DE LA MALADIE D'ALZHEIMER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.02.2021 KR 20210015425**

(43) Date of publication of application:
**13.12.2023 Bulletin 2023/50**

(73) Proprietor: **Ajou University Industry-Academic Cooperation Foundation**
**Gyeonggi-do 16499 (KR)**

(72) Inventors:
- **CHOI, Sangdun**
  **Suwon-si Gyeonggi-do 16493 (KR)**
- **HASEEB, Muhammad**
  **Suwon-si Gyeonggi-do 16499 (KR)**
- **NASIR, Javaid**
  **Suwon-si Gyeonggi-do 16499 (KR)**
- **FARZANA, Yasmeen**
  **Suwon-si Gyeonggi-do 16499 (KR)**
- **SEO, Jee Yeon**
  **Suwon-si Gyeonggi-do 16229 (KR)**
- **HEO, Jae Kyung**
  **Suwon-si Gyeonggi-do 16229 (KR)**
- **SHIN, Ho Chul**
  **Suwon-si Gyeonggi-do 16229 (KR)**

(74) Representative: **Mewburn Ellis LLP**
**Aurora Building**
**Counterslip**
**Bristol BS1 6BX (GB)**

(56) References cited:
**WO-A1-2020/249667 CN-A- 106 963 767**
**KR-A- 20170 109 678**

- **DATABASE REGISTRY 5 May 2008 (2008-05-05), ANONYMOUS : "Benzenesulfonamide, N-[5-[[[(3-acetylphenyl)amino]carbonyl] amino]-2- benzothiazolyl]-4-methyl- (CA INDEX NAME) ", XP055957501, retrieved from STN Database accession no. 1019134-09-8**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DATABASE REGISTRY 5 May 2008 (2008-05-05), ANONYMOUS : "Benzoic acid, 3-[[[[2-[[(4-methylphenyl)sulfonyl]amino]-5-benzothiazolyl]amino]carbonyl]amino]-, methyl ester (CA INDEX NAME)", XP055957505, retrieved from STN Database accession no. 1019134-96-3**
- **DATABASE REGISTRY 5 May 2008 (2008-05-05), ANONYMOUS: "Benzenesulfonamide, N-[5-[[[[(3-methoxyphenyl)methyl]amino]carbonyl]amino]-2-benzothiazolyl]-4-methyl- (CA INDEX NAME)", XP055957507, retrieved from STN Database accession no. 1019134-82-7**

## Description

### Technical Field

The present invention relates to a small-molecule compound having a function of inhibiting NLRP3 inflammasome activity, and more particularly, to a small-molecule compound represented by Formula 1, a composition for inhibiting NLRP3 inflammasome activity comprising the same, and a composition for preventing or treating NLRP3 inflammasome-related inflammatory diseases comprising the same.

### Background Art

Inflammasomes are multiprotein complexes of the innate immune system that detect pathogens via pattern recognition receptors, and are responsible for the activation of inflammatory responses (M. R. de Zoete, et al., Cold Spring Harb Perspect Biol 6, a016287 (2014)). Key components of the inflammasome are nucleotide-binding oligomerization domain (NOD)-like receptors (NLRs), absent in melanoma 2 (AIM2)-like receptors, pyrin receptors, and an enzymatic component (caspase 1). In addition to these components, most inflammasomes contain an adapter protein called an apoptosis-associated speck-like protein containing a caspase activation and recruitment domain (ASC) (H. Guo, et al., Nat Med 21, 677-687 (2015)). To date, several types of inflammasome such as NLR pyrin domain-containing (NLRP1, NLRP2, NLRP3, NLRP6, NLRP7, and NLRP12), NLR family CARD domain-containing protein 4 (NLRC4), and interferon-inducible protein 16 (IFI16) have been reported (M. Lamkanfi, V. M. Dixit, Cell 157, 1013-1022 (2014)).

NLRP3 (nucleotide-binding oligomerization domain [NOD]-like receptor family, pyrin domain-containing protein 3), which is the most widely studied type of inflammasome, is linked with several complex diseases of the central nervous system (K. Zhou, et al., J Immunol Res 2016, 9238290 (2016)). The key component of the NLRP3 scaffold consists of CARD (ASC) and precursor enzyme pro-caspase 1. Upon sensing danger stimuli, NLRP3 binds to ASC and interacts with cysteine protease caspase 1 to assemble the inflammasome. This results in caspase 1 activation and subsequently the maturation and secretion of proinflammatory cytokines IL-1β and IL-18 (K. Schroder, J. Tschopp, Cell 140, 821-832 (2010)). In addition, caspase 1 activation can mediate the cell death either directly (via a process known as pyroptosis) or indirectly (via apoptosis) (V. Sagulenko et al., Cell Death Differ 20, 1149-1160 (2013)). Besides the canonical NLRP3 inflammasome, the noncanonical NLRP3 inflammasome is also a developing area of interest. The latter type is formed by caspase 11 in mice. Nevertheless, humans do not express caspase 11, and recent findings have shown that orthologs thereof in human cells are caspase 4 and caspase 5, which play a similar role (J. Shi et al., Nature 514, 187-192 (2014)). NLRP3 inflammasome activation may be implemented through several cellular mechanisms and cytoskeletal signatures. A recent study has shown that NIMA-related kinase 7 (NEK7) is a novel regulator of the NLRP3 inflammasome, a protein involved in cell cycle progression. Upon interaction of NEK7 with the leucine-rich repeat (LRR) domain of NLRP3, it activates the NLRP3 inflammasome independently of its kinase activity (Y. He, et al., Nature 530, 354-357 (2016)). Recent developments have greatly improved our knowledge of the molecular mechanisms by which various inflammasomes are activated and their involvement in the initiation or progression of diseases. In addition, NLRP3 affects several autoimmune and autoinflammatory diseases, including metabolic disorders (obesity, gout, type 2 diabetes mellitus, and atherosclerosis), and neurodegenerative diseases (Alzheimer's disease (AD), traumatic brain injury, multiple sclerosis, and Parkinson's disease) (T. Strowig, et al., Nature 481, 278-286 (2012)).

The involvement of the NLRP3 inflammasome in the development of AD has been demonstrated in transgenic (APP/PS1) mice that show chronic amyloid-β (Aβ) deposition with caspase 1 and NLRP3 deficiency. These mice are characterized by decreased Aβ secretion, cognitive dysfunction, and neuroinflammation. The expression of caspase 1 has also been reported in the brain of patients with Alzheimer's disease, indicating a correlation between inflammasome activation and Alzheimer's disease in humans (M. T. Heneka et al., Nature 493, 674-678 (2013)). Thus, these studies have shown that the NLRP3 inflammasome is a potential therapeutic target for the treatment of neuroinflammation and AD.

Several biologic therapeutics, including the antibodies canakinumab, anakinra, and rilonacept, target the inflammasome products IL-1β, IL-1RA, and IL-18 for the treatment of NLRP3-related diseases (C. A. Dinarello, J. W. van der Meer, Semin Immunol 25, 469-484 (2013)). Several small-molecule inhibitors of the NLRP3 inflammasome, including glyburide, parthenolide, Bay11-708, auranofin, CRID3 and β-hydroxybutyrate (BHB) have been reported, but some of these inhibitors are non-specific and have limited potency (E. Isakov, P. Weisman-Shomer, M. Benhar, Biochim Biophys Acta 1840, 3153-3161 (2014)). Furthermore, MCC950 has recently been identified as a highly selective NLRP3 inflammasome inhibitor that blocks canonical and noncanonical NLRP3 inflammasomes at nanomolar concentrations (R. C. Coll et al., Nat Med 21, 248-255 (2015)). In another study, a small-molecule NLRP3 inhibitor, JC-124, was identified and tested for AD-related deficiency in transgenic mice (TgCRND8) (J. Yin et al., Mol Neurobiol 55, 1977-1987 (2018)). Overall, these data suggest the development of NLRP3 inflammasome inhibitors as potential treatments for AD.

Accordingly, the present inventors have made extensive efforts to develop a novel NLRP3 inflammasome inhibitor, and as a result, have identified a small-molecule agent referred to as NLRP3 inhibitory compound 7 (NIC7) and its

potent derivative NIC7w. In addition, the present inventors have found through *in vitro* experiments that both NIC7 and NIC7w inhibit the NLRP3 inflammasome and prevent the expression of caspase 1 and IL-1β, and that, in mouse models of AD, NIC7 exhibited significantly improved *in vivo* activity and significant improvement in mouse cognitive behavior, thereby completing the present invention.

WO 2020/249667 A1 (Nodthera Ltd. [GB]), published 17 December 2020, discloses sulfonylurea derivatives and their uses. CN 106963767 A (The Second Affiliated Hospital of PLA Third Military Medical University), published 21 July 2017, discloses UQCRC1 activators.

The information described in the Background Art is only for improving understanding of the background of the present invention, and it is not to be construed as including information forming the related art already known to those of ordinary skill in the art to which the present invention belongs.

## Summary of the Invention

An object of the present invention is to provide a small-molecule compound having a function of inhibiting NLRP3 inflammasome activity represented by any one formula selected from the group consisting of the following Formulas 1-1 to 1-12.

Another object of the present invention is to provide a compound represented by the following Formula 1, or a pharmaceutically acceptable salt thereof, for use in a method of inhibiting NLRP3 inflammasome activity.

Still another object of the present invention is to provide a composition for use in a method of preventing or treating NLRP3 inflammasome-related inflammatory disease comprising the small-molecule compound represented by the following Formula 1, or a pharmaceutically acceptable salt thereof.

Disclosed herein, but not claimed, is a method for preventing or treating NLRP3 inflammasome-related inflammatory disease comprising administering the small-molecule compound.

Disclosed herein, but not as a claimed solution, is use of the small-molecule compound for preventing or treating NLRP3 inflammasome-related inflammatory disease.

Disclosed herein, but not as a claimed solution, is the use of the small-molecule compound for manufacture of a medicament for preventing or treating NLRP3 inflammasome-related inflammatory disease.

To achieve the above objects, the present invention provides a compound represented by any one formula selected from the group consisting of the following Formulas 1-1 to 1-12, or a pharmaceutically acceptable salt thereof:

[Formula 1-1]

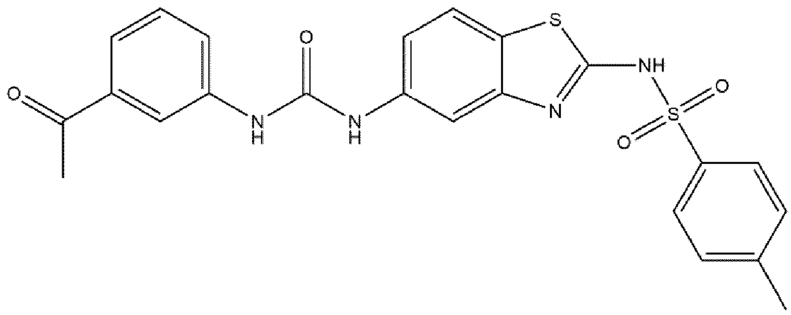

;

[Formula 1-2]

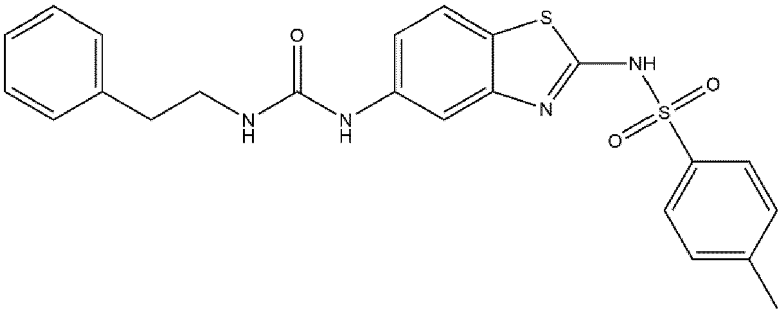

;

[Formula 1-3]

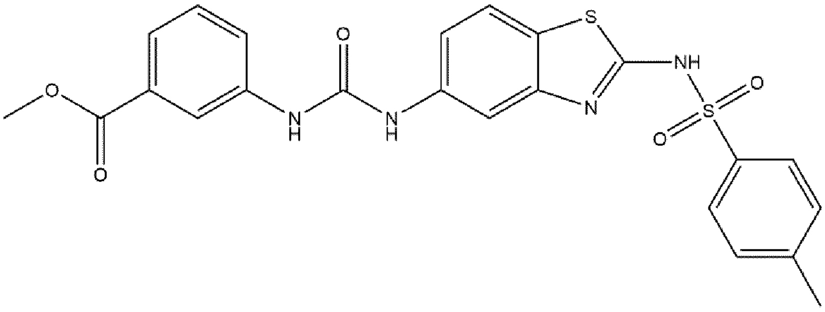

;

[Formula 1-4]

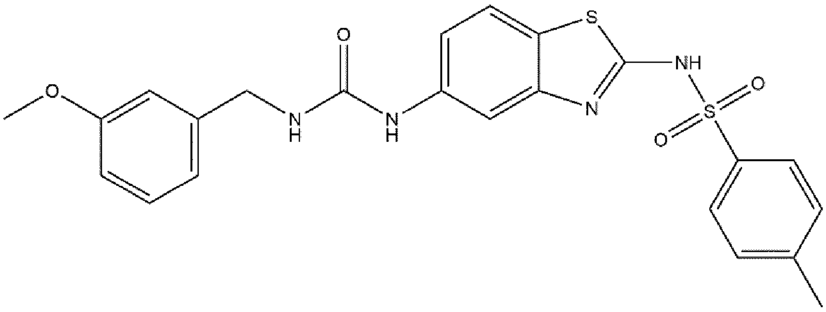

;

[Formula 1-5]

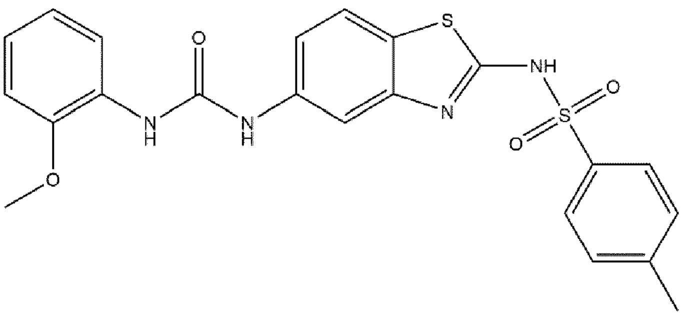

;

[Formula 1-6]

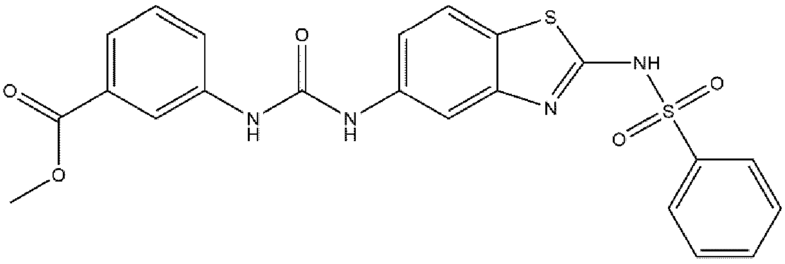

;

[Formula 1-7]

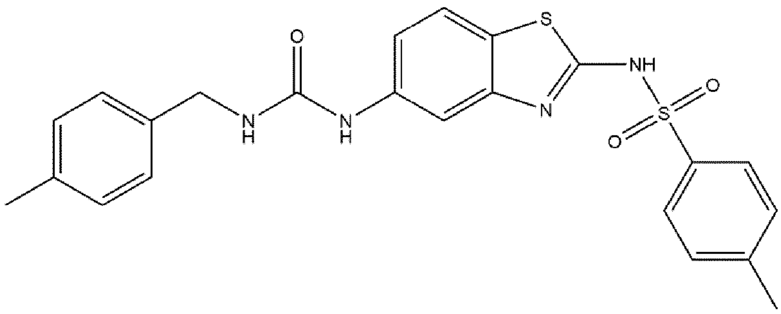

;

[Formula 1-8]

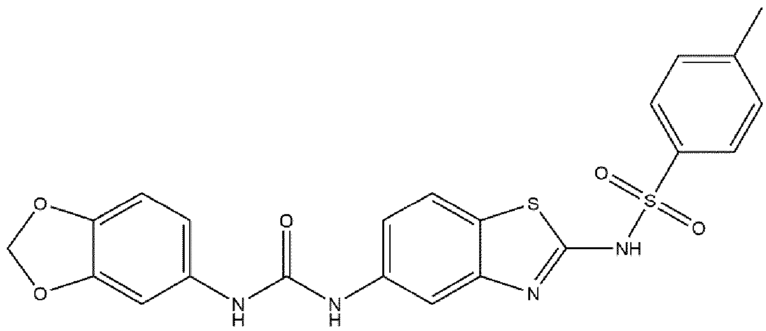

;

[Formula 1-9]

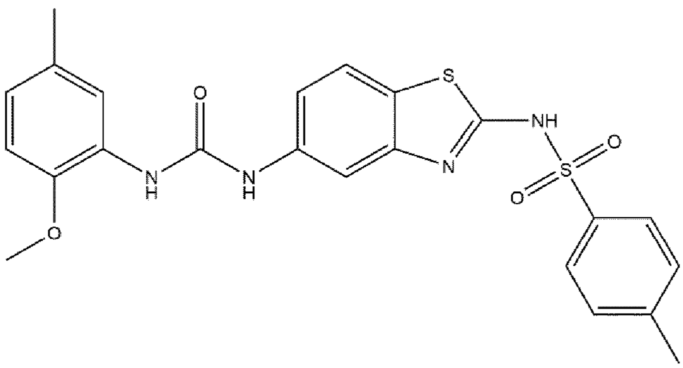

;

[Formula 1-10]

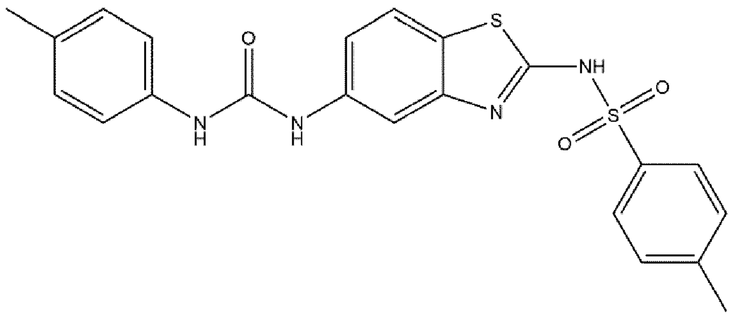

;

[Formula 1-11]

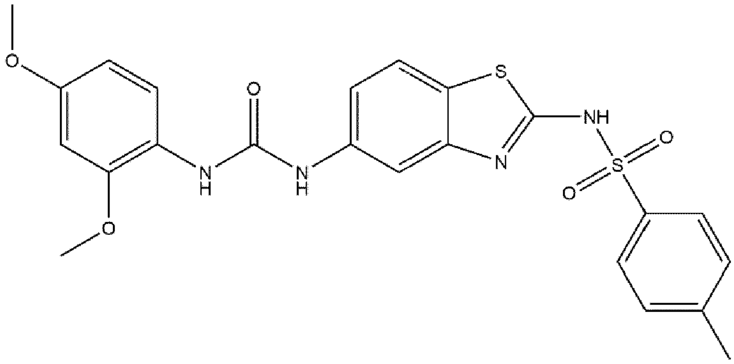

;

and

[Formula 1-12]

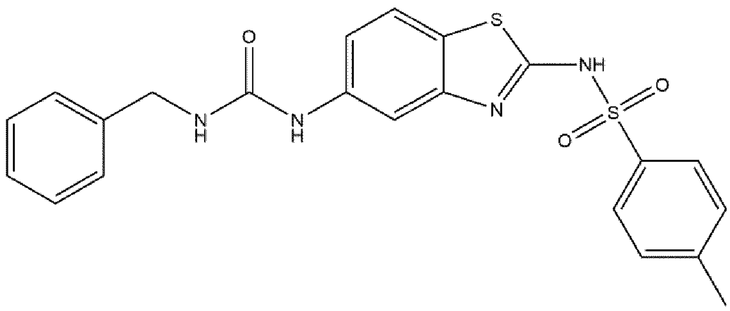

.

The present invention also provides a compound represented by the following Formula 1, or a pharmaceutically acceptable salt thereof, for use in a method of inhibiting NLRP3 inflammasome activity:

[Formula 1]

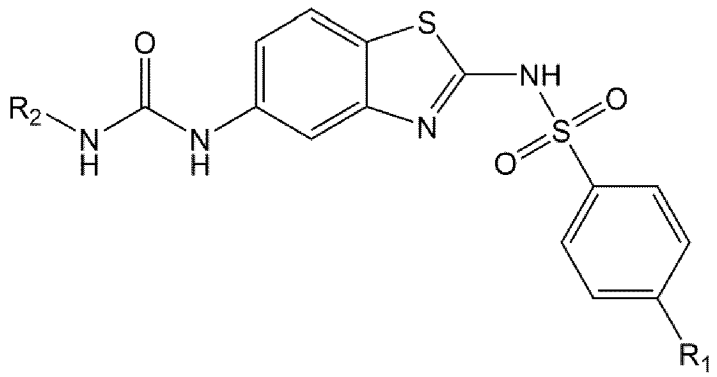

wherein $R_1$ and $R_2$ are the same as or different from each other and each independently selected from the group consisting of a hydrogen atom, a halogen atom, a substituted or unsubstituted hydrocarbon group, a substituted or unsubstituted heterogeneous group, a substituted or unsubstituted carbocyclic group, a substituted or unsubstituted heterocyclic group, a substituted or unsubstituted aromatic group, and a substituted or unsubstituted heteroaromatic group.

The present invention also provides a composition for use in a method of preventing or treating NLRP3 inflammasome-related inflammatory disease, the composition comprising a compound represented by the following Formula 1, or a pharmaceutically acceptable salt thereof:

[Formula 1]

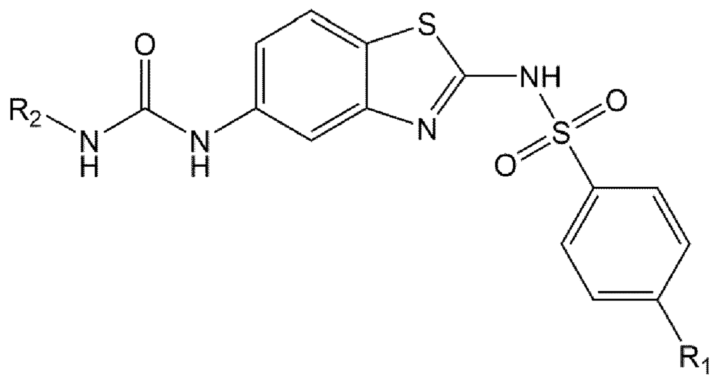

wherein $R_1$ and $R_2$ are the same as or different from each other and each independently selected from the group consisting of a hydrogen atom, a halogen atom, a substituted or unsubstituted hydrocarbon group, a substituted or unsubstituted heterogeneous group, a substituted or unsubstituted carbocyclic group, a substituted or unsubstituted heterocyclic group, a substituted or unsubstituted aromatic group, and a substituted or unsubstituted heteroaromatic group.

.

**Brief Description of Drawings**

FIG. 1 is an overall summary of the virtual screening workflow for the identification of primary leads. The number in each box indicates the number of ligands carried forward to the next level. The subsequent pharmacophore screening ligands were inputs for the independent rigid and induced-fit dockings.

FIG. 2 shows two sites of NLRP3 considered for molecular docking. FIG. 2(a) shows site I (HD2 domain and LRR) of NLRP3, corresponding to the NEK7-binding site of NLRP3, and FIG. 2(b) shows site II, corresponding to the NACHT domain and ADP-binding site on NLRP3. LRR is colored in cyan, HD2 in brown, HD1 in green, winged helix domain (WHD) in purple, and NBD in blue. A detailed view of the binding site is given on the right in each panel.

FIG. 3 shows the functional screening of NLRP3 inhibitors. FIGS. 3a and 3b are graphs showing the results of evaluating the toxicity of each compound to PMA-differentiated THP-1 cells by MTT assay after 24 hours of treatment with each compound at the indicated concentrations. FIG. 3(c) is a graph showing the results of evaluating the inhibition of NLRP3 by each non-toxic compound by measuring the secretion level of IL-1β. PMA-differentiated THP-1

cells were primed with LPS (10 ng/mL) for 4 hours, and then treated with each compound at the indicated concentration for 1 hour. NLRP3 was activated by treating the cells for 1 hour with nigericin (10 μM), and the supernatant was collected to check the cytokine level, using ELISA. The presented data are the average of three independent experiments where each experiment was conducted in duplicate, and statistical analysis was performed using a two-tailed paired Student's t-test (*P < 0.05).

FIG. 4 shows the cytotoxicity of NIC7 derivatives. FIGS. 4a and 4b are graphs showing the results of evaluating the toxicity of each compound to PMA-differentiated THP-1 cells by MTT assay after the treatment for 24 hours with each derivative at the indicated concentrations. The presented data are the average of three independent experiments where each experiment was conducted in duplicate, and statistical analysis was performed using a two-tailed paired Student's *t*-test (*P < 0.05).

FIG. 5 shows functional screening of NIC7 derivatives.

FIGS. 5a to 5c show the results of evaluating the inhibition of NLRP3 by each non-toxic compound by measuring the secretion level of IL-1β. PMA-differentiated THP-1 cells were primed with LPS (10 ng/mL) for 4 hours, and then treated with each compound for 1 hour at the indicated concentrations. NLRP3 was activated by treating the cells for 1 hour with nigericin (10 μM), and the supernatant was collected to check the cytokine level, using an ELISA. The presented data are the average of three independent experiments where each experiment was conducted in duplicate, and statistical analysis was performed using a two-tailed paired Student's *t*-test (**P* < 0.05). FIG. 5(d) shows the results of Western blotting performed to evaluate the inhibition of the NLRP3 signaling pathway by NIC7w (10 μM), NIC7 (10 μM) or MCC950 (1 μM). PMA-differentiated THP-1 cells were treated as described above, total protein was extracted therefrom, and immunoblotting was performed with primary antibodies specific for IL-1β, caspase 1, NLRP3, and β-actin. β-actin served as an internal control.

FIG. 6 shows recovery from cognitive impairment in the AD model. The AD model was generated by injecting A$\beta_{1-42}$ (black arrow) on day 7, and NIC7 (100 nmol/g), donepezil (1 mg/kg), or DMSO as vehicle (1%) was intraperitoneally administered on day 0 (red arrow) for 3 days/week.

In FIGS. 6a and 6b, spatial working memory was evaluated in terms of spontaneous alternation rate (FIG. 6a) and total arm entries (FIG. 6b) by the Y-maze test in each group (n = 6). FIG. 6(c) shows recognition memory, evaluated using the novel object recognition test, and data are presented as the percentage recognition rate for each group. FIG. 6(d) shows the body weight in each group, evaluated at the indicated time points. Statistical analysis was performed using a two-tailed paired Student's *t*-test (**P* < 0.05).

FIG. 7 shows two-dimensional structures of initial active ligands identified in cell-based assays. NIC7 was identified as a preliminary hit with an NLRP3-inhibitory ability.

FIG. 8 shows two-dimensional structures of active derivatives of NIC7 identified in cell-based assays. Among all the derivatives, NIC7w was found to be the most potent NLRP3 inhibitor.

FIG. 9 shows the interaction of NIC7 or NIC7w with NLRP3.

FIG. 9(a) shows the binding mode of NIC7 in HD2 and LRR of NLRP3, and a magnified view of the detailed interactions with residues 5 Å around of the ligand is shown on the right. FIG. 9(b) shows the binding of NIC7w with HD2 and LRR of NLRP3, and the right side shows a zoomed view of the detailed interactions occurring within 5 Å of the ligand. LRR is colored in cyan, HD2 in brown, HD1 in green, WHD in purple, and NBD in blue, and the ligands are shown as stick models. Dashed lines represent hydrogen bonds, and numbers represent distances in Angstroms (Å).

## Detailed Description and Preferred Embodiments of the Invention

Unless otherwise defined, all technical and scientific terms used in the present specification have the same meanings as commonly understood by those skilled in the art to which the present disclosure pertains. In general, the nomenclature used in the present specification is well known and commonly used in the art.

In the present disclosure, the present inventors provide both *in vitro* and *in vivo* evidence that a small-molecule inhibitor (NIC7) of NLRP3 inflammasome significantly improves mouse cognitive behavior in the AD model. NLRP3 inflammasome activation and subsequent secretion of caspase 1 and IL-1β were also attenuated by NIC7 administration.

The present inventors aimed to identify specific small-molecule inhibitors of the NLRP3 inflammasome using a large multiconformational chemical library through *in silico* virtual screening. Through experimental validation of a set of top-scoring virtual hits in cell-based bioassay, it was confirmed that a compound named NIC7 induced significant inhibition of NLRP3 inflammasome, caspase 1 and IL-1β. Subsequent evaluation of structural derivatives having modifications around the major scaffold of NIC7 revealed that an analog (named NIC7w) was able to inhibit the secretion of NLRP3 inflammasomes, caspase 1 and IL-1β in a dose-dependent manner, more strongly and significantly than NIC7.

Recently, several pharmacological agents such as MCC950, VX-765, JC-124 and PLX3397 have been developed that directly or indirectly target the NLRP3 inflammasome (R. Kuwar et al., J Neuroinflammation 16, 81 (2019)). Nonetheless, certain inhibitors are limited by the inability to use the crystal structure of NLRP3 bound to a small-molecule modulator. Computational drug discovery principles rely heavily on accurate determination of binding sites for

receptors, which makes interpretation of docking results difficult. Alternative approaches such as homology modeling or quantitative structure-activity relationship modeling exist, but the accuracy of these approaches is still debated (G. Sliwoski, et al., Pharmacol Rev 66, 334-395 (2014)).

In an Example of the present invention, the cryo-EM structure of NLRP3 was used because the crystal structure of human NLRP3 has not yet been solved. For structure-based virtual screening, the present inventors defined two distinct ligand-binding sites: site I (corresponding to HD2 and LRR of NLRP3 and overlapping the NEK7-binding site), and site II (corresponding to the NACHT domain of NLRP3). For reference, both the initial hit NIC7 and its potent analogue NIC7w belong to a docking round in which site I is defined as a binding pocket. In the cryo-EM structure, the HD2 domain and its surrounding residues have been reported as important binding regions in NLRP3 (H. Sharif et al., Nature 570, 338-343 (2019)). Based on these facts, the binding of NIC7 or NIC7w to site I of NLRP3 is very reasonable.

In an Example of the present invention, a specific inhibitor (NIC7) of NLRP3 inflammasome and a potent analog (NIC7w) thereof were identified via a computational drug discovery approach. *In vitro* experiments showed that both NIC7 and NIC7w prevent the expression of caspase 1 and IL-1β. In addition, the present inventors tested the *in vivo* activity of NIC7 in the AD model, examined the spatial working memory of mice in the Y-maze test, performed a novel object recognition test, and analyzed body weight. The present inventors confirmed metabolic stability (and cytochrome P450 inhibition) of NIC7 and NIC7w, indicating that they exhibit better half-life and stability than a positive control compound.

Overall, the results demonstrate the ability of NIC7 to improve mouse cognitive behavior in the AD model. This suggests that NIC7 and NIC7w may be used as promising drug candidates for future clinical trials.

In one aspect, the present invention is directed to a novel compound represented by any one formula selected from the group consisting of the following Formulas 1-1 to 1-12, or a pharmaceutically acceptable salt thereof:

[Formula 1-1]

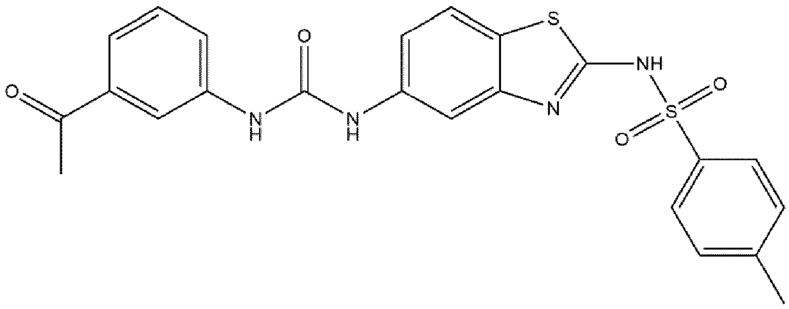

;

[Formula 1-2]

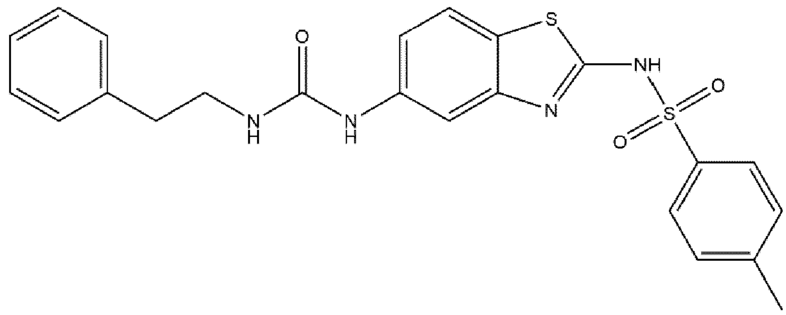

;

[Formula 1-3]

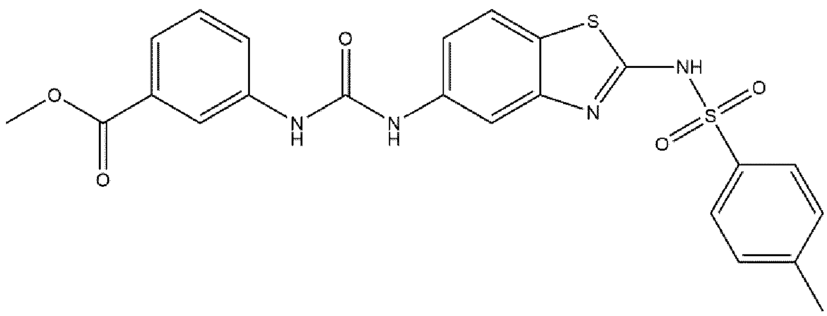

;

[Formula 1-4]

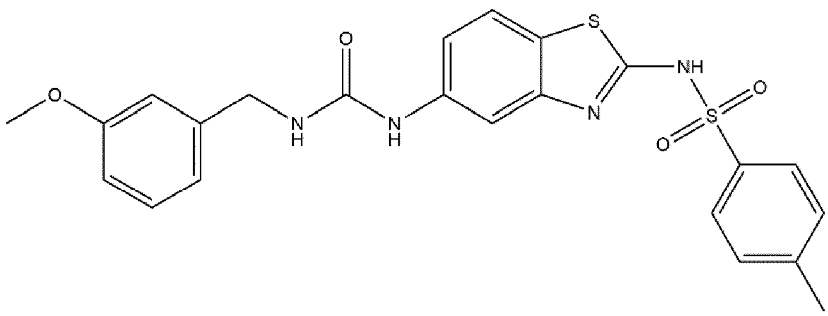

;

[Formula 1-5]

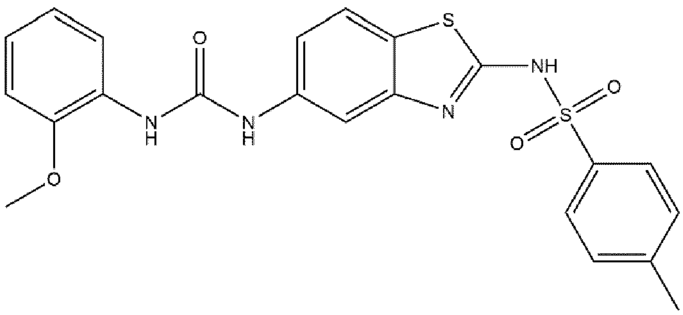

;

[Formula 1-6]

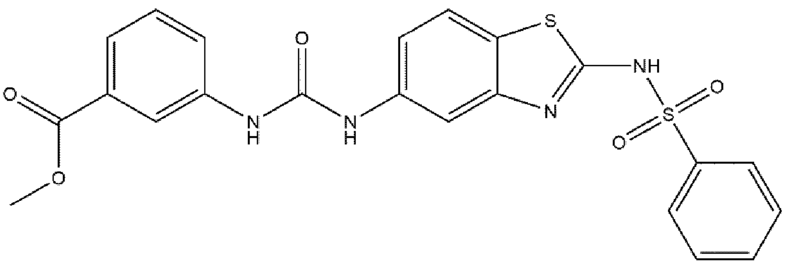

;

[Formula 1-7]

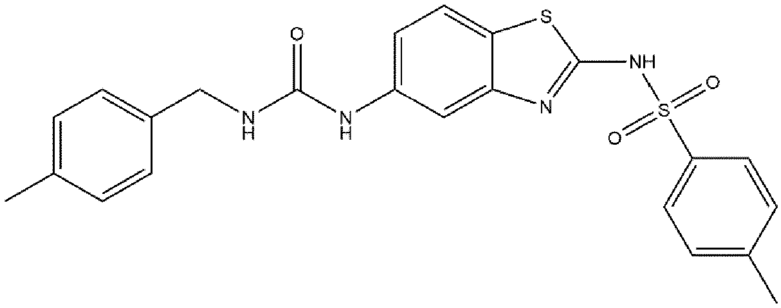

;

[Formula 1-8]

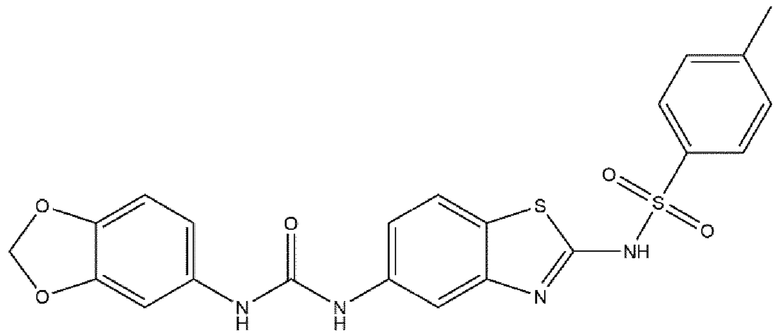

;

[Formula 1-9]

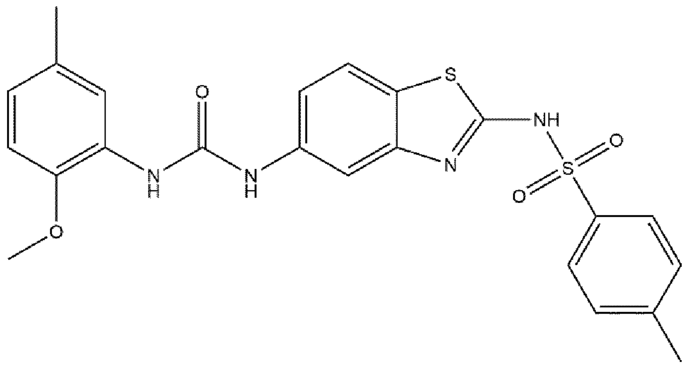

;

[Formula 1-10]

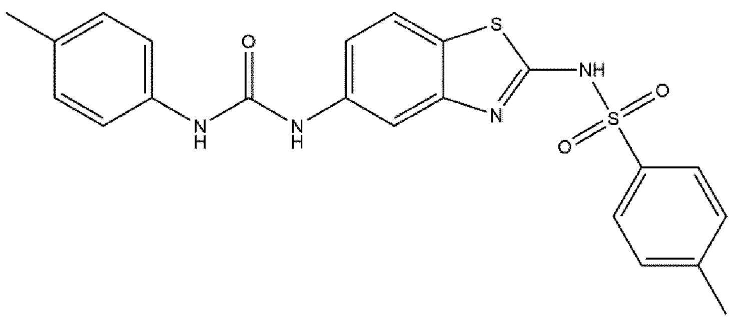

;

[Formula 1-11]

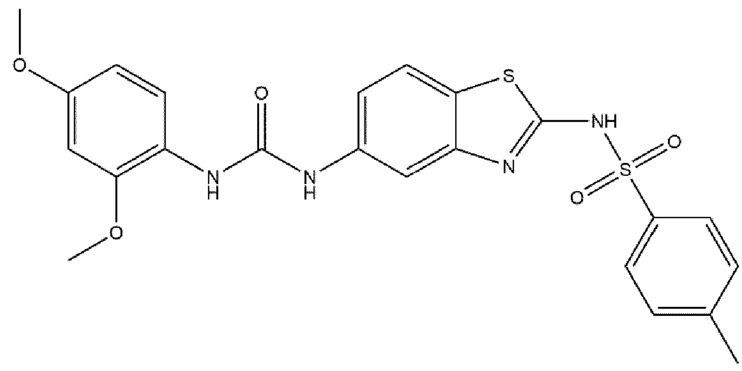

;

and

[Formula 1-12]

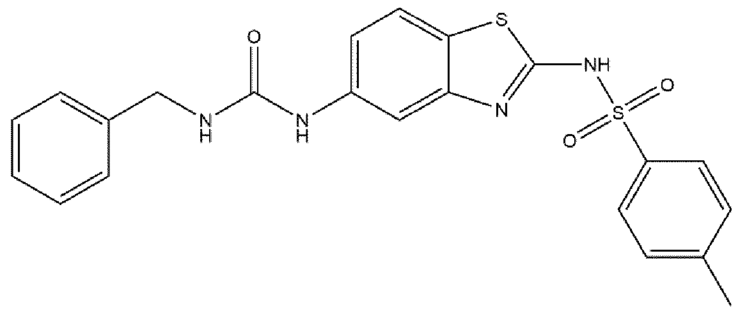

.

In another aspect, the present invention is directed to a compound for use in a method of inhibiting NLRP3 inflammasome activity, the compound represented by the following Formula 1, or a pharmaceutically acceptable salt thereof:

[Formula 1]

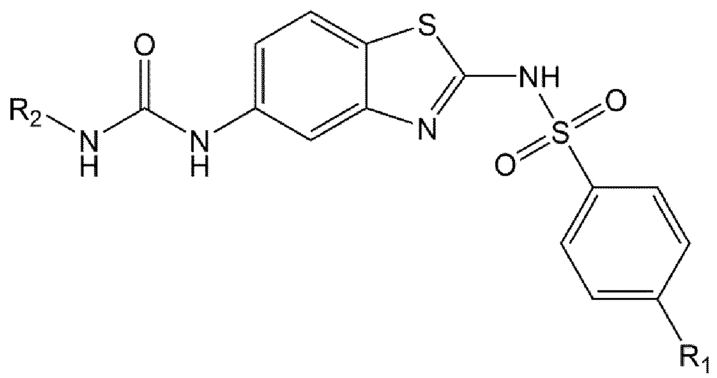

wherein $R_1$ and $R_2$ are the same as or different from each other and each independently selected from the group consisting of a hydrogen atom, a halogen atom, a substituted or unsubstituted hydrocarbon group, a substituted or unsubstituted heterogeneous group, a substituted or unsubstituted carbocyclic group, a substituted or unsubstituted heterocyclic group, a substituted or unsubstituted aromatic group, and a substituted or unsubstituted heteroaromatic group.

The compound of the present disclosure may exist as a tautomer, and both tautomeric forms are within the scope of the present invention even if only one thereof can be described herein.

In general, a reference to a particular element, such as hydrogen or H, is meant to include all isotopes of that element, where appropriate.

Specific examples of the term "halogen (or halo)" include fluorine (F), chlorine (Cl), bromine (Br), and iodine (I).

As used herein, the term "hydrocarbon group" refers to a chain having 1 to 25 carbon atoms, preferably 1 to 12 carbon atoms, more preferably 1 to 10 carbon atoms, most preferably 1 to 8 carbon atoms. The hydrocarbon group may have a linear or branched chain structure. Typical hydrocarbon groups have one or two branches, typically one branch. Typically, hydrocarbon groups are saturated. Unsaturated hydrocarbon groups may have one or more double bonds, one or more triple bonds, or combinations thereof. Typical unsaturated hydrocarbon groups have one or two double bonds or one triple bond; more typically unsaturated hydrocarbon groups have one double bond.

When the term "unsaturated" is used in conjunction with any group, the group may be fully unsaturated or partially unsaturated. However, when the term "unsaturated" is used in conjunction with a specific group defined herein, the term maintains the limitations of that specific group. For example, an unsaturated "carbocyclic group", based on the limitations of the "carbocyclic group" as defined herein, does not encompass an aromatic group.

Where the term "alkyl group" is used, either alone or within other terms such as "haloalkyl group" and "alkylamino group", it encompasses linear or branched carbon radicals having, for example, one to twenty carbon atoms or, in specific embodiments, one to twelve carbon atoms. Examples of such groups include, but are not limited to, methyl, ethyl, n-propyl, isopropyl, n-butyl, isobutyl, sec-butyl, tert-butyl, pentyl, iso-amyl, hexyl, and the like.

The term "haloalkyl group" encompasses groups wherein any one or more of the alkyl carbon atoms is substituted with halo as defined above. Specifically encompassed are monohaloalkyl, dihaloalkyl and polyhaloalkyl groups including perhaloalkyl. A monohaloalkyl group, for one example, may have either an iodo, bromo, chloro or fluoro atom within the group. Dihalo and polyhaloalkyl groups may have two or more of the same halo atoms or a combination of different halo groups. Examples of haloalkyl groups include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl.

The term "hydroxyalkyl group" encompasses linear or branched alkyl groups having, for example and without being limited to, one to ten carbon atoms, any one of which may be substituted with one or more hydroxyl groups. Examples of such groups include hydroxymethyl, hydroxyethyl, hydroxypropyl, hydroxybutyl and hydroxyhexyl.

The term "alkoxy group" encompasses linear or branched oxy-containing groups each having alkyl portions of, for example and without being limited to, one to ten carbon atoms. Examples of such groups include methoxy, ethoxy, propoxy, butoxy and tert-butoxy. In certain embodiments, lower alkoxy groups have one to three carbon atoms. The "alkoxy" groups may be further substituted with one or more halo atoms, such as fluoro, chloro or bromo, to provide "haloalkoxy" groups. Examples of such groups include fluoromethoxy, chloromethoxy, trifluoromethoxy, trifluoroethoxy, fluoroethoxy, and fluoropropoxy.

The term "aralkyl group" encompasses aryl-substituted alkyl groups. In one embodiment, the aralkyl groups may be "lower aralkyl" groups having aryl groups attached to alkyl groups having one to six carbon atoms, and examples of such groups include benzyl, diphenylmethyl and phenylethyl. The aryl in said aralkyl may be additionally substituted with halo, alkyl, alkoxy, haloalkyl and haloalkoxy.

The term "heterogeneous group" means a saturated or unsaturated chain of non-hydrogen member atoms comprising carbon atoms and at least one heteroatom. Heterogeneous groups typically have 1 to 25 member atoms. Preferably, the chain contains 1 to 12 member atoms, 1 to 10 member atoms, more preferably 1 to 8 member atoms. The chain may be linear or branched. Typical branched heterogeneous groups have one or two branches, more typically one branch. Typically, heterogeneous groups are saturated. Unsaturated heterogeneous groups may have one or more double bonds, one or more triple bonds, or both. Typical unsaturated heterogeneous groups have one or two double bonds or one triple bond.

The term "carbocyclic group" means a saturated or unsaturated carbocyclic hydrocarbon ring. Carbocyclic groups are not aromatic. Carbocyclic groups are monocyclic or polycyclic. Polycyclic carbocyclic groups can be fused, spiro, or bridged ring systems. Monocyclic carbocyclic groups may contain 4 to 10 carbon atoms, typically 4 to 7 carbon atoms, more typically 5 to 6 carbon atoms in the ring. Bicyclic carbocyclic groups may contain 8 to 12 carbon atoms, typically 9 to 10 carbon atoms in the rings.

The term "heterocyclic group" means a saturated or unsaturated ring structure containing carbon atoms and 1 or more heteroatoms in the ring. Heterocyclic groups are not aromatic. Heterocyclic groups are monocyclic or polycyclic. Polycyclic heterocyclic groups can be fused, spiro, or bridged ring systems. Monocyclic heterocyclic groups may contain 4 to 10 member atoms (i.e., including both carbon atoms and at least 1 heteroatom), typically 4 to 7 member atoms, more typically 5 to 6 member atoms in the ring. Bicyclic heterocyclic groups may contain 8 to 18 member atoms, typically 9 or 10 member atoms in the rings. Examples of heterocyclic groups include, but are not limited to, those wherein heterocyclyl is azetidinyl, pyrrolidinyl, tetrahydrofuranyl, tetrahydro-thienyl, pyrazolidinyl, imidazolidinyl, oxazolidinyl, isoxazolidinyl,

thiazolidinyl, piperidinyl, tetrahydropyranyl, tetrahydrothiopyranyl, piperazinyl, morpholinyl, thiomorpholinyl, 1,1-dioxothiomorpholin-4-yl, azepanyl, diazepanyl, homopiperazinyl, oxazepanyl, dihydroindolyl, dihydrofuryl, dihydroimidazolinyl, dihydrooxazolyl, tetrahydropyridinyl, dihydropyranyl, dihydrobenzofuranyl, benzodioxolyl, or benzodioxanyl.

The term "aromatic group" or "aryl group" means an aromatic group having one or more rings wherein such rings may be attached together in a pendent manner or may be fused. In one embodiment, an aromatic group contains one, two or three rings. Monocyclic aromatic groups may contain 4 to 10 carbon atoms, typically 4 to 7 carbon atoms, more typically 4 to 6 carbon atoms in the ring. Typical polycyclic aromatic groups have two or three rings. Polycyclic aromatic groups having two rings typically have 8 to 12 carbon atoms, preferably 8 to 10 carbon atoms in the rings. Examples of aromatic groups include, but are not limited to, phenyl, naphthyl, tetrahydronaphthyl, indanyl, biphenyl, phenanthryl, anthryl or acenaphthyl.

The term "heteroatom" means an atom other than carbon. Typically, heteroatoms are selected from the group consisting of sulfur, phosphorous, nitrogen and oxygen atoms. Groups containing more than one heteroatom may contain different heteroatoms.

The term "heteroaromatic group" or "heteroaryl group" means an aromatic group having one or more rings wherein such rings may be attached together in a pendent manner or may be fused, wherein the aromatic group has at least one heteroatom. Monocyclic heteroaromatic groups may contain 4 to 10 member atoms, typically 4 to 7 member atoms, more typically 4 to 6 member atoms in the ring. Typical polycyclic heteroaromatic groups have two or three rings. Polycyclic aromatic groups having two rings typically have 8 to 12 member atoms, more typically 8 to 10 member atoms in the rings. Examples of monocyclic heteroaryl include, but are not limited to, thiazolyl, oxazolyl, thiophenyl, furanyl, pyrrolyl, imidazolyl, isoxazolyl, isothiazolyl, pyrazolyl, triazolyl, triazinyl, thiadiazolyl, tetrazolyl, oxadiazolyl, pyridinyl, pyridazinyl, pyrimidinyl, pyrazinyl, and groups similar thereto. Examples of bicyclic heteroaryl include, but are not limited to, indolyl, azaindolyl, indolinyl, benzothiophenyl, benzofuranyl, benzimidazolyl, benzooxazolyl, benzisoxazolyl, benzthiazolyl, benzthiadiazolyl, benztriazolyl, quinolinyl, isoquinolinyl, purinyl, furopyridinyl, and groups similar thereto.

In the present disclosure, preferably, $R_1$ may be a hydrogen atom or methyl, and $R_2$ is a substituted or unsubstituted aromatic group, an alkyl group, an arylalkyl group, or a heterocycloalkyl group.

In the present disclosure, $R_1$ and $R_2$ may be substituents selected from the following groups, without being limited thereto:

$R_1$: a hydrogen atom or methyl; and

$R_2$:

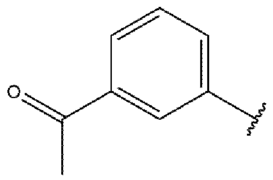 , 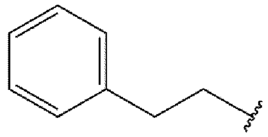 , 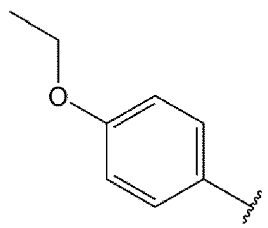 ,

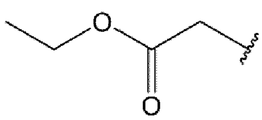 , 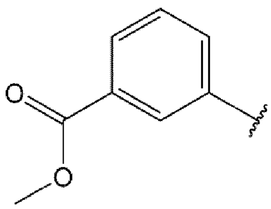 , 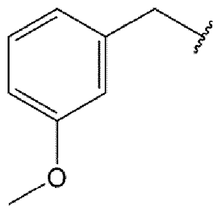 , 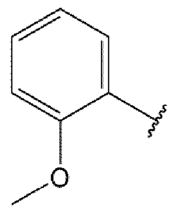 ,

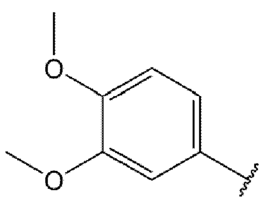 , 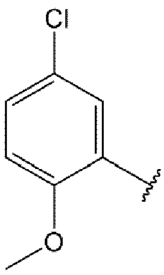 , 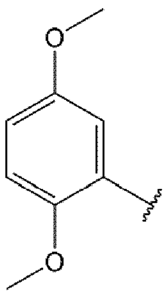 , 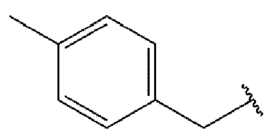 ,

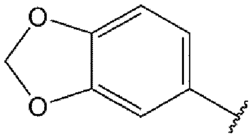, 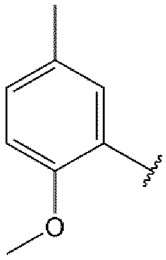, 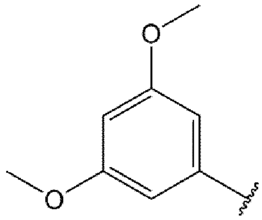, 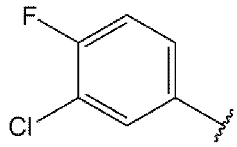,

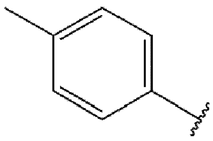, 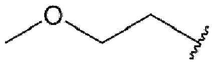, 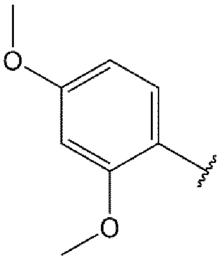, 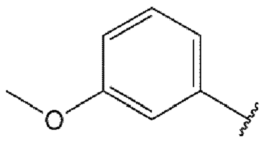,

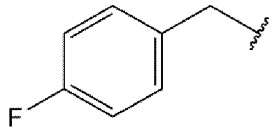, 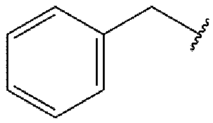 or 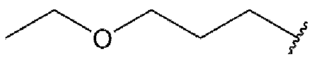.

In the present disclosure, the compound represented by Formula 1 may be a compound represented by any one formula selected from the group consisting of the following Formulas 1-1 to 1-12:

[Formula 1-1]

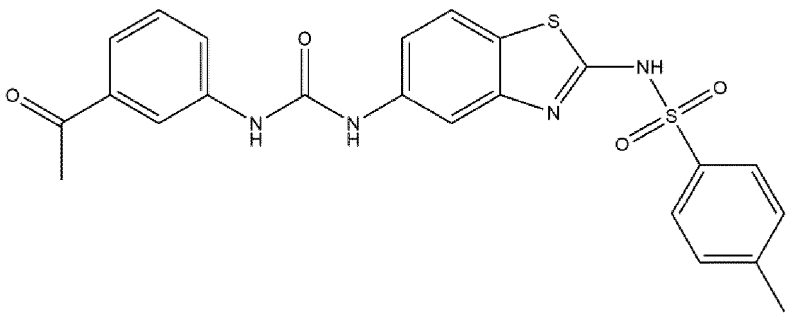;

[Formula 1-2]

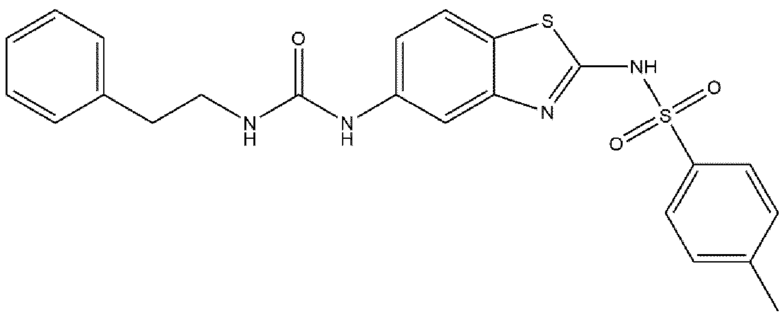;

[Formula 1-3]

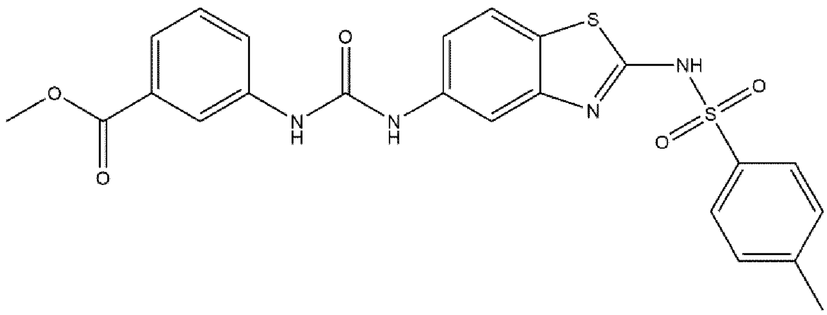

;

[Formula 1-4]

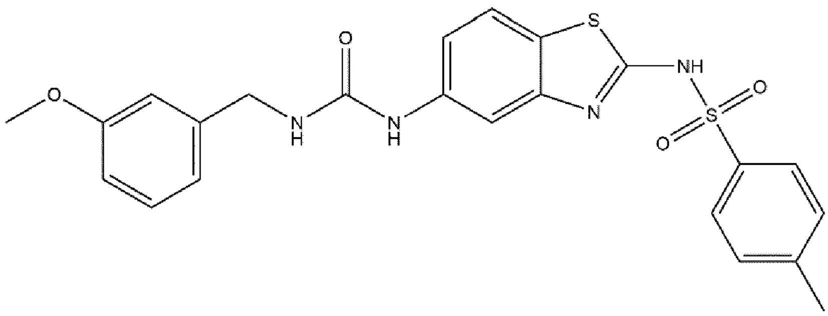

;

[Formula 1-5]

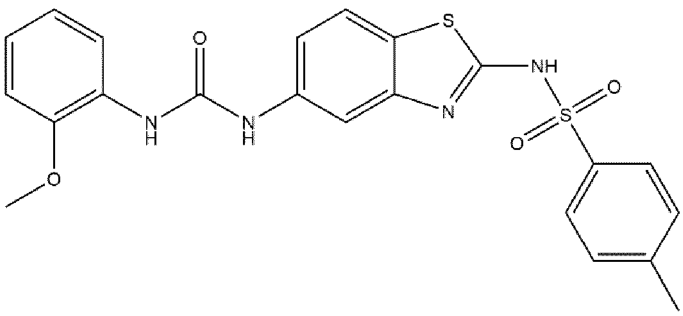

;

[Formula 1-6]

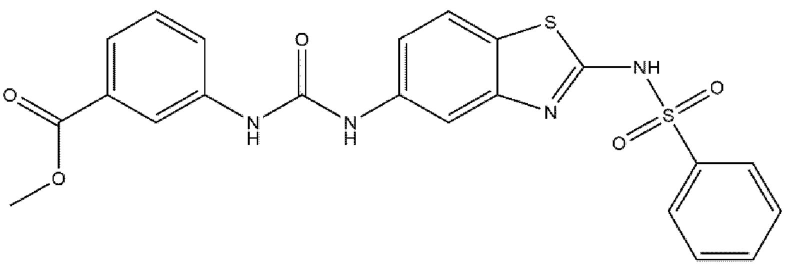

;

[Formula 1-7]

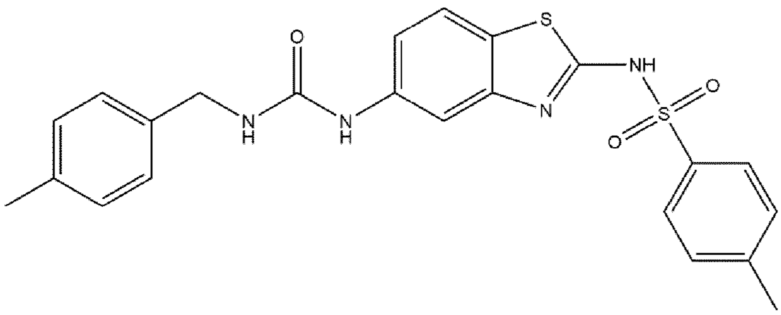

;

[Formula 1-8]

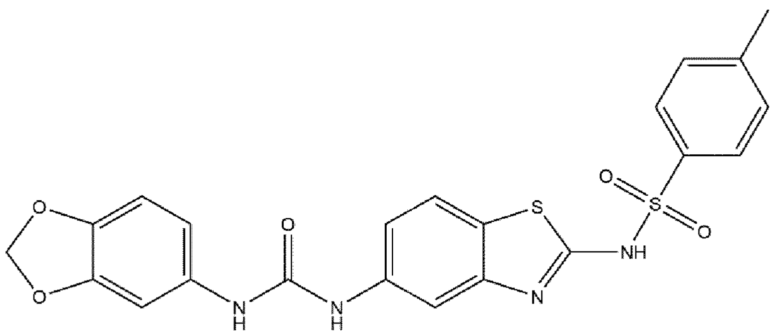

;

[Formula 1-9]

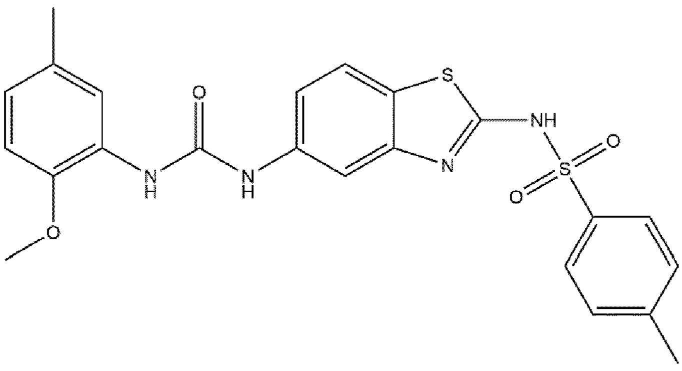

;

[Formula 1-10]

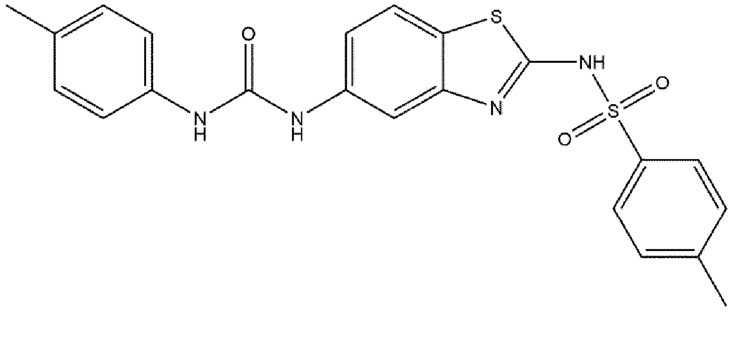

;

[Formula 1-11]

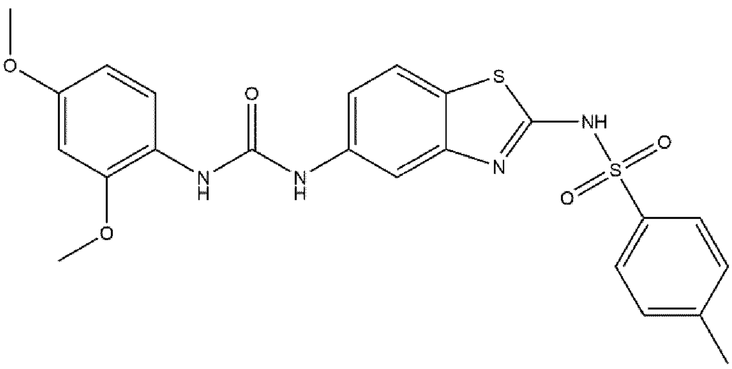

;

[Formula 1-12]

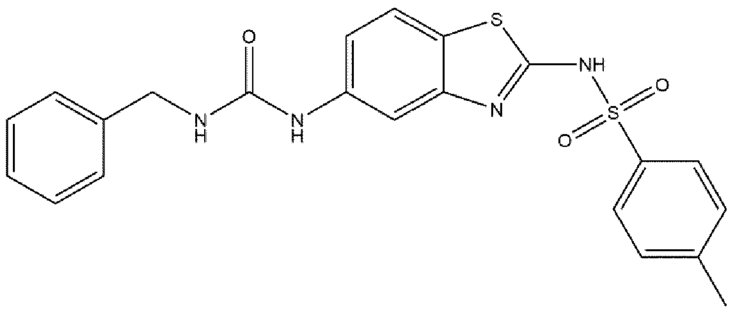

.

In the present specification, the compound of Formula 1-1 is named NIC7 (NLRP3 inhibitory compound 7), and the compounds of Formulas 1-2 to 1-12, which are derivatives of NIC7, are named NIC7a, NIC7d, NIC7e, NIC7f, NIC7j, NIC7k, NIC7l, NIC7m, NIC7p, NIC7r, and NIC7w, respectively (Table 1).

[Table 1]

| Name | Structure | IUPAC Name |
|---|---|---|
| NIC7 | 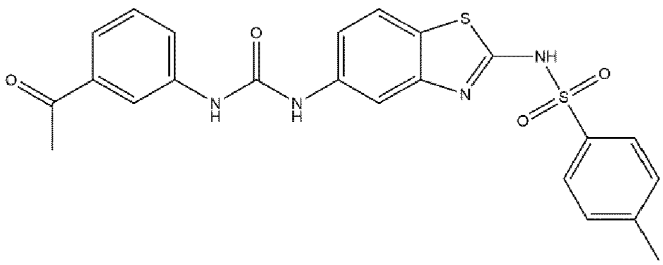 | N-(5-(3-(3-acetylphenyl)urei do) benzo[d]thiazo l-2-yl)-4-methylbenzenesulf onamide |
| NIC7a | 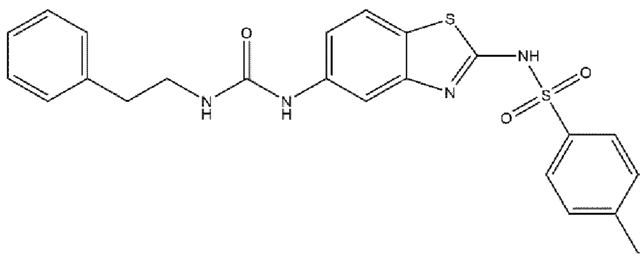 | 4-methyl-N-(5-(3-phenethylureido) b enzo[d]thiazol-2-yl)benzenesulfona mide |

(continued)

| Name | Structure | IUPAC Name |
|---|---|---|
| NIC7d | 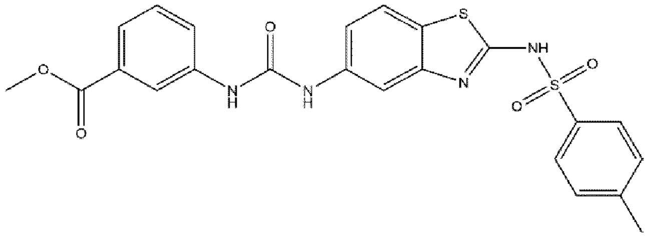 | Methyl 3-(3-(2-((4-methylphenyl) sulf onamido)benzo[d]t hiazol-5-yl) ureido)benzoat e |
| NIC7e | 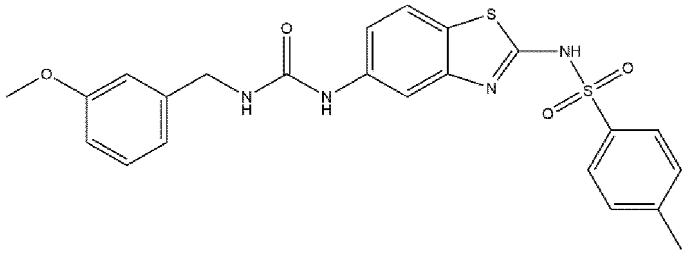 | N-(5-(3-(3-methoxybenzyl)ure ido) benzo[*d*]thiaz ol-2-yl)-4-methylben-zenesulf onamide |
| NIC7f | 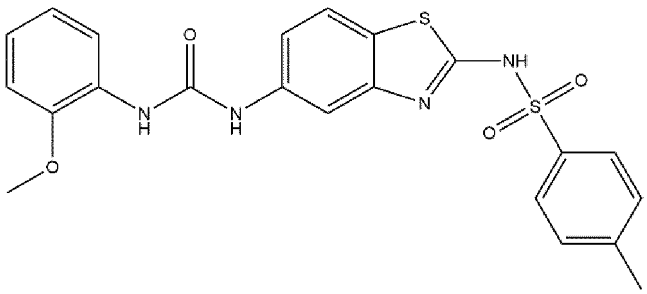 | N-(5-(3-(2-methoxyphenyl)ure ido) benzo[*d*]thiaz ol-2-yl)-4-methylben-zenesulf onamide |
| NIC7j | 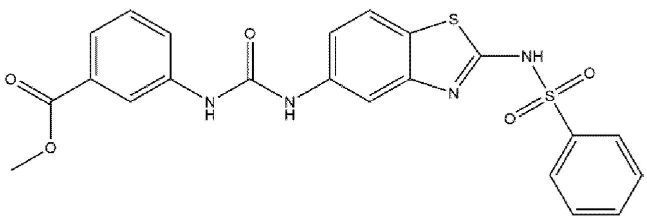 | Methyl 3-(3-(2-(phenylsulfonamid o) benzo[*d*]thiazol -5-yl)ureido)ben-zoat e |
| NIC7k | 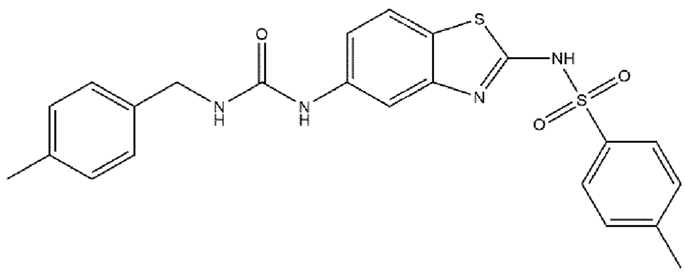 | 4-methyl-N-(5-(3-(4-methylbenzyl) urei do)benzo[*d*]thiazo 1-2-yl)ben-zenesulfona mide |
| NIC7l | 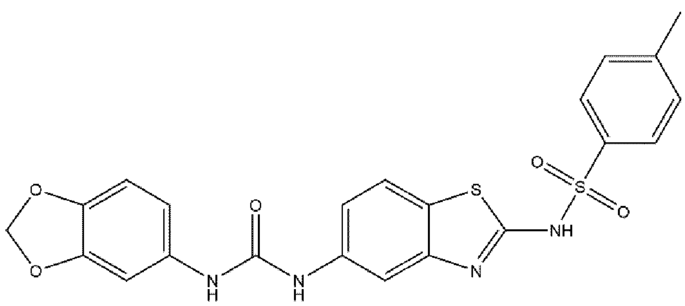 | N-(5-(3-(benzo[*d*][1,3]dio xol-5-yl) ureido)benzo[*d* ]thiazol-2-yl)-4-methylbenzenesulf onamide |

(continued)

| Name | Structure | IUPAC Name |
|---|---|---|
| NIC7m | 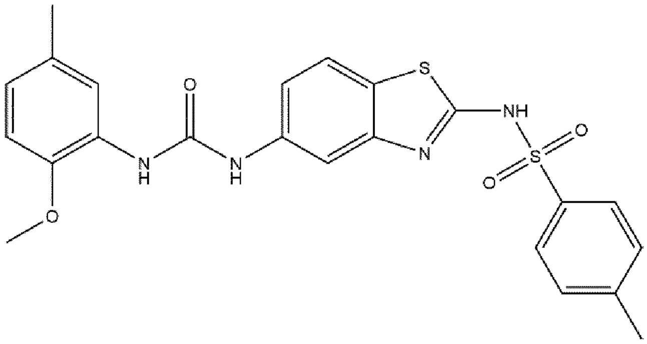 | N-(5-(3-(2-methoxy-5-methylphenyl)urei do)benzo[*d*]thiazo l-2-yl)-4-methylbenzenesulf onamide |
| NIC7p | 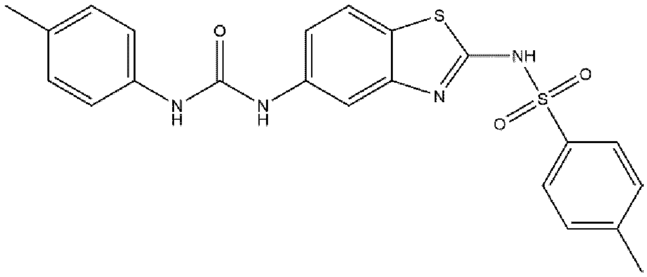 | 4-methyl-N-(5-(3-(p-tolyl)ureido) benz o[*d*]thiazol-2-yl)benzenesulfona mide |
| NIC7r | 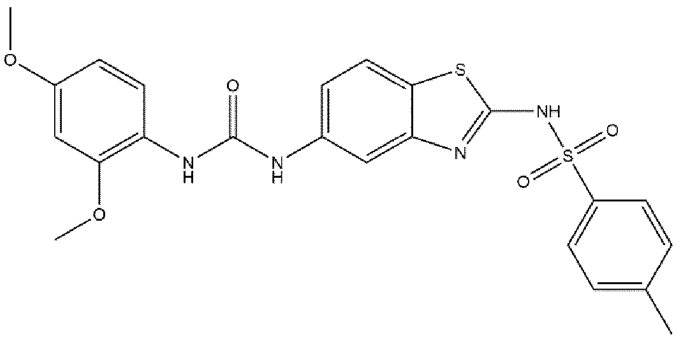 | N-(5-(3-(2,4-dimethoxyphenyl)u reido)benzo[*d*]thi azol-2-yl)-4-methylbenzenesulf onamide |
| NIC7w | 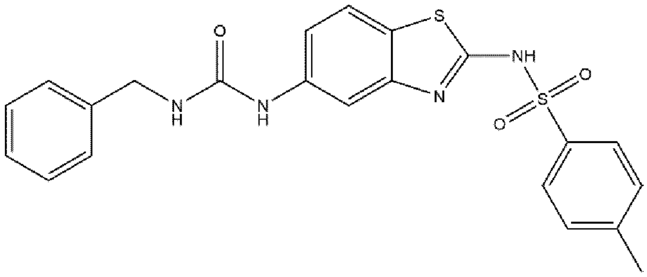 | N-(5-(3-benzylureido)benz o[*d*]thiazol-2-yl)-4-methylbenzenesulf onamide |

The compound according to the present disclosure may be used in the form of a pharmaceutically acceptable salt, and an acid addition salt formed by a pharmaceutically acceptable free acid is useful as the salt. Inorganic and organic acids may be used as free acids. Examples of the inorganic acid include hydrochloric acid, hydrobromic acid, sulfuric acid, phosphoric acid, and the like, and examples of the organic acid include citric acid, acetic acid, lactic acid, maleic acid, fumaric acid, gluconic acid, methanesulfonic acid, glycolic acid, succinic acid, tartaric acid, 4-toluenesulfonic acid, galacturonic acid, embonic acid, glutamic acid, aspartic acid, and the like.
The compound according to the present disclosurecomprises not only pharmaceutically acceptable salts, but also any salts, hydrates and solvates that may be prepared by conventional methods.
In addition, the compound according to the present disclosure may be prepared in a crystalline or amorphous form, and when the compound of Formula 1 is prepared in a crystalline form, it may be optionally hydrated or solvated.
In one Example of the present disclosure, the compound represented by Formula 1 exhibited the effect of inhibiting NLRP3 inflammasome activity by inhibiting the expression of IL-1β and caspase 1, which are key components of

NLRP3 inflammasome signaling.

Therefore, in another aspect, the present disclosure is directed to a composition for inhibiting NLRP3 inflammasome activity comprising the compound represented by Formula 1, or a pharmaceutically acceptable salt thereof.

In the present disclosure, the compound represented by Formula 1 may be a compound represented by any one formula selected from the group consisting of Formula 1-1 to Formula 1-12 above, without being limited thereto.

As used herein, the term "inhibition" refers to a phenomenon in which biological activity or signaling activity is lowered due to deficiency, incompatibility, or many other causes. Specifically, it may mean partially or completely blocking, reducing, or preventing the activity of NLRP3, or delaying, inactivating, or downregulating activation of NLRP3.

As used herein, the term "inhibitor" refers to a molecule that partially or completely inhibits the effects of other molecules such as receptors or intracellular mediators by any mechanism. In the present specification, the "composition for inhibiting NLRP3 inflammasome" is used in the same sense as the term "NLRP3 inflammasome inhibitor". In the present disclosure, the inhibition of inflammasome activity refers to an *in vivo* modification that causes a decrease in inflammasome activation in a target cell, etc. For the purposes of the present disclosure, the inhibition of inflammasome activity may be inhibition of inflammasome production, inhibition of cytokine production caused by inflammasomes, or inhibition of ASC oligomer formation. Preferably, the composition according to the present invention is able to inhibit inflammasome activity by inhibition of ASC polymer formation corresponding to a stage before inflammasome formation, without being limited thereto.

According to several studies, the NLRP3 inflammasome is one of the key components of the innate immune system and is associated with the inflammatory response caused by IL-1$\beta$, IL-18 and caspase 1 in the pathogenesis of AD (M. Saresella et al., Mol Neurodegener 11, 23 (2016)). NLRP3 inflammasome activation is triggered by three major signaling pathways: mitochondrial reactive oxygen species generation, potassium efflux, and cathepsin release after lysosomal destabilization (L. Gao et al., Inflamm Res 66, 17-24 (2017)). Once activated, the NLRP3 inflammasome forms a molecular platform to activate caspase 1. It contributes to the maturation and activation of downstream IL-1$\beta$ and IL-18, induces pyroptosis, and ultimately amplifies inflammatory responses (H. D. Liu et al., Neurochem Res 38, 2072-2083 (2013)). Also, gene expression analysis of AD patients showed higher expression of NLRP3, ASC, caspase 1, IL-1$\beta$ and IL-18. Microglia are a major source of NLRP3 inflammasome expression and have been reported to be a major cell type in the brain responsible for IL-1$\beta$ and IL-18 secretion (A. Gustin et al., PLoS One 10, e0130624 (2015)). Therefore, targeting the NLRP3 inflammasome is a viable strategy for the treatment of Alzheimer's disease and is the subject of intensive research.

According to one Example of the present invention, the compound represented by Formula 1 inhibited the expression of IL-1$\beta$ and caspase 1, which are key components of NLRP3 inflammasome signaling, and exhibited improvement in mouse cognitive behavior in an AD mouse model, suggesting that the compound represented by Formula 1 can be usefully used as a composition for preventing or treating NLRP3 inflammasome-related inflammatory diseases, including AD.

Therefore, in still another aspect, the present invention is directed to a composition for use in a method of preventing or treating NLRP3 inflammasome-related inflammatory disease comprising the compound represented by Formula 1, or a pharmaceutically acceptable salt thereof.

Disclosed herein, but not as a claimed solution, is a method for preventing or treating NLRP3 inflammasome-related inflammatory disease comprising administering the compound represented by Formula 1, or a pharmaceutically acceptable salt thereof.

Disclosed herein, but not as a claimed solution, is the use of the compound represented by Formula 1, or a pharmaceutically acceptable salt thereof for preventing or treating NLRP3 inflammasome-related inflammatory disease.

Disclosed herein, but not as a claimed solution, is the use of the compound represented by Formula 1, or a pharmaceutically acceptable salt thereof for manufacture of a medicament for preventing or treating NLRP3 inflammasome-related inflammatory disease.

In the present disclosure, the NLRP3 inflammasome-related inflammatory disease is a disease that occurs when the inflammasome is abnormally and excessively activated. Preferably, the NLRP3 inflammasome-related inflammatory disease may be a metabolic disease, a neuroinflammatory disease, or an autoinflammatory disease, without being limited thereto.

In the present disclosure, the metabolic disease collectively refers to diseases caused by metabolic disorders *in vivo.* Preferably, the metabolic disease may be obesity, hyperlipidemia, hypercholesterolemia, arteriosclerosis, type 2 diabetes, non-alcoholic fatty liver (NAFLD), or non-alcoholic steatohepatitis (NASH), more preferably type 2 diabetes or arteriosclerosis, without being limited thereto.

A recent study revealed that NLRP3 protein expression increases in adipocytes of obese patients, and that ceramide synthesized in obese adipocytes activates the NLRP3 inflammasome, and that the incidence of type 2 diabetes is significantly reduced in the absence of inflammasome activity in NLRP3 gene-deficient mice (Ryan W. Grant and Vishwa D. Dixit, Front Immunol. 2013; 4: 50). In addition, a high incidence of arteriosclerosis was reported in patients with hyperlipidemia when inflammation occurred in blood vessels, and it has been found that inflammatory cells recognize

cholesterol crystals and induce the formation and synthesis of NLRP3 inflammasomes, thus increasing the inflammatory response of blood vessels (Peter Duewell, et al., Nature. 2010 Apr 29;464(7293):1357-61). Therefore, the NLRP3 inflammasome plays a very important role in inducing and/or promoting metabolic disease.

In the present disclosure, the neuroinflammatory disease refers to a disease caused by damage to nerve tissue by an inflammatory response. Preferably, the neuroinflammatory disease is Alzheimer's disease, Parkinson's disease, Huntington's disease, Lou Gehrig's disease, Creutzfeldt-Jakob disease, multiple sclerosis, amyotrophic lateral sclerosis, diffuse Lewy body disease, leukoencephalitis, temporal lobe epilepsy, or inflammatory spinal cord injury, more preferably Alzheimer's disease, without being limited thereto.

In Alzheimer's disease, microglial inflammasome activation has been reported as an important mechanism, and even in an animal model of Alzheimer's disease, the cytokines caspase-1 and IL-1 generated by inflammasomes in the microglia are increased (Emily L. Goldberg and Vishwa Deep Dixit, Immunol Rev. 2015 May;265(1):63-74).

In the present disclosure, the auto-inflammatory disease is classified as a group of diseases in which systemic inflammation frequently occurs in a state in which autoantibodies or antigen-specific T cells are not found, unlike autoimmune disease. The auto-inflammatory disease is characterized mainly by dysregulation of innate immunity (Journal of Rheumatic Disease Vol. 21. No. 5. May 2018). Preferably, the auto-inflammatory disease may be Muckle-Wells syndrome (MWS), adult latent autoimmune diabetes (LADA), familial cold autoinflammatory syndrome (FCAS), cryopyrin-associated periodic syndrome (CAPS), neonatal-onset multisystem inflammatory syndrome (NOMID), chronic infantile neurological cutaneous and articular (CINCA) syndrome, familial Mediterranean fever (FMF), a certain form of juvenile arthritis, such as systemic onset juvenile idiopathic arthritis (SJIA), a certain form of juvenile rheumatoid arthritis, such as systemic onset juvenile idiopathic rheumatoid arthritis, or a certain form of adult rheumatoid arthritis, without being limited thereto.

As used herein, the term "prevention" refers to any action that suppresses or delays NLRP3 inflammasome-related inflammatory disease by administering a pharmaceutical composition comprising the compound represented by Formula 1 or a pharmaceutically acceptable salt thereof. In addition, as used herein, the term "treatment" used in the present disclosure refers to any action that alleviates or cures the symptoms of NLRP3 inflammasome-related inflammatory disease by administering a pharmaceutical composition comprising the compound represented by Formula 1 or a pharmaceutically acceptable salt thereof.

The composition for use in a method of preventing or treating NLRP3 inflammasome-related inflammatory disease according to the present invention may comprise a pharmaceutically effective amount of the compound represented by Formula 1 alone or in combination with at least one pharmaceutically acceptable carrier, excipient or diluent. The pharmaceutically effective amount as used herein refers to an amount sufficient to prevent, ameliorate, and treat symptoms of NLRP3 inflammasome-related inflammatory disease.

The term "pharmaceutically acceptable" refers to an additive which is physiologically acceptable and, when administered to human beings, does not cause allergic reactions such as gastrointestinal disorders and dizziness, or similar reactions. Examples of the carrier, excipient, and diluent include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oil. In addition, the composition may further comprise fillers, anti-aggregating agents, lubricants, wetting agents, flavoring agents, emulsifying agents, and preservatives.

The term "carrier" refers to a substance that facilitates the addition of a compound into a cell or tissue.

The term "diluent" is defined as a substance that stabilizes the biologically active form of a target compound and is diluted with water into which the compound is dissolved.

In addition, the composition of the present invention may comprise at least one known active ingredient having a therapeutic effect on NLRP3 inflammasome-related inflammatory disease together with the compound represented by Formula 1 above.

The composition of the present invention may be formulated using a method known in the art so as to provide quick, sustained or delayed release of the active ingredient after administration to non-human mammals. The composition may be in the form of powders, granules, tablets, emulsions, syrups, aerosols, soft or hard gelatin capsules, sterilized injection solutions, or sterilized powders.

The composition of the present invention may be administered through various routes, including oral, transdermal, subcutaneous, intravenous or intramuscular routes. The dosage of the active ingredient may be appropriately selected depending on various factors such as the route of administration, the patient's age, sex, body weight, and the severity of the disease, and the composition according to the present invention may be co-administered with a known compound having an effect of preventing, ameliorating or treating NLRP3 inflammasome-related inflammatory disease.

The composition according to the present invention may be in the form of a food composition.

In the present disclosure, the food composition is preferably a health functional food or a food additive. The health functional food or food additive is preferably in the form of powders, granules, tablets, capsules or beverages, without being limited thereto.

The food of the present disclosure may comprise the compound of Formula 1 according to the present disclosure, alone or together with other foods or food ingredients, and may be appropriately used according to a conventional method.

There is no particular limit to the kind of food. Examples of foods to which the compound of Formula 1 according to the present disclosure may be added include meats, sausages, bread, chocolate, candies, snack, confectionery, pizza, ramen, other noodles, gum, dairy products including ice cream, various soups, beverages, teas, drinks, alcoholic beverages, and multi-vitamin preparations. The foods include all health foods in a conventional sense.

The food composition of the present disclosure may be a beverage composition. The beverage composition may additionally comprise various flavoring agents or natural carbohydrates, like conventional beverages. Examples of the natural carbohydrates include monosaccharides such as glucose and fructose, disaccharides such as maltose and sucrose, polysaccharides such as dextrins and cyclodextrins, and sugar alcohols such as xylitol, sorbitol, and erythritol. As sweeteners, natural sweeteners such as thaumatin and stevia extract, or synthetic sweeteners such as saccharin and aspartame may be used.

Essential ingredients that may be comprised in the food composition of the present disclosure comprise the compound of Formula 1 according to the present disclosure, and additional ingredients such as various herbal extracts, supplementary food additives, or natural carbohydrates. In addition, supplementary food additives may be additionally added, including conventional supplementary food additives known in the art, for example, flavoring agents, coloring agents, fillers, stabilizers, and the like. Examples of the natural carbohydrates include monosaccharides such as glucose and fructose, disaccharides such as maltose and sucrose, polysaccharides, for example, conventional sugars such as dextrins and cyclodextrins, and sugar alcohols such as xylitol and sorbitol. Examples of the flavoring agents include natural flavoring agents (thaumatin, stevia extracts, such as rebaudioside A, glycyrrhizin, etc.) and synthetic flavoring agents (saccharin, aspartame, etc.). In addition, the food composition of the present disclosure may comprise various nutrients, vitamins, minerals (electrolytes), flavoring agents such as synthetic flavoring agents and natural flavoring agents, colorants, fillers (cheese, chocolate, etc.), pectic acid and a salt thereof, alginic acid and a salt thereof, organic acids, protective colloidal thickeners, pH adjusting agents, stabilizers, preservatives, glycerin, alcohol, carbonizing agents that are used in carbonated beverages, and the like. Additionally, the food composition of the present disclosure may comprise fruit flesh that is used for the preparation of natural fruit juice, fruit juice beverages or vegetable beverages. These components may be used individually or in combination. The content of these additives is not so critical, but is generally selected in the range of 0.01 to 0.1 parts by weight based on 100 parts by weight of the food composition of the present disclosure.

Hereinafter, the present invention will be described in more detail with reference to examples. These examples are only for illustrating the present invention, and it will be apparent to those skilled in the art that the scope of the present invention is not to be construed as being limited by these examples.

## Example 1: Materials and methods

### Example 1-1: Cell line and reagents

THP-1 cells were grown in RPMI 1640 medium supplemented with 10% fetal bovine serum (FBS) (Thermo Fisher Scientific, Inc., Waltham, MA, USA) and 1% penicillin/streptomycin solution (Gibco). Differentiation into macrophages was performed by treatment with 80 nM PMA for 24 to 48 hours. The cells were incubated in a humidified incubator (Thermo Fisher Scientific, Inc.) at 5% $CO_2$ and 37°C. LPS (from *Escherichia coli* 0111: B4) and PMA were purchased from Sigma-Aldrich Co. (St. Louis, MO, USA). Nigericin was acquired from InvivoGen (San Diego, CA, USA).

### Example 1-2: Cell viability analysis

Cell viability was calculated using a colorimetric MTT assay (Sigma-Aldrich Co.). THP-1 ($10^5$/well; 24 hours) cells were differentiated overnight with PMA (80 nM) in 96-well plates (BD Biosciences, San Jose, CA, USA) and treated with various concentrations of each test compound for 24 hours. After that, the medium was replaced with a 100 μL/well MTT solution (10%), and the cells were incubated at 37°C for 3 hours. This solution was replaced with 100 μL/well dimethyl sulfoxide (DMSO) and the plates were further incubated at 37°C for 30 min. The plates were read at a wavelength of 540 nm on a microplate reader (Molecular Devices, Silicon Valley, California).

### Example 1-3: Enzyme-linked immunosorbent assay (ELISA)

THP-1 cells ($10^5$/well) were differentiated with PMA (80 nM, 48 hours) in a 96-well plate (BD Biosciences). The differentiated macrophages were primed with LPS (10 ng/mL) for 4 hours. Thereafter, the cells were treated with each compound for 1 hour in a serum-free medium, followed by activation of NLRP3 by nigericin for an additional 1 hour. The supernatant was collected and the inhibition of human IL-1β secretion was analyzed using the Human IL-1β Uncoated

ELISA Kit (Thermo Fisher Scientific, Inc.). A microplate spectrophotometer system (Molecular Devices) was employed to read absorbance in the plate.

Example 1-4: Western blot analysis

Cytoplasmic proteins were extracted using NE-PER™ Nuclear and Cytoplasmic Extraction Reagent (Thermo Fisher Scientific, Inc.), and the total-protein was measured using a Bicinchoninic (BCA) assay kit (Sigma-Aldrich Co.). The proteins were separated by SDS-PAGE and transferred to a membrane using a Mini-PROTEAN Tetra Cell and Mini Trans-Blot Electrophoretic Transfer Cell System (Bio-Rad Laboratories, Hercules, CA, USA). The membranes were blocked with skim milk (5%) for 1 hour, and immunoblotted with antibodies against NLRP3, IL-1β, mature IL-1β (Cell Signaling Technology Inc., Danvers, MA, USA), caspase 1 and β-actin (Santa Cruz Biotechnology Inc., Dallas, TX, USA) at 4°C overnight. After washing with PBS supplemented with 0.1% of Tween 20, the membranes were treated with a peroxidase-conjugated anti-rabbit or anti-mouse IgG antibody (1:1,000) for 2 hours at room temperature. The SuperSignal West Pico ECL solution (Thermo Fisher Scientific, Inc.) was used to detect protein bands that were visualized on a ChemiDoc™ Touch Imaging System (Bio-Rad Laboratories).

Example 1-5: Microsomal Stability

Stock solutions of NIC7 and NIC7w were prepared at a concentration of 10 mM in DMSO, and were diluted to a concentration of 100 μM with 100% methanol as a working solution. These NIC7 and NIC7w solutions were diluted to a concentration of 1 μM with potassium phosphate buffer (pH 7.4), and then each solution was incubated with 0.5 mg/mL liver microsomes (human) at 37°C for 5 minutes. The reaction was initiated by adding a NADPH-generating system (3.3 mM glucose-6-phosphate (G6P), 1.3 mM β-$NADP^+$, 3.3 mM $MgCl_2$, and 0.4 U/mL glucose-6-phosphate dehydrogenase (G6PD)) at 37°C. The reaction was stopped at 0 and 30 minutes using cold acetonitrile containing carbamazepine as an internal standard at 20 ng/mL. After vortexing and centrifugation, the supernatant was analyzed with liquid chromatography with tandem mass spectrometry (LC-MS/MS). All microsomal assays were performed in duplicate.

Example 1-6: Cytochrome P450 inhibition

The inhibitory effects of NIC7 and NIC7w on the metabolism of the five major P450 probe substrates were evaluated using a previously reported method (S. Seino, Diabetologia 55, 2096-2108 (2012)) with minor modifications. A cocktail of substrates (phenacetin O-deethylase, tolbutamide 4-hydroxylase, mephenytoin 4-hydroxylase, dextromethorphan O-demethylase, and midazolam 1'-hydroxylase) was used for five major P450 enzymes, CYP1A2, CYP2C9, CYP2C19, CYP2D6, and CYP3A4, respectively. NIC7 and NIC7w were prepared at a concentration of 10 mM in DMSO and dissolved in methanol at a concentration of 1 mM. In all experiments, the final concentration of an organic solvent (methanol) for cocktail incubation was set at 1.0% (v/v). The concentrations of all substrates in the incubation mixtures are listed in Table 2 below.

[Table 2] CYP450 enzymes used for pharmacokinetic analysis

| Enzyme | Substrate | Concentration (μM) | Metabolite |
|---|---|---|---|
| CYP1A2 | Phenacetin | 100 | Acetaminophen |
| CYP2C9 | Tolbutamide | 100 | Hydroxytolbutamide |
| CYP2C19 | Mephenytoin | 20 | Hydroxymephenytoin |
| CYP2D6 | Dextromethorphan | 5 | Dextrorphan |
| CYP3A4 | Midazolam | 50 | Hydroxymidazolam |

The effects of NIC7, NIC7w and MCC950 were evaluated using the five CYP450 enzymes. The relevant substrates along with the concentrations used and the metabolites generated are listed in Table 2 above.

Both NIC7 and NIC7w were used at 10 μM, and pooled human liver microsomes were assayed at 0.25 mg/mL concentration. The substrate cocktail solution was incubated with the microsomes for 5 min at 37°C. The reaction was initiated by adding an NADPH-generating system (the same as described in Examples 1-5) at 37°C and 1300 rpm. The reaction was stopped at 10 min by the addition of cold acetonitrile containing carbamazepine as an internal standard at 20 ng/mL. After vortexing and centrifugation, the supernatant was analyzed with LC-MS/MS. All microsomal incubations were performed in triplicate.

Example 1-7: LC-MS/MS (Liquid Chromatograph-Tandem Mass Spectrometer) analysis

After sample preparation, 0.1 mL acetonitrile containing 20 ng/mL internal standard was added to a 50 μL aliquot of each biological sample. After vortexing and centrifugation at 12,000 rpm for 10 minutes, 10 μL of the supernatant was analyzed with LC-MS/MS.

In the LC system, a reversed-phase $C_{18}$ column (BEH $C_{18}$, 2.1 mm x 100 mm i.d., 1.7 μm; Waters, Ireland) was maintained at 4°C. The composition of the mobile phase was changed from 80% of 0.1% formic acid in water to 80% of 0.1% formic acid in acetonitrile for 3 minutes, and then switched to 80% of 0.1% formic acid in water for 3.1 minutes, and maintained at 0.5 at mL/min for 5 minutes. The analytes were monitored on an API5500 triple quadrupole mass spectrometer MS (AB Sciex, Foster City, CA) equipped with a turbo ion spray interface for electrospray ionization and operated in positive ion mode at 5.5 kV and 500°C at 50 L/min of atomizing gas flow, 50 L/min of turbo ion-spray gas flow, 20 L/min of curtain gas flow, 5.5 kV of ring voltage and 5 Torr of collision gas (nitrogen) pressure. The mass transitions for NIC7, NIC7w and the internal standard were *m/z* 481.1→165.0 (collision energy, 45 eV), 453.1→165.0 (41.0 eV) and 236.9→194.0 (29 eV), respectively, in the multiple reaction monitoring mode.

Example 1-8: AD Model

6-week-old male C57BL/6 mice (19 to 22 g) were used, and 5 mice per cage were bred in a feedlot. To create an AD model, experimental animals were administered isoflurane by inhalation, and then injected with $A\beta_{1-42}$ (5 μL) using a Hamilton microsyringe with a 26-gauge needle. The AD model mice were trained for 20 minutes/mouse for 2 days before the experimental procedure, and then subjected to Y-maze tests and novel object recognition tests (for cognitive evaluation) . Behavioral evaluation was performed 7 days after $A\beta_{1-42}$ injection. The animals were subdivided into AD, NIC7-treated, vehicle (DMSO)-treated, donepezil-treated (positive control) and normal (no-disease) groups. Cognitive evaluation was performed on days 7, 14, 21 and 28 after injection.

Example 1-9: Behavioral analysis

The alteration in spatial cognitive behavior was assessed using a Y-maze apparatus with three horizontal identical arms (35 cm long × 15 cm high × 5 cm wide) symmetrically arranged at 120° to each other. The evaluation was performed in the Y-maze test 7 days after Aβ injection. After introduction into the Y-maze, each animal was allowed to move freely for 2 minutes and then measured for 5 minutes. The test subjects tended to enter an arm different from the arm they had previously visited. The number of arm entries and triads was recorded to calculate the percentage of alternation. The entry is defined as the presence of all four limbs within a particular maze arm.

A novel object recognition test was performed in an open field box containing two identical objects. Animals were first familiarized with these identical objects for 2 minutes, and then one of the objects was replaced with a novel object, followed by reading for 5 minutes. The time spent with an identical object ($t_A$) and the time spent with a novel object ($t_B$) were calculated, and the recognition index ratio was calculated via the formula $(t_B/(t_A + t_B)) \times 100$.

Example 1-10: Preparation of the Virtual screening library

Chemical libraries were downloaded from the ZINC database (drug-like and lead-like) as well as from various commercial vendors (Table 3) (J. J. Irwin, et al., J Chem Inf Model 52, 1757-1768 (2012)).

[Table 3] Chemical compound libraries used for virtual screening

| Compound library | Number of compounds |
|---|---|
| ZINC drug-like | 14, 480, 911 |
| ZINC lead-like | 5, 449, 805 |
| MolPort | 1, 041, 282 |
| Enamine | 3, 006, 354 |
| ChemBridge | 1, 591, 767 |
| ChemDiv | 1, 960, 042 |
| Life Chemicals | 500,011 |
| Maybridge | 72,257 |

(continued)

| Compound library | Number of compounds |
|---|---|
| **Total** | **28,102,429** |
| *Ligand libraries are freely available from MolPort (https://www.molport.com/shop/index) and ZINC database (https://zinc.docking.org/)* | |

Chemical structures underwent a "wash" procedure in the Molecular Operating Environment (MOE) software (M. O. E. (MOE) (Molecular Operating Environment, 2019.01; Chemical Computing Group ULC, 1010 Sherbooke St. West, Suite #910, Montreal, QC, Canada, H3A 2R7, 2020) by considering the removal of duplicate entries, disconnected groups, salt, and inorganic metal ions. Structures with reactive groups were eliminated and the largest fragments in the library were retained. Explicit hydrogen atoms were added, and equilibration was performed again by strong base protonation and strong acid deprotonation at pH 7.0. Intramolecular bonds were scaled to reasonable lengths, and up to 10 tautomeric states were listed for each structure (the most feasible tautomer for each ligand). The partial charge for the ligand was calculated and energy minimization was performed using the Merck molecular force field MMFF94x until a root mean square gradient of 0.01 was reached.

Example 1-11: Molecular fingerprint-based structural similarity

Fingerprints were determined for all molecules in the screening library, using the bit-packed MACCS Structural Keys (FP: BIT MACCS) scheme. In this technique, each molecule is represented as a bit vector encoding the presence or absence of structural features, where a specific bit position is assigned to each feature. A MOE in-house support vector language (SVL) script was used to identify chemical structures having at least 60 to 75% similarity to a selected NLRP3 antagonist set (Table 4).

[Table 4] List of NLRP3 inhibitors

| Inhibitor | Target | Activity (~$IC_{50}$) | Reference |
|---|---|---|---|
| MCC950 | NLRP3 | 8 nM | M. R. de Zoete, et al., Cold Spring Harb Perspect Biol 6, a016287 (2014) |
| CY-09 | NLRP3 | 5 μM | H. Guo, et al., Nat Med 21, 677-687 (2015) |
| Oridonin | NLRP3 | 0.5 μM | H. Wen, et al., Immunity 39, 432-441 (2013) |
| Tranilast | NLRP3 | 25-50 μM | M. Lamkanfi, V. M. Dixit, Cell 157, 1013-1022 (2014) |
| MNS | NLRP3 | 2 μM | J. P. de Rivero Vaccari, et al., J Cereb Blood Flow Metab 34, 369-375 (2014) |
| OLT1177 | NLRP3 | 1 μM | K. Zhou, et al., J Immunol Res 2016, 9238290 (2016) |
| Bay 11-7082 | NLRP3, NLRC4 | 5 μM | K. Schroder, J. Tschopp, Cell 140, 821-832 (2010) |
| BOT-4-one | NLRP3, NLRC4 | 0.59-1.28 μM | V. Sagulenko et al., Cell Death Differ 20, 1149-1160 (2013) |
| BC7 | NLRP3 | 1.16 μM | |
| BC23 | NLRP3 | 2.29 μM | J. Shi et al., Nature 514, 187-192 (2014) |
| NBC6 | NLRP3 | 574 nM | |
| *Ligand names are presented as their respective literature/database identifiers.*<br>*~$IC_{50}$: approximate inhibitory concentration; NLRC4: NOD-, LRR- and CARD-containing 4; NLRP: NOD-, LRR- and pyrin domain-containing protein* | | | |

The above ligands were tested for the fingerprint-based similarity search and pharmacophore generation against the multiconformational compound library.

The search was performed using a similarity metric (TC). TC measures a similarity between two (A and B) fingerprints by comparing the common features of each ligand to the total. The similarity metric is based on the following formula:

#AB/(#A + #B - #AB)

wherein both A and B are fingerprints, and "#" represents the number of features in each fingerprint. The resulting ligands were stored in a separate library for further screening.

Example 1-12: Preparation of protein structure *in silico* (computer programming in virtual experiment)

The cryo-EM structure of human NLRP3 [PDB ID: 6NPY] (H. Sharif et al., Nature 570, 338-343 (2019)) in complex with NEK7 was retrieved from PDB. Unnecessary ligands including water present in the structure were eliminated. The structure was protonated at pH 7.0 and the energy was minimized through the Amber10: EHT force field until a root mean square gradient of 0.01 was reached.

Example 1-13: Pharmacophore generation and screening

Various pharmacophore models were generated for each ligand based on the best-ranking interaction with either NLRP3 site I or site II. Using the planar-polar-charged-hydrophobic scheme, pharmacophore features were assigned around important ligand groups. Next, ligand-based virtual screening was performed to identify structures that satisfy the essential pharmacophore constraints applied to each ligand. The resulting hits were combined into a single library and subjected to structure-based virtual screening through molecular docking.

Example 1-14: Structure-based virtual screening

Virtual screening of the compound library that resulted from the pharmacophore screening was performed on both site I and site II of NLRP3, separately. Site I (Q636, E637, E638, E743 and D748) corresponds to the HD2 and LRR of NLRP3, whereas site II (I149, E150, L162, R165, Y166, A225, A226, G227, I228, G229, K230, T231, I232, R235, H258, R260, R349, F371, Y379, P410, L411, W414, F506, V510, I519 and H520) corresponds to the NACHT domain of NLRP3. Docking was performed using the triangle matcher placement method and the London dG scoring function. Ligand poses were recorded using the MMFF94x force field and GBVI/WSA dG scoring function. The residues of NLRP3 were kept rigid and the ligands remained flexible during docking calculation. At least 15 different docked poses of each ligand were saved and ranked by the binding affinity S-score. Each docking round was repeated with the induced-fit docking method, where both ligand and receptor side chains were allowed to adjust their conformations to obtain the best fit. The top-scoring 19 consensus ligands from both rigid and induced-fit docking rounds were selected for the experimental validation of their NLRP3 antagonistic activity.

Example 1-15: Identification of potent derivatives of initial lead

Structural derivatives of NIC7, an initial lead, were retrieved from the MolPort database. For further analysis, ligands characterized by 85 to 95% structural similarity with the parent scaffold NIC7 were considered. About 100 ligands were downloaded as SDF files and converted to PDB format in MOE software. The ligands were washed and energy minimized using the same protocol used to prepare the initial screening library mentioned above. Derivatives were docked and ranked by the binding score for site I and site II of NLRP3 with the same docking parameters as described in Examples 1-14. The top-scoring 25 ligands that fit best into the pocket and showed maximal interactions were selected for the experimental validation of their antagonistic activity.

Example 1-16: Statistical analysis

The presented data are the averages of 3 independent experiments (each experiment was performed in duplicate), and statistical analysis was performed using a two-tailed paired Student's t-test ($P < 0.05$).

**Example 2: Synthesis and physicochemical characterization of NIC7 derivatives**

Synthesis processes for derivatives (NIC7a to NIC7y) of NIC7 (N-(5-(3-(3-acetylphenyl)ureido)benzo[d]thiazol-2-yl)-4-methylbenzenesulfonamide) and physicochemical properties thereof are as follows.

1) NIC7a: 4-methyl-N-(5-(3-phenethylureido)benzo[*d*]thiazol-2-yl)benzenesulfonamide

i) Synthesis of 4-methyl-N-(5-nitrobenzo[d]thiazol-2-yl)benzenesulfonamide

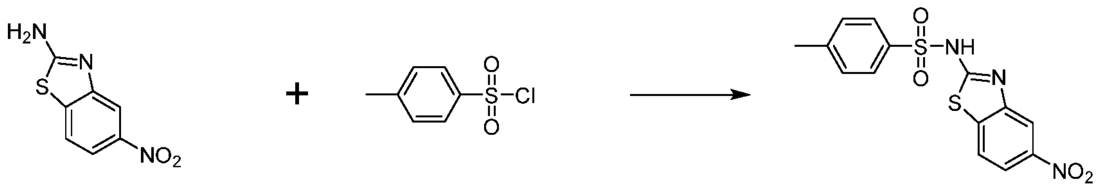

500 mg (2.56 mmol) of 2-amino-5-nitrobenzothiazole was dissolved in 20 mL of dichloromethane, and then 586 mg (3.07 mmol) of 4-methylbenzenesulfonyl chloride, 4-(dimethylamino)pyridine 156 mg (1.28 mmol) and 857 μL (6.15 mmol) of triethylamine were added thereto, followed by stirring at room temperature for 18 hours. After completion of the reaction, the organic layer diluted with dichloromethane was washed three times with saturated brine, dried using anhydrous sodium sulfate, and concentrated under reduced pressure to remove the solvent. Purification by column chromatography yielded 689 mg (77% yield) of the title compound.

ii) Synthesis of N-(5-aminobenzo[d]thiazol-2-yl)-4-methylbenzenesulfonamide

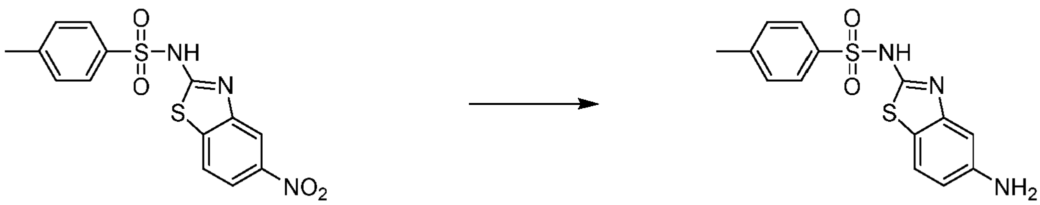

686 mg (1.97 mmol) of 4-methyl-N-(5-nitrobenzo[d]thiazol-2-yl)benzenesulfonamide was dissolved in 20 mL of methanol, and then 69 mg of palladium on carbon was added thereto. The reaction solution was stirred at room temperature for 24 hours under a hydrogen gas atmosphere (1 atm). After completion of the reaction, palladium on carbon was filtered out through celite, and the solvent was removed by concentration, thus obtaining 573 mg (91% yield) of the title compound.

iii) Synthesis of 4-methyl-N-(5-(3-phenethylureido)benzo[d]thiazol-2-yl)benzenesulfonamide

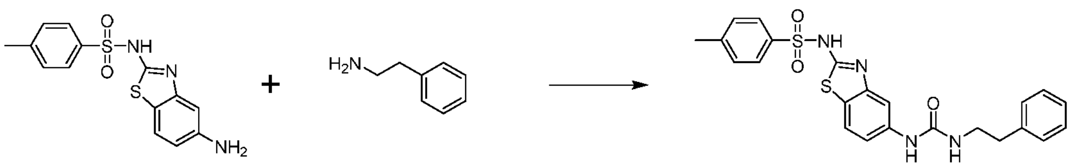

573 mg (1.79 mmol) of N-(5-aminobenzo[d]thiazol-2-yl)-4-methylbenzenesulfonamide was dissolved in 15 mL of tetrahydrofuran, and 248 μL of 2-phenethylamine (1.97 mmol) and 320 mg (1.97 mmol) of carbodiimidazole were added thereto, and the reaction mixture was stirred under reflux for 4 hours. After completion of the reaction, the reaction mixture was diluted with ethyl acetate and washed with 1N aqueous hydrochloric acid aqueous solution and saturated brine. The organic layer was dried over anhydrous sodium sulfate, and the solvent was removed by concentration under reduced pressure. Purification by column chromatography yielded 778 mg (93% yield) of the title compound.

The NMR data are as follows:

$^1$H NMR (400 MHz, DMSO$d_6$) δ 12.89 (s, 1H), 8.60 (s, 1H), 7.63 (m, 3H), 7.47 (d, J = 7.2 Hz, 2H), 7.30 (m, 4H), 7.21 (m, 3H), 6.97 (d, J = 1.6Hz, 1H), 6.07 (s, 1H), 3.31 (t, J = 8.0 Hz, 2H), 2.78 (t, J = 8.0 Hz, 2H), 2.41 (s, 3H).

NIC7b to NIC7y were synthesized with reference to the synthesis process for NIC7a.

2) NIC7b: N-(5-(3-(4-ethoxyphenyl)ureido)benzo[d]thiazol-2-yl)benzenesulfonamide

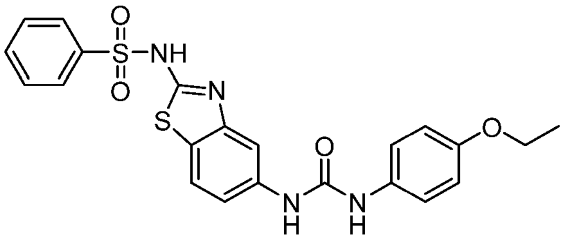

$^1H$ NMR (400 MHz, DMSOd$_6$) δ 13.0 (s, 1H), 8.64 (s, 1H), 8.33 (s, 1H), 7.86 (d, J = 7.2 Hz, 2H), 8.80 (s, 1H), 7.56 (m, 3H), 7.30 (d, J = 8.0 Hz, 2H), 7.04 (d, J=7.2 Hz, 1H), 6.79 (d, J = 8.0 Hz, 2H), 4.01 (q, J = 7.2 Hz, 2H), 1.38 (t J = 7.2 Hz, 3H)

3) NIC7c: Ethyl ((2-((4-methylphenyl)sulfonamido)benzo[*d*]thiazol-5-yl) carbamoyl) glycinate

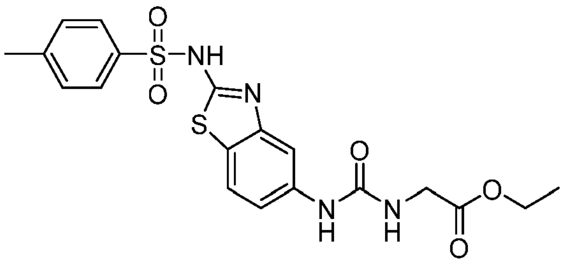

$^1H$ NMR (400 MHz, DMSOd$_6$) δ 12.47 (s, 1H), 8.95 (s, 1H), 7.72 (m 2H), 7.50 (d, J = 7.2 Hz, 2H), 7.32 (d, J = 7.2 Hz, 2H), 7.01 (m, 1H), 6.42 (m, 1H), 4.17 (q, J=7.6 Hz, 2H), 3.88 (s, 2H), 2.39 (s, 3H), 1.28 (t, J = 7.6 Hz, 3H)

4) NIC7d: Methyl 3- (3- (2- ((4-methylphenyl)sulfonamido)benzo[*d*]thiazol-5-yl)ureido)benzoate

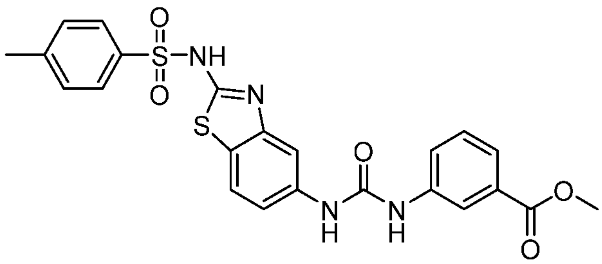

$^1H$ NMR (400 MHz, DMSOd$_6$) δ 12.94 (s, 1H), 8.74 (d, J = 7.2 Hz, 2H), 8.15 (s, 1H), 7.82 (s, 1H), 7.73 (s, J = 7.2 Hz, 2H), 7.62 (d, J = 6.8 Hz, 1H), 7.55 (d, J = 7.2 Hz, 2H), 7.45 (m, 4H), 7.07 (d, J = 6.8 Hz, 1H), 3.89 (s, 3H), 2.40 (s, 3H)

5) NIC7e: N-(5-(3-(3-methoxybenzyl)ureido)benzo[*d*]thiazol-2-yl)-4-methylbenzenesulfonamide

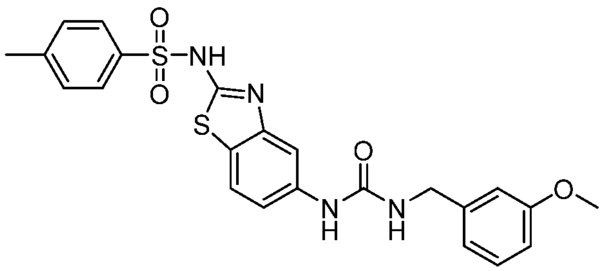

$^1H$ NMR (400 MHz, DMSOd$_6$) δ 12.90 (s, 1H), 8.71 (s, 1H), 7.76 (s, 1H), 7.72 (d, J = 7.6 Hz, 2H), 7.50 (d, J = 8.0 Hz, 1H), 7.31 (d, J = 7.6 Hz, 2H), 7.22 (t, J = 8.0 Hz, 1H), 7.01 (d, J = 8.0 Hz, 1H), 6.86 (m, 2H), 6.77 (m, 1H), 6.53 (m, 1H), 4.29 (s, 2H), 3.75 (s, 3H), 2.38 (s, 3H)

6) NIC7f: N-(5-(3-(2-methoxyphenyl)ureido)benzo[*d*]thiazol-2-yl)-4-methylbenzenesulfonamide

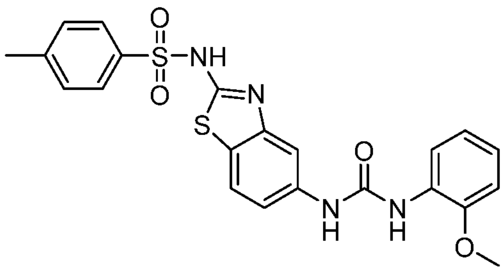

$^1H$ NMR (400 MHz, DMSOd$_6$) δ 12.94 (s, 1H), 9.47 (s, 1H), 8.16 (s, 1H), 8.13 (d, J = 2.8 Hz, 1H), 7.82 (s, 1H), 7.73 (d, J = 7.2 Hz, 2H), 7.55 (d, J = 7.2 Hz, 2H), 7.32 (d, J = 7.2 Hz, 2H), 7.04 (d, J = 2.8 Hz, 1H), 6.90 (m, 2H), 3.90 (s, 3H), 2.39 (s, 3H)

7) NIC7g: N-(5-(3-(3,4-dimethoxyphenyl)ureido)benzo[*d*]thiazol-2-yl)-4-methylbenzenesulfonamide

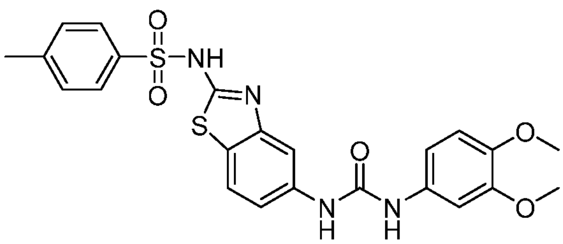

$^1H$ NMR (400 MHz, DMSOd$_6$) δ 12.90 (s, 1H), 8.75 (s, 1H), 8.41 (s, 1H), 7.82 (s, 1H), 7.75 (s, 1H), 7.73 (s, 1H), 7.75 (d, J = 7.2 Hz, 2H), 7.33 (d, J = 7.2 Hz, 2H), 7.22 (s, 1H), 7.04 (m, 1H), 6.81 (s, 1H), 3.77 (s, 3H), 3.74 (s, 3H), 2.40 (s, 3H)

8) NIC7h: N-(5-(3-(5-chloro-2-methoxyphenyl)ureido)benzo[d]thiazol-2-yl)benzenesulfonamide

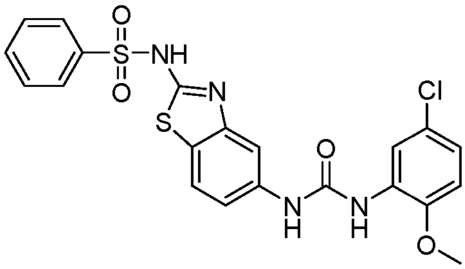

$^1H$ NMR (400 MHz, DMSOd$_6$) δ 13.01 (s, 1H), 9.53 (s, 1H), 8.29 (s, 1H), 8.22 (s, 1H), 7.86 (d, J = 7.2 Hz, 2H), 7.80 (s, 1H), 7.57 (m, 4H), 7.05 (d, J = 6.8 Hz, 1H), 6.94 (m, 2H), 3.80 (s, 3H)

9) NIC7i: N-(5-(3-(2,5-dimethoxyphenyl)ureido)benzo[d]thiazol-2-yl)benzenesulfonamide

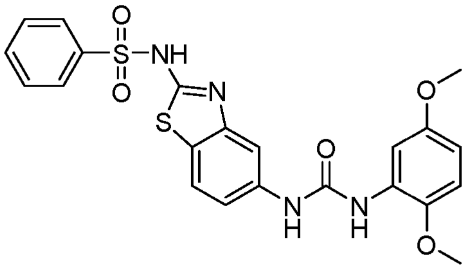

$^1H$ NMR (400 MHz, DMSOd$_6$) δ 12.98 (s, 1H), 9.50 (s, 1H), 8.18 (s, 1H), 7.85 (m, 3H), 7.56 (m, 4H), 7.04 (d, J = 6.4 Hz, 1H), 6.86 (d, J = 7.2 Hz, 2H), 6.45 (d, J = 6.4 Hz, 1H), 3.84 (s, 3H), 3.72 (s, 3H)

10) NIC7j: Methyl 3-(3-(2-(phenylsulfonamido)benzo[*d*]thiazol-5-yl)ureido)benzoate

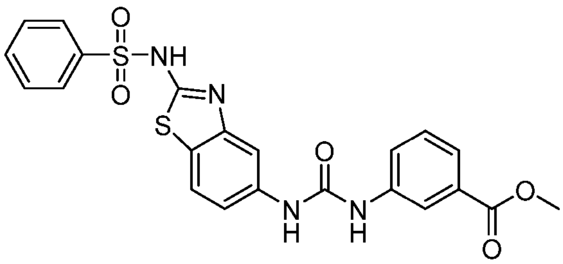

$^1H$ NMR (400 MHz, DMSOd$_6$) δ 13.01 (s, 1H), 8.84 (d, J = 7.6 Hz, 2H), 8.16 (s, 1H), 7.85 (m, 4H), 7.57 (m, 5H), 7.38 (t, d = 7.6 Hz, 1H), 7.08 (d, J = 7.6 Hz, 1H), 3.90 (s, 3H)

11) NIC7k: 4-methyl-N-(5-(3-(4-methylbenzyl)ureido)benzo[*d*]thiazol-2-yl)benzenesulfonamide

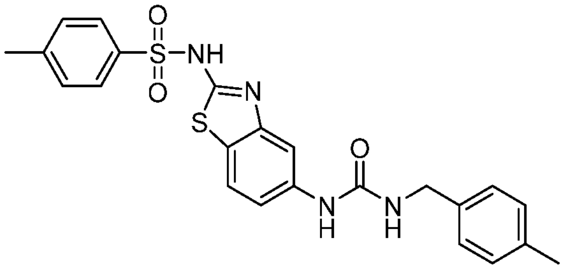

$^1H$ NMR (400 MHz, $DMSOd_6$) δ 12.91(s, 1H), 8.69 (s, 1H), 7.77 (s, 1H), 7.74 (d, J = 6.4 Hz, 2H), 7.48 (d, J = 6.8 Hz, 1H), 7.32 (d, J = 6.4 Hz, 2H), 7.14 (m, 4H), 6.99 (d, J = 6.4 Hz, 1H), 6.48 (m, 1H), 4.25 (s, 2H), 2.40 (s, 3H), 2.31 (2, 3H)

12) NIC7l: N-(5-(3-(benzo[*d*][1,3]dioxol-5-yl)ureido)benzo[d]thiazol-2-yl)-4-methylbenzenesulfonamide

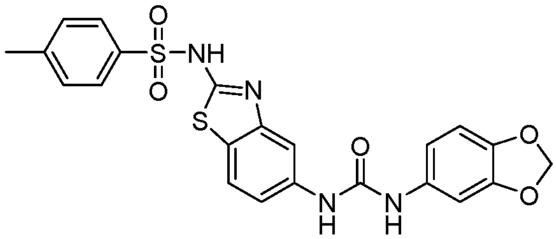

$^1H$ NMR (400 MHz, $DMSOd_6$) δ 12.95 (s, 1H), 8.76 (s, 1H), 8.45 (s, 1H), 7.79 (s, 1H), 7.74 (d, J = 6.4 Hz, 2H), 7.54 (d, J = 6.4 Hz, 1H), 7.32 (d, J = 6.4 Hz, 2H), 7.18 (s, 1H), 7.03 (m, 1H), 6.74 (m, 2H), 5.95 (s, 2H), 2.40 (s, 3H)

13) NIC7m: N-(5-(3-(2-methoxy-5-methylphenyl)ureido)benzo[*d*]thiazol-2-yl)-4-methylbenzenesulfonamide

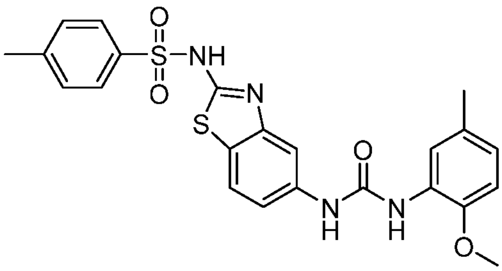

$^1H$ NMR (400 MHz, $DMSOd_6$) δ 12.98 (s, 1H), 9.45 (s, 1H), 8.08 (s, 1H), 7.96 (s, 1H), 7.81 (d, J = 1.6 Hz, 1H), 7.74 (d, J = 7.4 Hz, 2H), 7.55 (d, J = 7.4 Hz, 1H), 7.33 (d, J = 7.4 Hz, 2H), 7.04 (d, J = 6.8 Hz, 1H), 6.83 (d, J = 6.8 Hz, 1H), 6.71 (m, 1H), 3.88 (s, 3H), 2.40 (s, 3H), 2.28 (s, 3H)

14) NIC7n: N-(5-(3-(3,5-dimethoxyphenyl)ureido)benzo[d]thiazol-2-yl)-4-methylbenzenesulfonamide

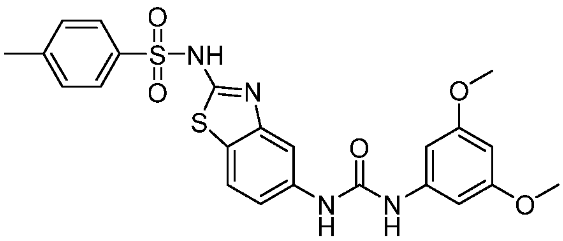

$^1H$ NMR (400 MHz, $DMSOd_6$) δ 12.92 (s, 1H), 8.79 (s, 1H), 8.55 (s, 1H), 7.81 (s, 1H), 7.74 (d, J = 6.8 Hz, 2H), 7.56 (d, J = 7.2 Hz, 1H), 7.34 (d, J = 6.8 Hz, 2H), 7.04 (d, J = 7.2 Hz, 1H), 6.64 (s, 2H), 6.10 (s, 1H), 3.77 (s, 6H), 2.40 (s, 3H)

15) NIC7o: N-(5-(3-(3-chloro-4-fluorophenyl)ureido)benzo[d]thiazol-2-yl)-4-methylbenzenesulfonamide

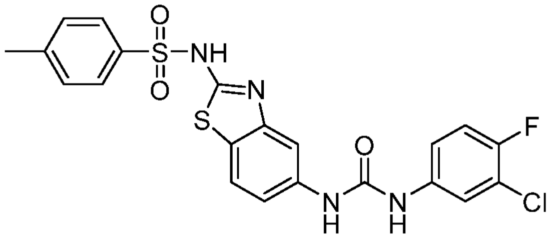

$^1H$ NMR (400 MHz, $DMSOd_6$) δ 12.97 (s, 1H), 8.89 (s, 1H), 8.75 (s, 1H), 7.74 (m, 4H), 7.56 (d, J = 7.2 Hz, 2H), 7.32 (d, J = 7.2 Hz, 2H), 7.27 (m, 1H), 7.22 (t, J = 7.2 Hz, 1H), 7.07 (dd, J = 7.2, 1.6 Hz, 1H), 2.40 (s, 3H)

16) NIC7p: 4-methyl-N-(5-(3-(p-tolyl)ureido)benzo[*d*]thiazol-2-yl)benzenesulfonamide

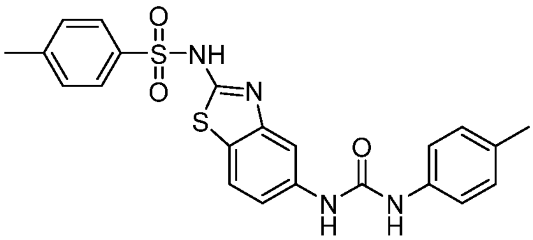

$^1H$ NMR (400 MHz, $DMSOd_6$) δ 12.95 (s, 1H), 8.77 (s, 1H), 8.43 (s, 1H), 7.80 (s, 1H), 7.74 (d, J = 6.8 Hz, 2H), 7.54 (d, J = 6.8 Hz, 1H), 7.32 (m, 4H), 7.05 (m, 3H), 2.40 (s, 3H), 2.29 (s, 3H)

17) NIC7q: N-(5-(3-(2-methoxyethyl)ureido)benzo[*d*]thiazol-2-yl)-4-methylbenzenesulfonamide

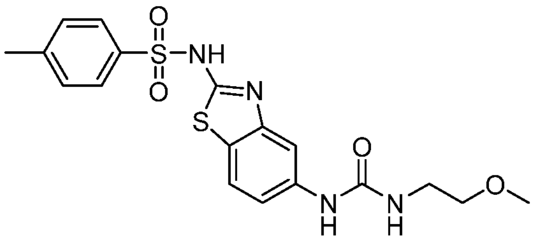

$^1H$ NMR (400 MHz, $DMSOd_6$) δ 12.86 (s, 1H), 8.67 (s, 1H), 7.73 (m, 3H), 7.47 (d, J = 6.8 Hz, 1H), 7.32 (d, J = 7.2 Hz, 2H), 6.96 (m, 1H), 6.15 (m, 1H), 3.42 (m, 2H), 3.31 (s, 3H), 3.24 (m, 2H), 2.38 (s, 3H)

18) NIC7r: N-(5-(3-(2,4-dimethoxyphenyl)ureido)benzo[*d*]thiazol-2-yl)-4-methylbenzenesulfonamide

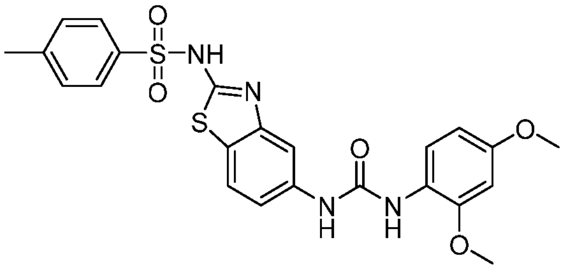

$^1H$ NMR (400 MHz, $DMSOd_6$) δ 12.91 (s, 1H), (9.32 (s, 1H), 7.89 (m, 2H), 7.80 (s, 1H), 7.73 (d, J = 6.8 Hz, 2H), 7.53 (d, J = 7.2 Hz, 1H), 7.32 (d, J = 6.8 Hz, 2H), 7.03 (d, J = 7.2 Hz, 1H), 6.58 (s, 1H), 6.42 (d, J = 7.2 Hz, 1H), 3.87 (s, 3H), 3.76 (s, 3H), 2.40 (s, 3H)

19) NIC7s: N-(5-(3-(2,5-dimethoxyphenyl)ureido)benzo[d]thiazol-2-yl)-4-methylbenzenesulfonamide

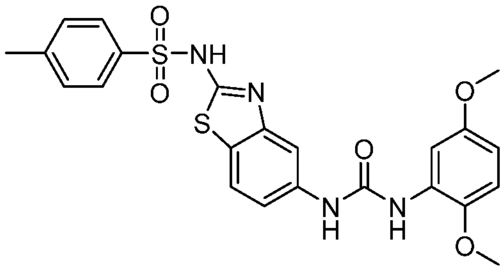

$^1H$ NMR (400 MHz, $DMSOd_6$) δ 12.91 (s, 1H), 9.48 (s, 1H), 8.17 (s, 1H), 7.83 (m, 1H), 7.73 (d, J = 6.8 Hz, 2H), 7.56 (d, J = 7.2 Hz, 1H), 7.33 (d, J = 6.8 Hz, 2H), 7.04 (d, J = 5.6 Hz, 1H), 6.86 (d, J = 7.2 Hz, 1H), 6.45 (d, J = 5.6 Hz, 1H), 3.87 (s, 3H), 3.72 (s, 3H), 2.39 (s, 3H)

20) NIC7t: N-(5-(3-(3-methoxyphenyl)ureido)benzo[d]thiazol-2-yl)-4-methylbenzenesulfonamide

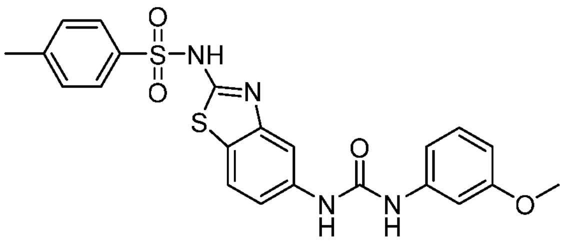

$^1H$ NMR (400 MHz, $DMSOd_6$) δ 12.90 (s. 1H), 8.84 (s, 1H), 8.58 (s, 1H), 7.80 (s, 1H), 7.73 (d, J = 6.8 Hz, 2H), 7.54 (d, J = 7.2 Hz, 1H), 7.33 (d, J = 6.8 Hz, 2H), 7.15 (m, 2H), 7.04 (d, J = 5.6 Hz, 1H), 6.88 (d, J = 5.6 Hz, 1H), 6.50 (d, J = 5.6 Hz, 1H), 3.76 (s, 3H), 2.40 (s, 3H)

21) NIC7u: N-(5-(3-(4-ethoxyphenyl)ureido)benzo[d]thiazol-2-yl)-4-methylbenzenesulfonamide

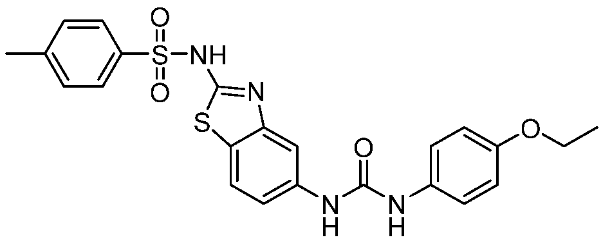

$^{1}$H NMR (400 MHz, DMSOd$_6$) δ 12.91 (s, 1H), 8.73 (s, 1H), 8.35 (s, 1H), 7.79 (s, 1H), 7.73 (d, J = 6.8 Hz, 2H), 7.54 (d, J = 7.2 Hz, 1H), 7.32 (m, 4H), 7.04 (d, J = 7.2 Hz, 1H), 6.80 (d, J = 6.8 Hz, 2H), 3.98 (q, J = 8.4 Hz, 2H), 2.40 (s, 3H), 1.32 (t, J = 8.4 Hz, 3H)

22) NIC7v: N-(5-(3-(4-fluorobenzyl)ureido)benzo[d]thiazol-2-yl)benzenesulfonamide

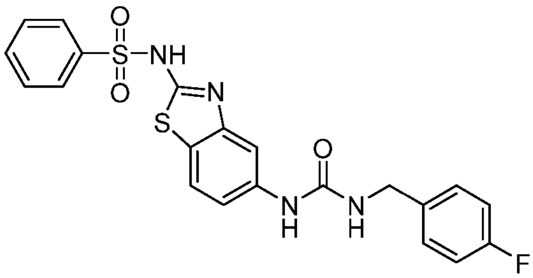

$^{1}$H NMR (400 MHz, DMSOd$_6$) δ 12.91 (s, 1H), 8.74 (s, 1H), 7.83 (d, J = 7.2 Hz, 1H), 7.76 (s, 1H), 7.54 (m, 4H), 7.32 (m, 2H), 7.08 (t, J = 7.2 Hz, 2H), 7.01 (d, J = 7.2 Hz, 1H), 6.56 (m, 1H), 4.28 (m, 2H)

23) NIC7w: N-(5-(3-benzylureido)benzo[*d*]thiazol-2-yl)-4-methylbenzenesulfonamide

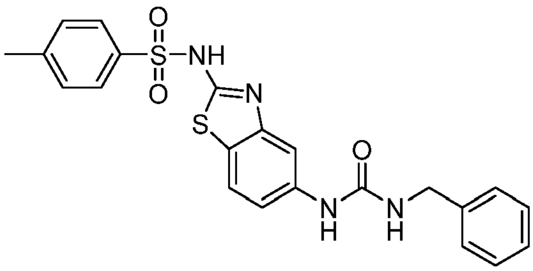

$^{1}$H NMR (400 MHz, DMSOd$_6$) δ 12.99 (s, 1H), 8.82 (s, 1H), 7.72 (m, 3H), 7.62 (d, J = 7.2 Hz, 1H), 7.33 (m, 7H), 7.05 (d, J = 7.2 Hz, 1H), 6.66 (m, 1H), 4.28 (m, 2H), 2.40 (s, 3H)

24) NIC7x: N-(5-(3-(3,4-dimethoxyphenyl)ureido)benzo[d]thiazol-2-yl)benzenesulfonamide

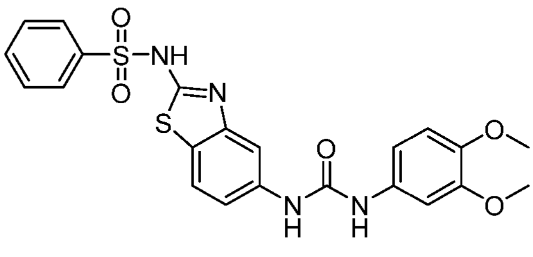

$^1H$ NMR (400 MHz, $DMSOd_6$) δ 12.97 (s, 1H), 8.72 (s, 1H), 8.39 (s, 1H), 7.86 (d, J = 6.8 Hz, 2H), 7.82 (s, 1H), 7.56 (m, 4H), 7.21 (s, 1H), 7.04 (d, J = 6.8 Hz, 1H), 6.80 (s, 2H), 3.78 (s, 3H), 3.73 (s, 3H)

25) NIC7y: N-(5-(3-(3-ethoxypropyl)ureido)benzo[*d*]thiazol-2-yl)benzenesulfonamide

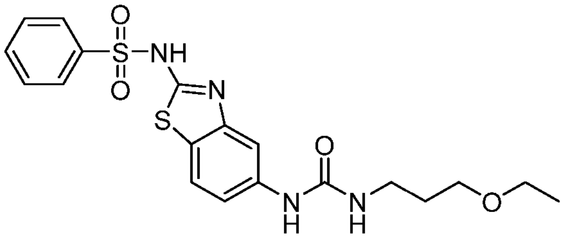

$^1H$ NMR (400 MHz, $DMSOd_6$) δ 12.95 (s, 1H), 8.61 (s, 1H), 7.84 (d, J = 7.2 Hz, 2H), 7.73 (s, 1H), 7.54 (m, 4H), 6.95 (d, J = 7.2 Hz, 1H), 3.44 (q, J = 8.0 Hz, 2H), 3.15 (m, 4H), 1.68 (m, 2H), 1.17 (t, J = 8.0 Hz, 3H)

**Example 3: Identification of NIC7 as an anti-NLRP3 lead**

To date, an experimental crystal structure of NLRP3 bound to a small-molecule ligand has not been available in the Protein Data Bank (PDB). Identification of a novel small-molecule inhibitor of NLRP3 (NIC7) was carried out via an *in silico* approach using the cryo-electron microscopy (EM) structure of the NLRP3-NEK7 complex (PDB ID: 6NPY) (H. Sharif et al., Nature 570, 338-343 (2019)) and a multifunctional library of over 28 million commercially available compounds (Table 3 and FIG. 1).

Compounds with drug-like physicochemical properties were isolated using a fingerprint-based similarity metric (Tanimoto coefficient; TC) with the selected NLRP3 inhibitors as reference molecules (Table 4). The resulting set of 367,328 ligands was screened via a pharmacophore model of reference ligands with well-defined intermolecular interactions with NLRP3. Next, the resultant set of 70,040 ligands was subjected to structure-based virtual screening on sites I and II of NLRP3. The docked ligand poses were ranked by the MMFF94x force field-based binding free energy (GBVI/WSA dG) scores. After a close visual inspection of the top about 200 virtual hits for potential fit into the site I and site II pockets of NLRP3 (FIG. 2), the top-scoring 19 consensus ligands (designated as "NIC1-NIC19") were experimentally validated to evaluate their inhibitory activity.

Before studying the antagonistic activity of the selected compounds, cell viability was evaluated using MTT (1-(4,5-dimethylthiazol-2-yl)-3,5-diphenylformazan) assay. Human monocyte cells (THP-1) were differentiated into macrophages by treatment with phorbol 12-myristate 13-acetate (PMA) for 24 hours, and then treated with 10 or 50 μM of each of the compounds to be screened (NIC1-NIC19) for an additional 24 hours. Among the 19 compounds, NIC1, NIC2, NIC6, NIC14, and NIC17 showed significant toxicity at higher concentrations (FIGS. 3a and 3b). The remaining compounds were tested for their functional activity in terms of inhibiting interleukin-1β (IL-1β) secretion caused by NLRP3. To this end, PMA-differentiated THP-1 cells were primed by activating the Toll-like receptor 4 (TLR4) signaling pathway with its ligand lipopolysaccharide (LPS), and treated with each compound together with MCC950 as a positive control, and then NLRP3 was activated with nigericin. Among all compounds tested, NIC7 showed the highest potency (FIG. 3c). Therefore, NIC7 was assumed as an appropriate lead compound for further optimization to enhance its inhibitory potency against NLRP3.

**Example 4: NIC7w exhibiting increased potency against NLRP3**

The present inventors attempted to improve the inhibitory effect of the primary lead NIC7 against the NLRP3 signaling pathway by structural modifications. The present inventors searched for commercially available NIC7 derivatives in the MolPort database (https://www.molport.com/shop/index) and identified 100 promising distinct derivatives structurally similar to the NIC7 main scaffold with various functional groups attached. The derivatives were first computationally docked to site I and site II of NLRP3 using the docked pose of NIC7 as a template and ranked based on their binding affinity score. The top 25 derivatives (referred to as "NIC7a-NIC7y"), which scored higher than NIC7 and showed maximal interactions within 4.5 Å were selected to test their NLRP3 blocking capability via an enzyme-linked immunosorbent assay (ELISA).

All the derivatives were first evaluated for their toxicity to THP-1 macrophages at the above-mentioned concentrations (10 and 50 μM). None of the molecules showed significant toxicity to the cells at the highest concentrations

used (FIGS. 4a and 4b). Therefore, all the compounds were subjected to screening to determine their inhibitory potential against NLRP3. According to the same procedure as described above, the secretion level of human IL-1β was analyzed using ELISA. NIC7w showed the most potent inhibition at the highest concentration of the compound (FIG. 5c). To further analyze inhibition of the NLRP3 signaling pathway using Western blot analysis, NIC7w was selected along with NIC7 and MCC950 (control). The present inventors noted inhibition of key components of the NLRP3 signaling pathway, i.e., IL-1β and caspase 1 (FIG. 5d).

**Example 5: Evaluation of memory impairment prevention effect of NIC7 in AD model**

NLRP3 has been reported to be associated with various hippocampal neurodegenerative diseases including Alzheimer's disease (R. H. Pirzada, et al., Genes (Basel) 11 (2020)).

The present inventors evaluated recovery of memory decline by NIC7, an NLRP3 inflammasome inhibitor. In this regard, spatial working memory was examined by the Y-maze test on an AD mouse model, and the spontaneous alternation rate was calculated by using the total arm entries. As a result, it was confirmed that, compared to vehicle-treated and untreated mice, NIC7-treated mice spent more time in the novel arm as did donepezil-treated mice (FIGS. 6a and 6b). Considering that the hippocampus is involved in recognition memory in addition to spatial working memory (N. J. Broadbent et al., Proc Natl Acad Sci USA 101, 14515-14520 (2004)), a novel object recognition test was also performed. Similar to the above results, it was confirmed that the recognition index rate was better in the NIC7- and donepezil-treated mice than in the vehicle-treated or untreated mice (FIG. 6c).

In addition, as a result of body weight analysis in all the groups, it was confirmed that the body weight significantly increased in the NIC7-treated mice (almost the same as that in normal mice) compared to the untreated mice (FIG. 6d). Overall, these results indicate the ability of NIC7 to improve mouse cognitive behavior in the AD models. Metabolic stability (and cytochrome P450 inhibition) of NIC7, NIC7w and MCC950 were also evaluated. To evaluate microsomal stability and CYP450 inhibition, MCC950, NIC7, and NIC7w were separately incubated with liver microsomes, and the products were analyzed by LC-MS/MS.

The metabolic stability assay determines the temporal intrinsic clearance of a test compound by incubation with microsomes or hepatocytes (L. E. Chovan, et al., Rapid Commun Mass Spectrom 18, 3105-3112 (2004)). In the case of NIC7w, the amount of the drug remaining after 30 minutes of metabolism increased by 29.8% compared to that of NIC7, indicating the improvement in metabolic stability (Table 5).

[Table 5] Pharmacokinetic analysis

| | | **NIC7** | **NIC7w** |
|---|---|---|---|
| **Pharmacokinetics** | **Microsomal stability** (% at 30 min) | Human: 19.7 % | Human: 49.5% |
| | **CYP inhibition** (% at 10μM) | 1A2: < 1%<br>2C9: 34.2%<br>2C19: < 1%<br>2D6: 9.8%<br>3A4: < 1% | 1A2: < 1%<br>2C9: 39.4%<br>2C19: 15.4%<br>2D6: 9.1%<br>3A4: < 1% |

Similarly, the cytochrome P450 inhibition assay is used to determine drug interactions by assessing inhibition of cytochrome P450 enzymes (S. A. Testino, Jr., G. Patonay, J Pharm Biomed Anal 30, 1459-1467 (2003)). In five enzymes of the P450 system, including CYP1A2, CYP2C9, CYP2C19, CYP2D6 and CYP3A4, NIC7 and NIC7w were tested along with the associated substrates and concentrations listed in Table 2 above. It was confirmed that both NIC7 and NIC7w acted mainly on CYP2C9 enzyme and hardly acted on other types of CYP enzymes, demonstrating the pharmacological properties of the two compounds (Table 5).

**Example 6: Binding mode of NIC7 and NIC7w**

NIC7, the initial lead, and its chemical derivatives contain sulfonamide and urea moieties (FIG. 7), suggesting that they are important functional groups commonly found in NLRP3 inflammasome inhibitors and antidiabetic drugs (S. Seino, Diabetologia 55, 2096-2108 (2012)). NIC7w, the most effective of all the derivatives, contains a 1-benzyl group accompanied by a urea group attached to the 3-benzothiazol-5-yl moiety (FIG. 8), which ensures a stronger inhibitory activity against the NLRP3 inflammasome. *In silico* molecular docking suggested that NIC7 and NIC7w occupied helical domain 2 (HD2) of NLRP3, showing an analogous binding site. Closer analysis of residues around 5 Å of the potent ligand showed that the intermolecular interactions involved similar and distinct amino acids of LRR and HD2 of NLRP3 (FIG. 9).

The interaction of NIC7 showed that the central benzothiazole moiety was stabilized by the charged amino acid

residues R697, E637, E638 and D646 (FIG. 9a). It was found that the nitrogen atom of the urea group formed a hydrogen bond with the side chain of the carboxyl group of E695. Moreover, methylbenzenesulfonamide was engaged by the hydrophobic residues L699 and G696 and the polar residue H698. The acetylphenyl groups are located inside the buried surfaces of the hydrophobic residues L628, L632, F648, M685 and P649. In addition, several other charged, polar and nonpolar residues may stabilize the ligand through van der Waals and salt bridge interactions.

It was found that the benzothiazole moiety in NIC7w forms charged contacts with E695 and R697 and electrostatic interactions with H698, P707 and S721. The methylbenzenesulfonamide moiety is stacked between charged residues E637, E638, E695, D639 and R697 (FIG. 9b). A hydrogen bond is formed between the oxygen atom of the sulfonamide group and the amino group of E638. Benzyl and urea groups interact closely with K694, N720, E743, R772 and W774 and may provide more stability. Additionally, residues L632, Q636, M645, E693, H722 and D745 are found within a 5 Å radius around NIC7w, supporting its stability through electrostatic and van der Waals interactions.

## Industrial Applicability

Dysregulation of the NLRP3 inflammasome is associated with complex diseases such as Alzheimer's disease (AD), type 2 diabetes and cryopyrin-associated periodic syndrome (CAPS). Nevertheless, oral NLRP3 inflammasome inhibitory small-molecules have not yet been successful as therapeutic agents for the treatment of Alzheimer's disease in human clinical practice. In the present disclosure, NIC7 ($C_{23}H_{20}N_4O_4S_2$) and its derivative NIC7w ($C_{22}H_20N_4O_3S_2$), which are NLRP3 inflammasome inhibitory compounds, were identified. NIC7 and NIC7w inhibited IL-1β and caspase 1, which are key components of NLRP3 inflammasome signaling, and exhibited improvement in mouse cognitive behavior in an AD mouse model. Consequently, these *in vivo* data suggest that the small-molecule NLRP3 inflammasome inhibitors are effective therapeutic agents for AD.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims.

## Claims

1. A compound represented by any one formula selected from the group consisting of the following Formulas 1-1 to 1-12, or a pharmaceutically acceptable salt thereof:

[Formula 1-1]

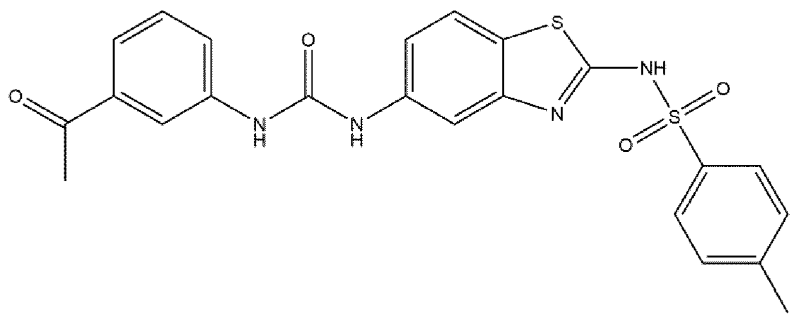

;

[Formula 1-2]

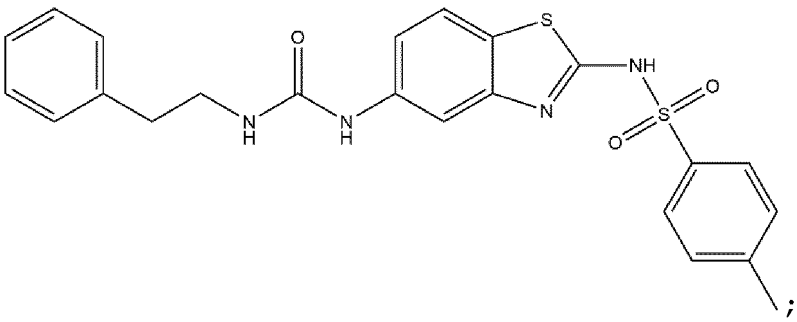

[Formula 1-3]

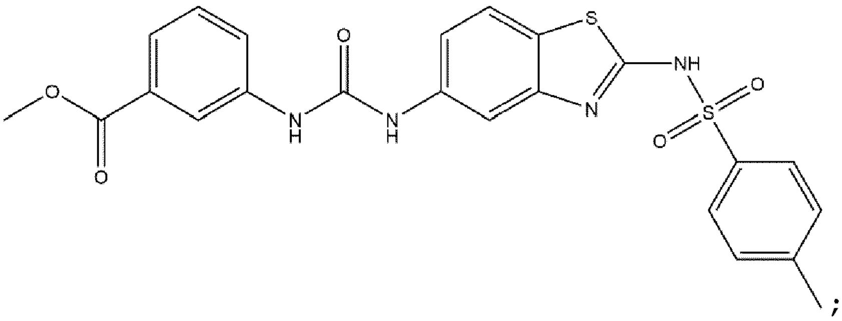

[Formula 1-4]

90

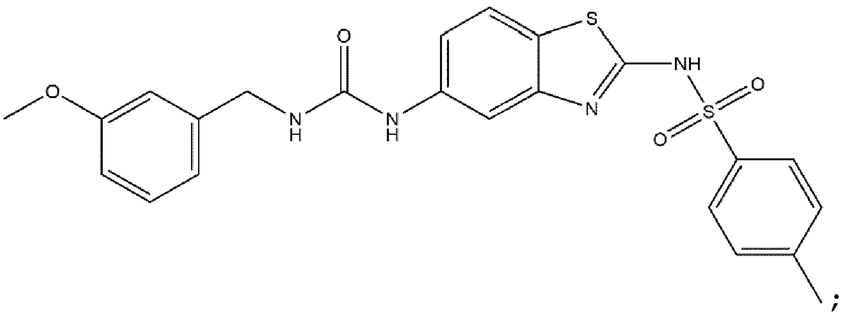

[Formula 1-5]

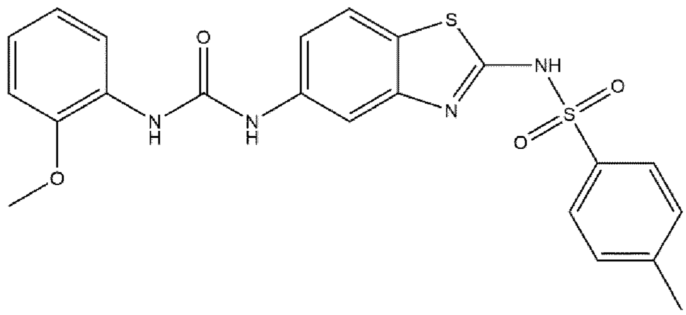

;

[Formula 1-6]

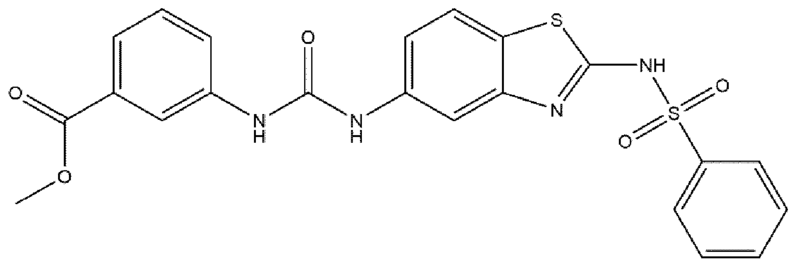

;

[Formula 1-7]

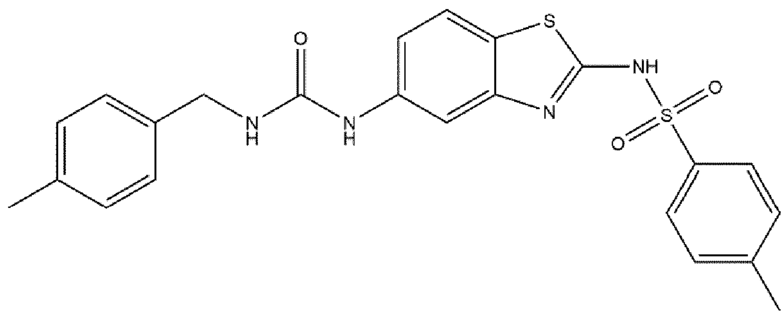

;

[Formula 1-8]

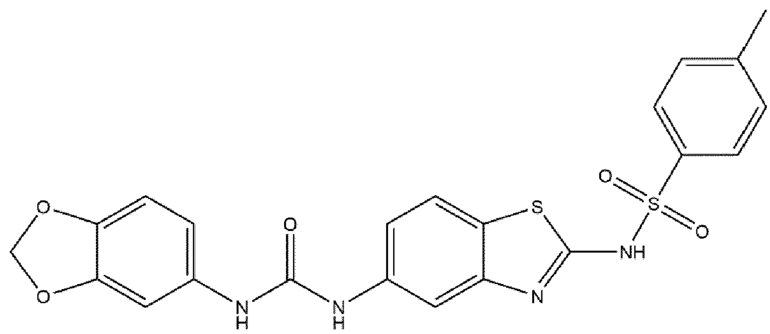

;

[Formula 1-9]

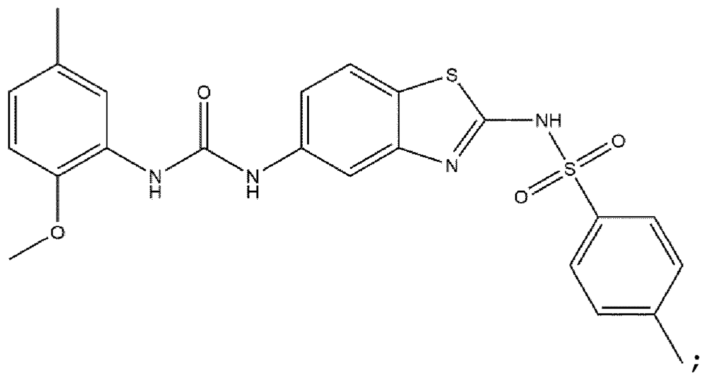

[Formula 1-10]

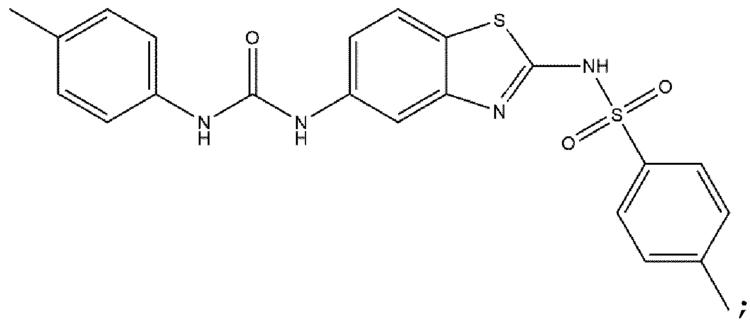

[Formula 1-11]

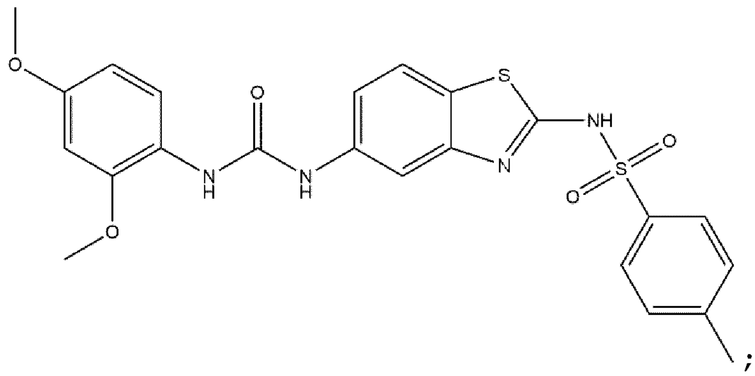

and

[Formula 1-12]

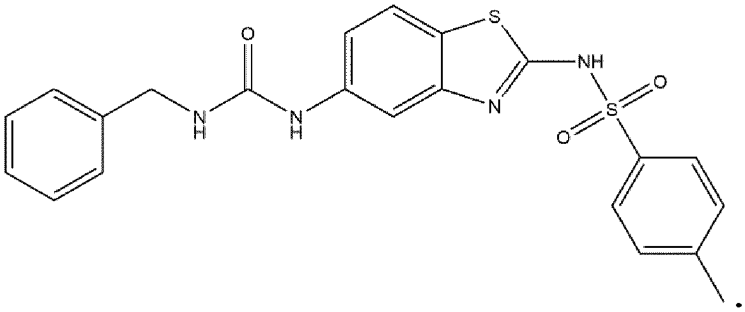

2. A compound represented by the following Formula 1, or a pharmaceutically acceptable salt thereof, for use in a method of inhibiting NLRP3 inflammasome activity:

[Formula 1]

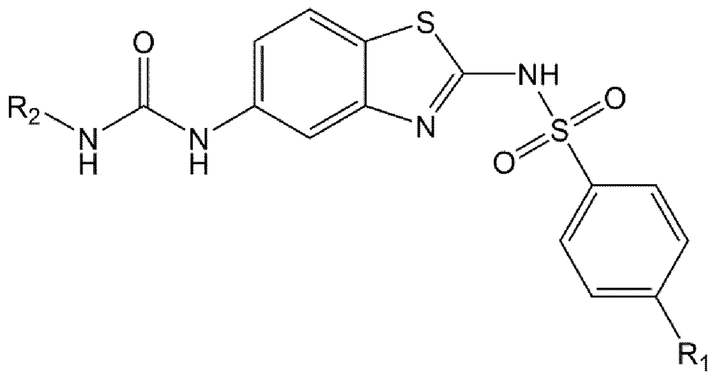

wherein $R_1$ and $R_2$ are the same as or different from each other and each independently selected from the group consisting of a hydrogen atom, a halogen atom, a substituted or unsubstituted hydrocarbon group, a substituted or unsubstituted heterogeneous group, a substituted or unsubstituted carbocyclic group, a substituted or unsubstituted heterocyclic group, a substituted or unsubstituted aromatic group, and a substituted or unsubstituted heteroaromatic group.

3. A composition for use in a method of preventing or treating NLRP3 inflammasome-related inflammatory disease, the composition comprising a compound represented by the following Formula 1, or a pharmaceutically acceptable salt thereof:

[Formula 1]

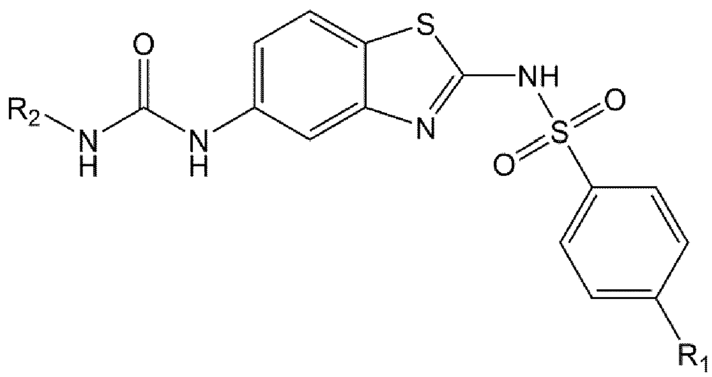

wherein $R_1$ and $R_2$ are the same as or different from each other and each independently selected from the group consisting of a hydrogen atom, a halogen atom, a substituted or unsubstituted hydrocarbon group, a substituted or unsubstituted heterogeneous group, a substituted or unsubstituted carbocyclic group, a substituted or unsubstituted heterocyclic group, a substituted or unsubstituted aromatic group, and a substituted or unsubstituted heteroaromatic group.

**4.** The composition for use according to claim 3, wherein the NLRP3 inflammasome-related inflammatory disease is a metabolic disease, a neuroinflammatory disease, or an autoinflammatory disease.

**5.** The composition for use according to claim 4, wherein the metabolic disease is obesity, hyperlipidemia, hypercholesterolemia, arteriosclerosis, type 2 diabetes, non-alcoholic fatty liver (NAFLD), or non-alcoholic steatohepatitis (NASH).

**6.** The composition for use according to claim 4, wherein the neuroinflammatory disease is Alzheimer's disease, Parkinson's disease, Huntington's disease, Lou Gehrig's disease, Creutzfeldt-Jakob disease, multiple sclerosis, amyotrophic lateral sclerosis, diffuse Lewy body disease, leukoencephalitis, temporal lobe epilepsy, or inflammatory spinal cord injury.

**7.** The composition for use according to claim 4, wherein the autoinflammatory disease is Muckle-Wells syndrome (MWS), adult latent autoimmune diabetes (LADA), familial cold autoinflammatory syndrome (FCAS), cryopyrin-associated periodic syndrome (CAPS), neonatal-onset multisystem inflammatory syndrome (NOMID), chronic infantile neurological cutaneous and articular (CINCA) syndrome, familial Mediterranean fever (FMF), systemic onset juvenile idiopathic arthritis (SJIA), systemic onset juvenile idiopathic rheumatoid arthritis, or rheumatoid arthritis.

**8.** The compound for use according to claim 2 or the composition for use according to claim 3, wherein $R_1$ and $R_2$ are substituents selected from the following groups:

$R_1$: a hydrogen atom or methyl; and
$R_2$:

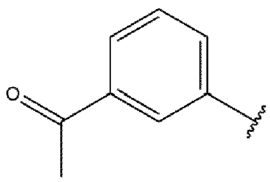 , 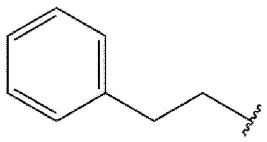 , 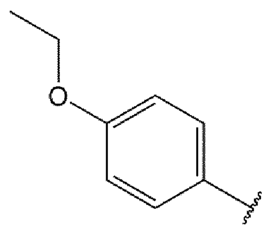 ,

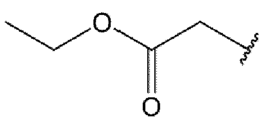 , 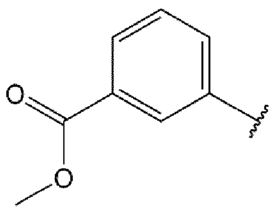 , 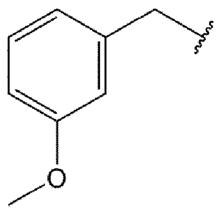 , 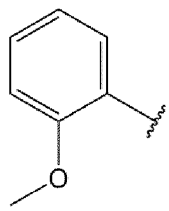 ,

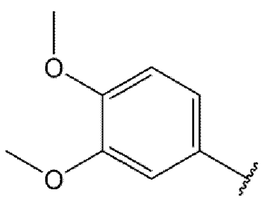 , 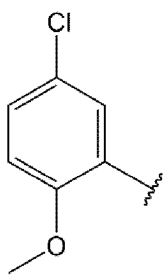 , 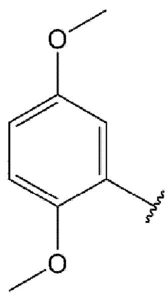 , 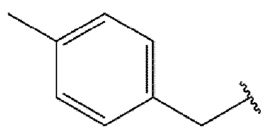 ,

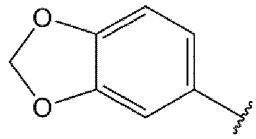 , 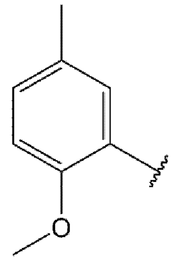 , 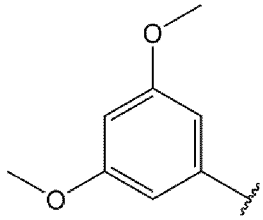 , 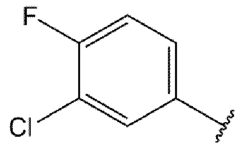 ,

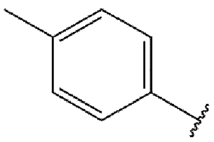 , 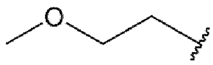 , 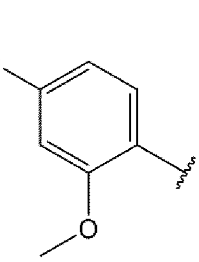 , 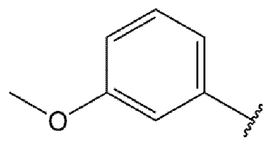 ,

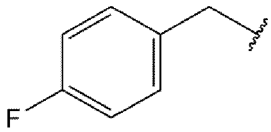 , 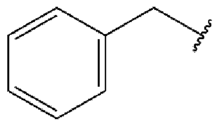 or 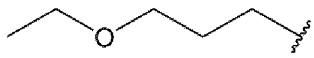 .

9. The compound for use according to claim 2 or the composition for use according to claim 3, wherein the compound represented by Formula 1 is a compound represented by any one formula selected from the group consisting of the following Formulas 1-1 to 1-12:

[Formula 1-1]

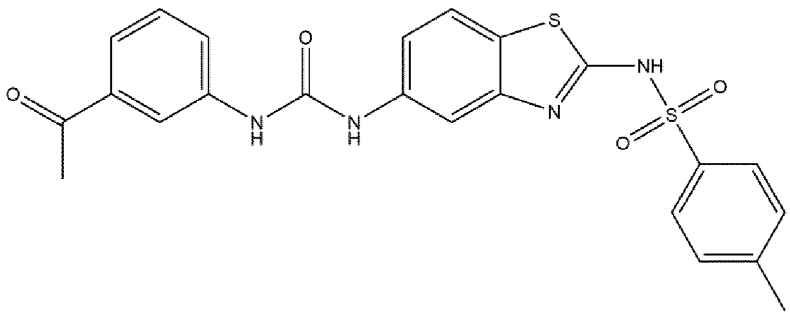 ;

[Formula 1-2]

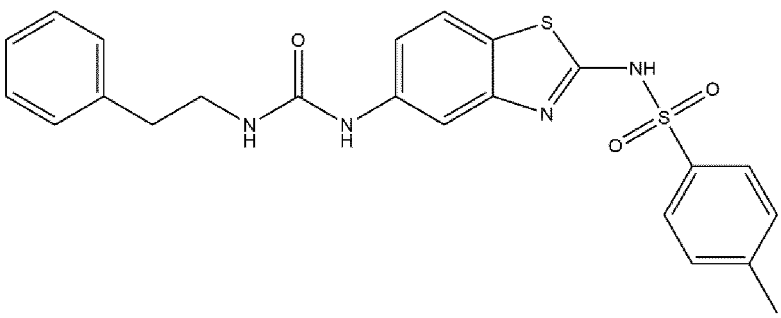 ;

[Formula 1-3]

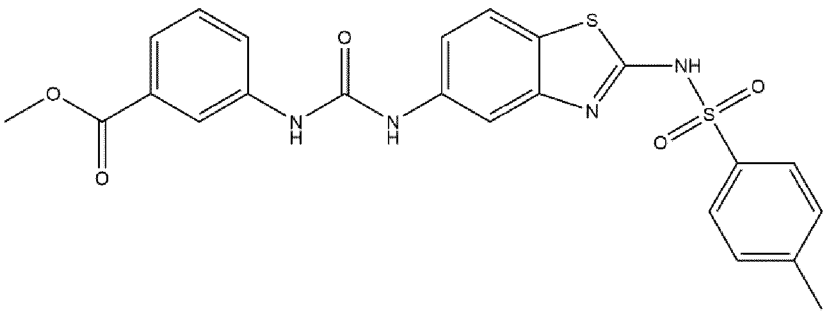

[Formula 1-4]

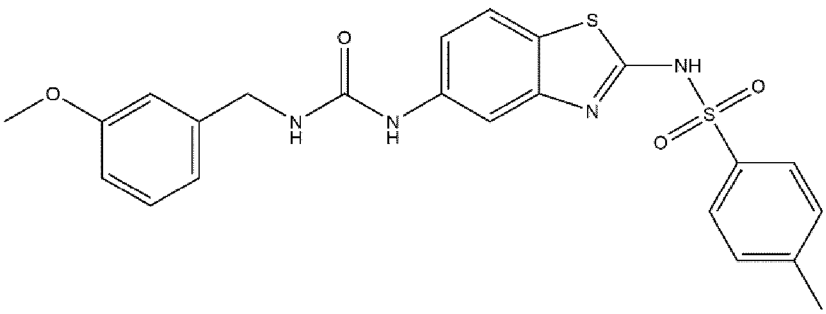

[Formula 1-5]

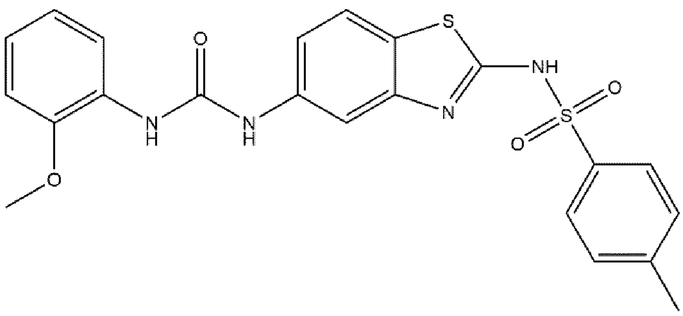

[Formula 1-6]

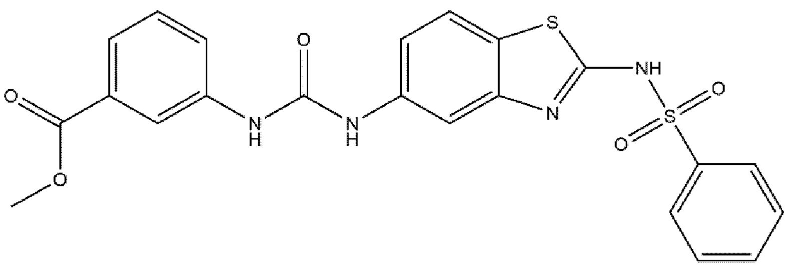

[Formula 1-7]

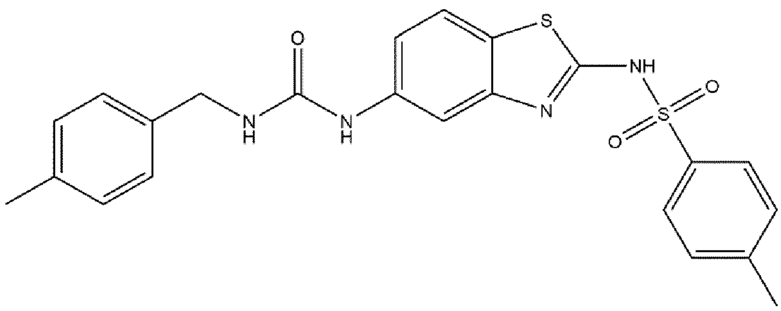

;

[Formula 1-8]

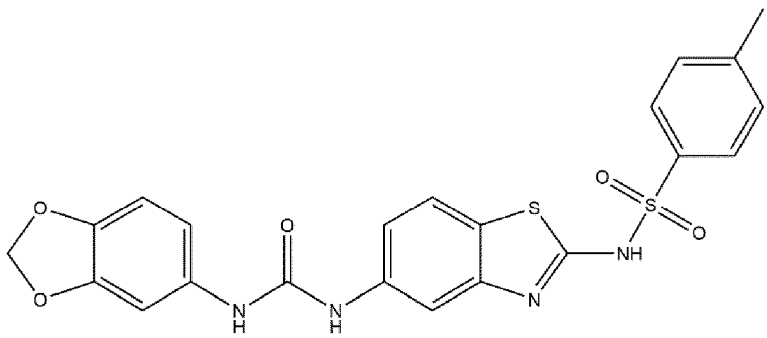

;

[Formula 1-9]

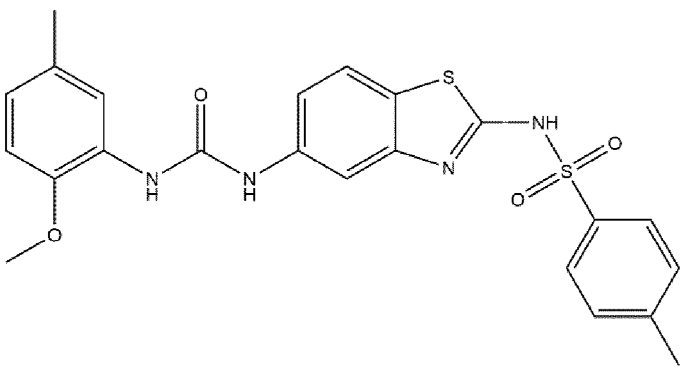

;

[Formula 1-10]

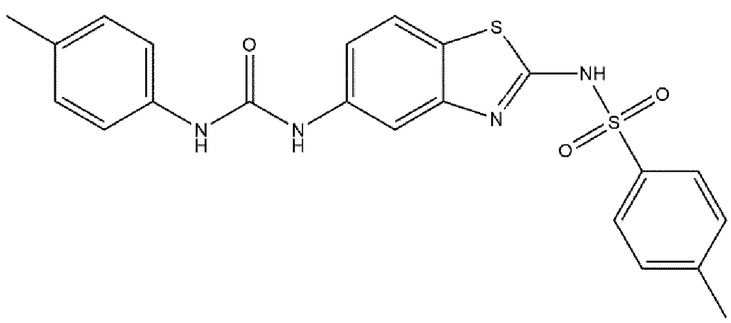

;

[Formula 1-11]

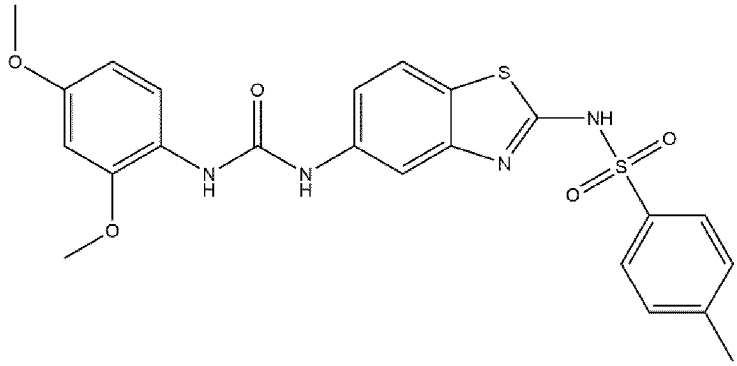
;

and

[Formula 1-12]

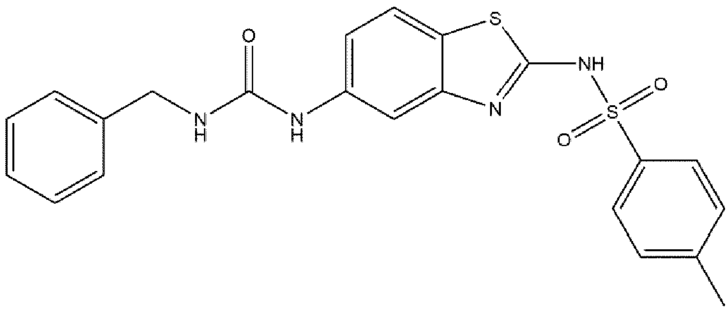
.

10. The composition for use according to any one of claims 3 to 9, wherein the composition is in the form of a food composition.

**Patentansprüche**

1. Verbindung, die durch eine aus der aus den folgenden Formeln 1-1 bis 1-12 bestehenden Gruppe ausgewählte Formel dargestellt ist, oder ein pharmazeutisch annehmbares Salz davon:

[Formel 1-1]

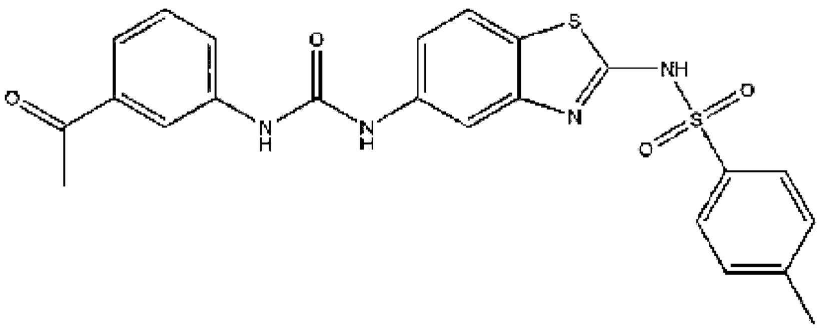
;

[Formel 1-2]

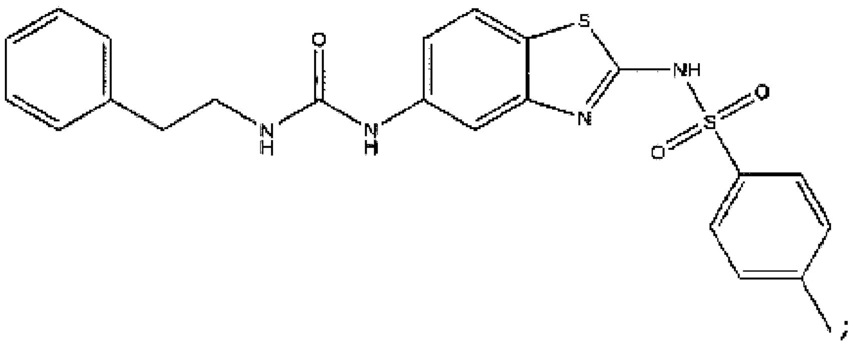

[Formel 1-3]

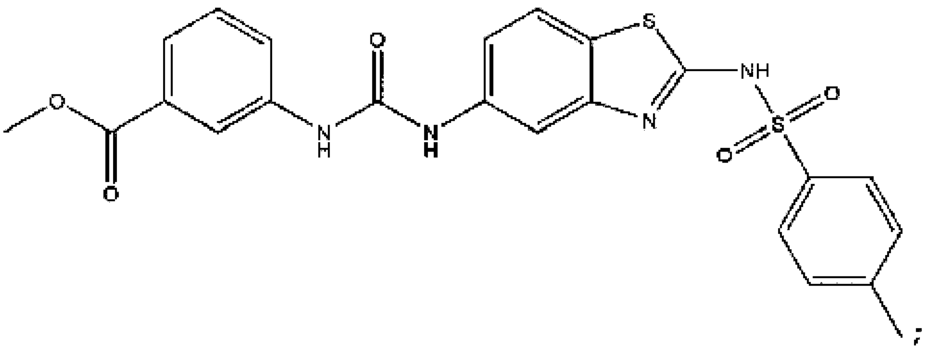

[Formel 1-4]

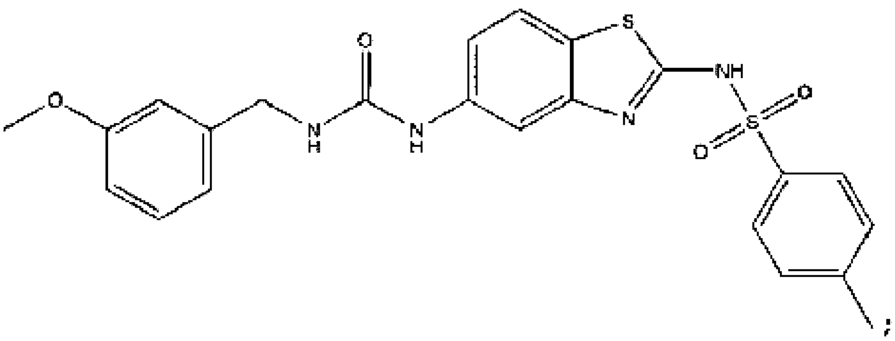

[Formel 1-5]

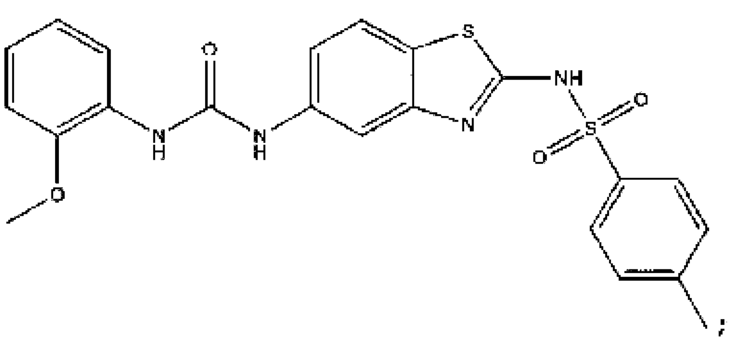

[Formel 1-6]

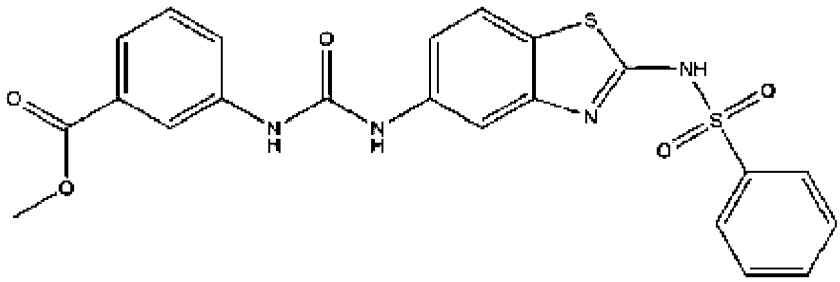

;

[Formel 1-7]

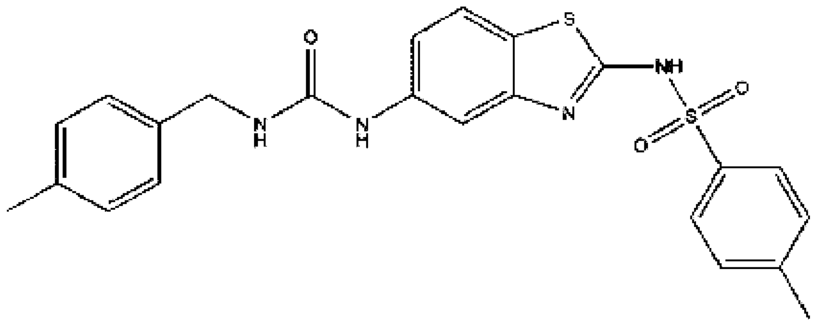

;

[Formel 1-8]

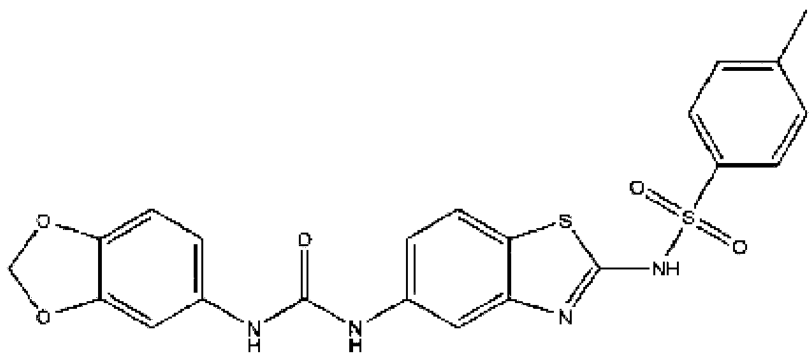

;

[Formel 1-9]

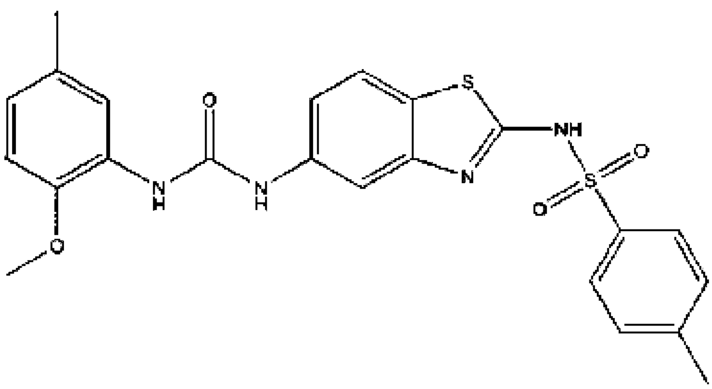

;

[Formel 1-10]

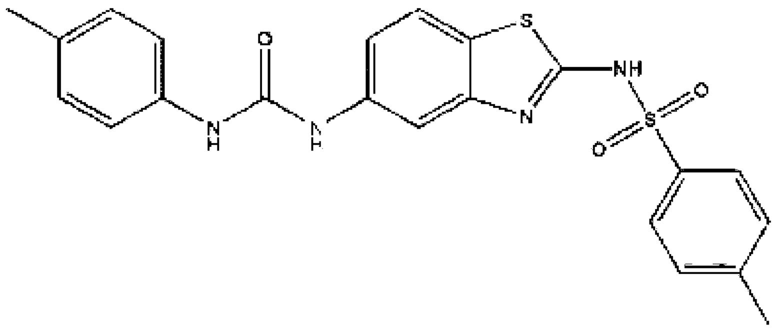
;

[Formel 1-11]

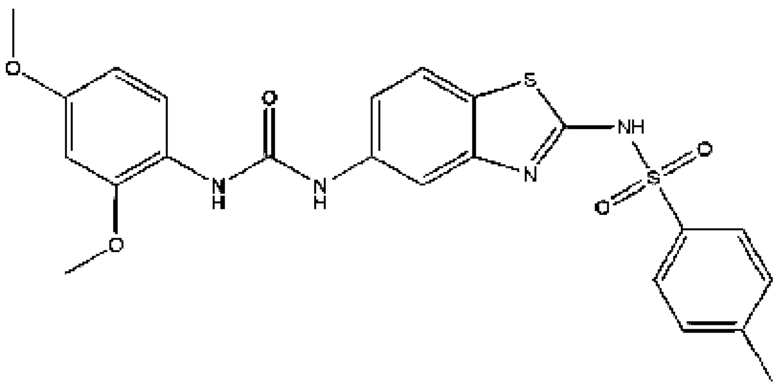

und

[Formel 1-12]

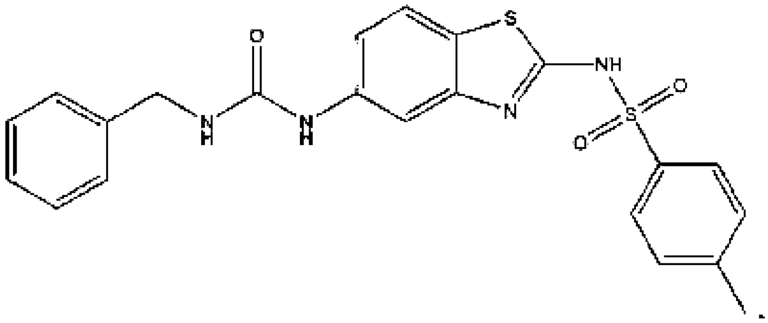

2. Verbindung, die durch die folgende Formel 1 dargestellt ist, oder ein pharmazeutisch annehmbares Salz davon zur Verwendung in einem Verfahren zur Hemmung von NLRP3-Inflammasom-Aktivität:

[Formel 1]

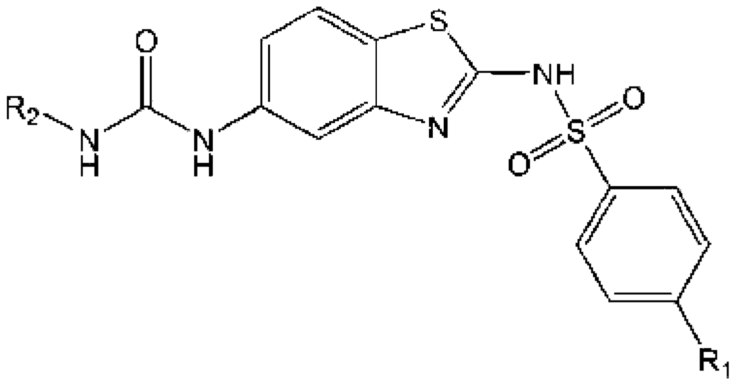

worin $R_1$ und $R_2$ gleich oder unterschiedlich voneinander sind und jeweils unabhängig voneinander aus der aus einem Wasserstoffatom, einem Halogenatom, einer substituierten oder unsubstituierten Kohlenwasserstoffgruppe, einer substituierten oder unsubstituierten heterogenen Gruppe, einer substituierten oder unsubstituierten carbozyklischen Gruppe, einer substituierten oder unsubstituierten heterozyklischen Gruppe, einer substituierten oder unsubstituierten aromatischen Gruppe und einer substituierten oder unsubstituierten heteroaromatischen Gruppe bestehenden Gruppe ausgewählt sind.

3. Zusammensetzung zur Verwendung in einem Verfahren zur Vorbeugung oder Behandlung einer mit NLRP3-Inflammasom zusammenhängenden Entzündungserkrankung, wobei die Zusammensetzung eine durch die folgende Formel 1 dargestellte Verbindung oder ein pharmazeutisch annehmbares Salz davon umfasst:

[Formel 1]

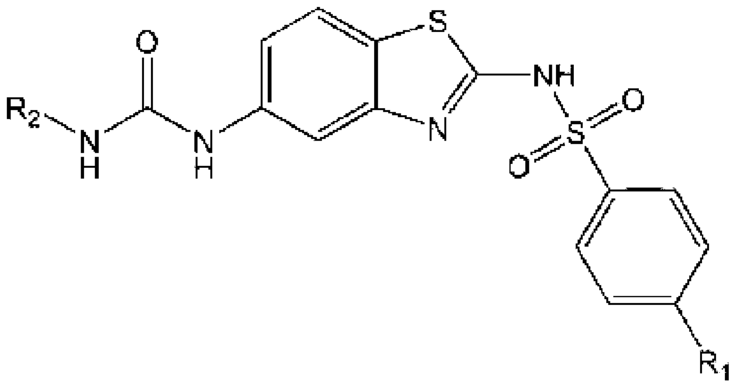

worin $R_1$ und $R_2$ gleich oder unterschiedlich voneinander sind und jeweils unabhängig voneinander aus der aus einem Wasserstoffatom, einem Halogenatom, einer substituierten oder unsubstituierten Kohlenwasserstoffgruppe, einer substituierten oder unsubstituierten heterogenen Gruppe, einer substituierten oder unsubstituierten carbozyklischen Gruppe, einer substituierten oder unsubstituierten heterozyklischen Gruppe, einer substituierten oder unsubstituierten aromatischen Gruppe und einer substituierten oder unsubstituierten heteroaromatischen Gruppe bestehenden Gruppe ausgewählt sind.

**4.** Zusammensetzung zur Verwendung nach Anspruch 3, wobei die mit NLRP3-Inflam-masom zusammenhängende Entzündungserkrankung eine Stoffwechselerkrankung, eine neuroinflammatorische Erkrankung oder eine autoimflammatorische Erkrankung ist.

**5.** Zusammensetzung zur Verwendung nach Anspruch 4, wobei die Stoffwechselerkrankung Adipositas, Hyperlipidämie, Hypercholesterinämie, Atherosklerose, Typ-2-Diabetes, nichtalkoholische Fettleber (NAFLD) oder nichtalkoholische Steatohepatitis (NASH) ist.

**6.** Zusammensetzung zur Verwendung nach Anspruch 4, wobei die neuroinflammatorische Erkrankung Alzheimer-Krankheit, Parkinson-Krankheit, Chorea Huntington, Lou-Gehrig-Krankheit, Creutzfeldt-Jakob-Krankheit, multiple Sklerose, amyotrophe Lateralsklerose, diffuse Lewy-Körperchen-Erkrankung, Leukoenzephalitis, Temporallappenepilepsie oder eine entzündliche Rückenmarksverletzung ist.

**7.** Zusammensetzung zur Verwendung nach Anspruch 4, wobei die autoinflammatorische Erkrankung Muckle-Wells-Syndrom (MWS), adulter latenter Autoimmundiabetes (LADA), familiäres kälteinduziertes autoinflammatorisches Syndrom (FCAS), cryopyrinassoziiertes periodisches Syndrom (CAPS), neonatal beginnendes entzündliches Multisystemsyndrom (NOMID), chronisch infantiles neurologisch-kutanes-artikuläres Syndrom (CINCA), familiäres Mittelmeerfieber (FMF), systemische juvenile idiopathische Arthritis (SJIA), systemische juvenile idiopathische rheumatoide Arthritis oder rheumatoide Arthritis ist.

**8.** Verbindung zur Verwendung nach Anspruch 2 oder Zusammensetzung zur Verwendung nach Anspruch 3, worin $R_1$ und $R_2$ Substituenten sind, die aus folgenden Gruppen ausgewählt sind:

$R_1$: ein Wasserstoffatom oder Methyl; und
$R_2$:

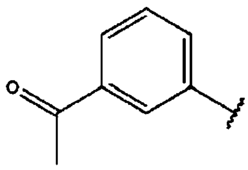 , 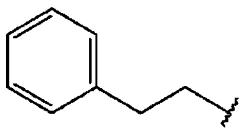 , 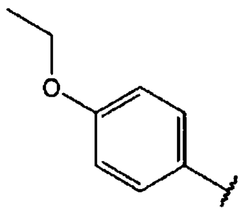 ,

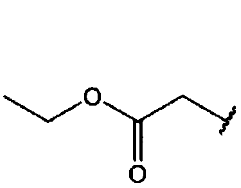 , 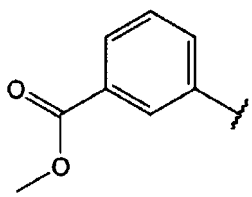 , 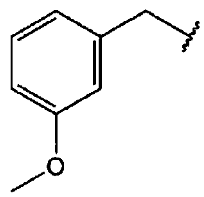 , 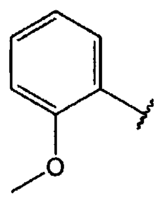 ,

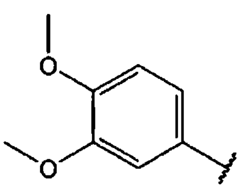 , 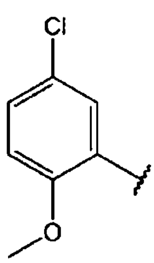 , 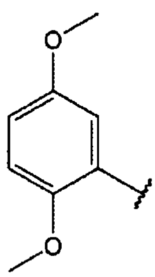 , 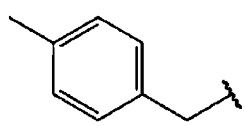 ,

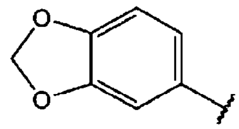, 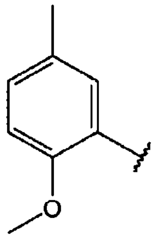, 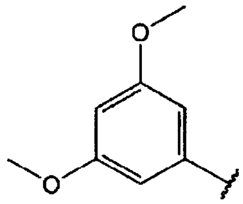, 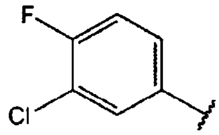,

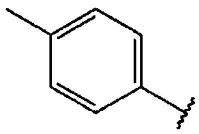, 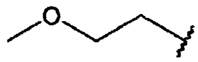, 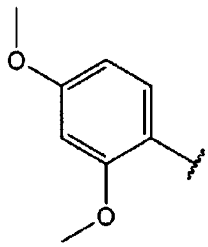, 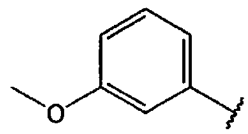,

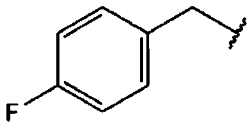, 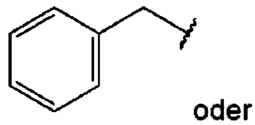 oder 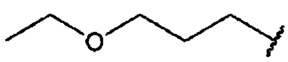.

9. Verbindung zur Verwendung nach Anspruch 2 oder Zusammensetzung zur Verwendung nach Anspruch 3, wobei die durch die Formel 1 dargestellte Verbindung eine Verbindung ist, die durch eine aus der aus den folgenden Formeln 1-1 bis 1-12 bestehenden Gruppe ausgewählte Formel dargestellt ist:

[Formel 1-1]

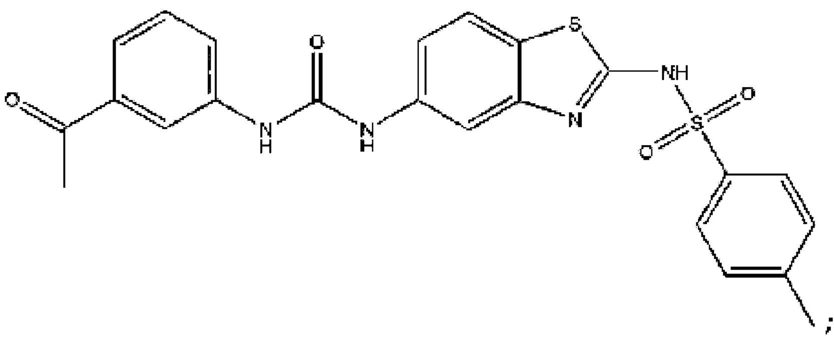;

[Formel 1-2]

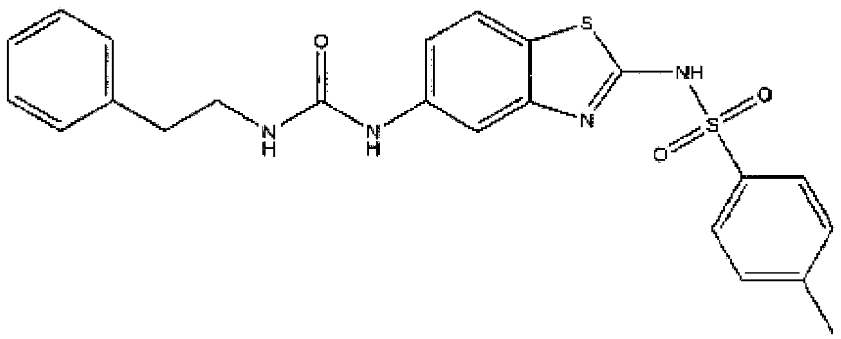;

[Formel 1-3]

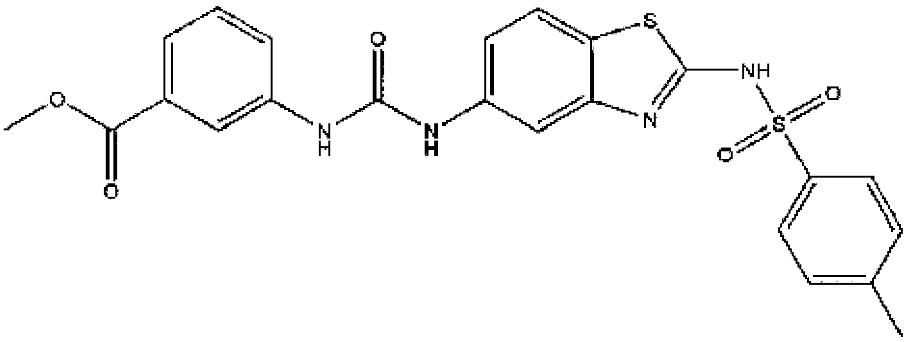

;

[Formel 1-4]

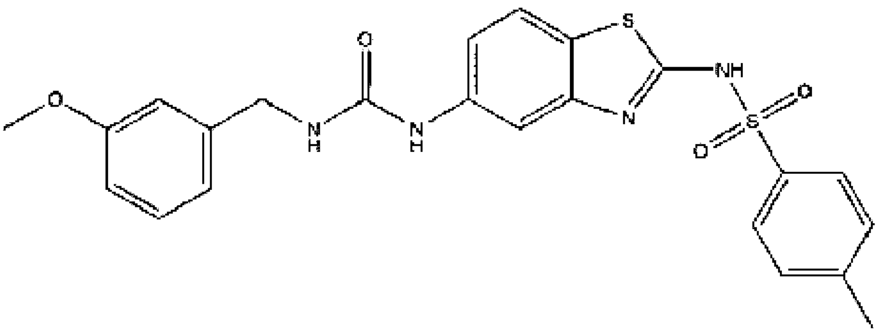

;

[Formel 1-5]

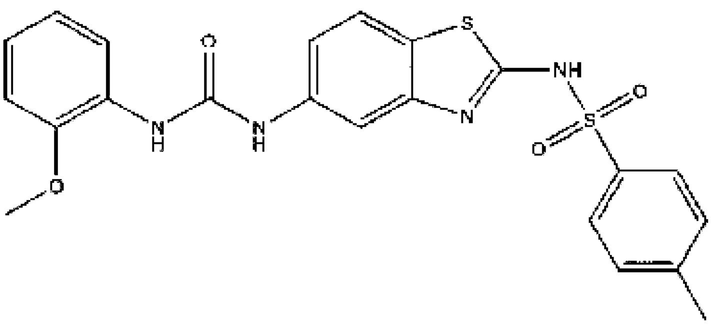

;

[Formel 1-6]

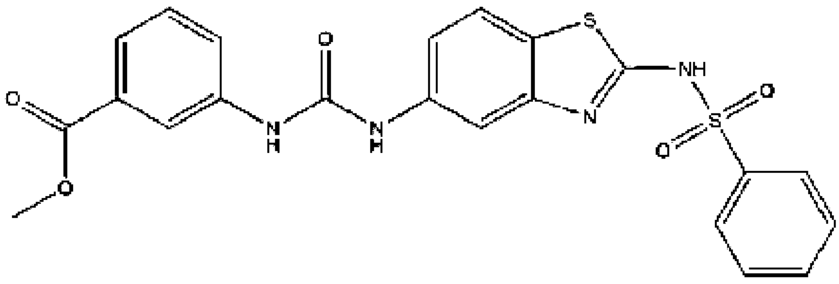

;

[Formel 1-7]

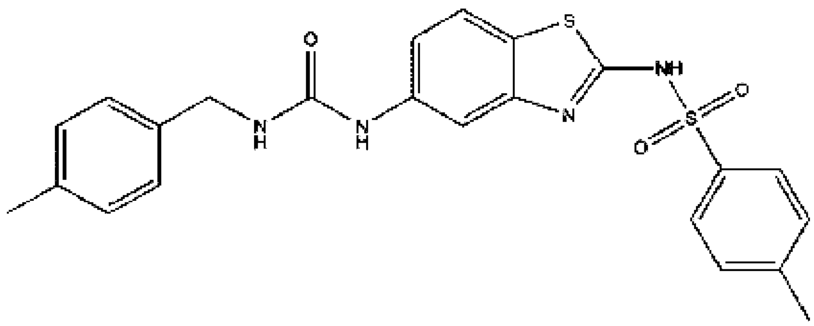

;

[Formel 1-8]

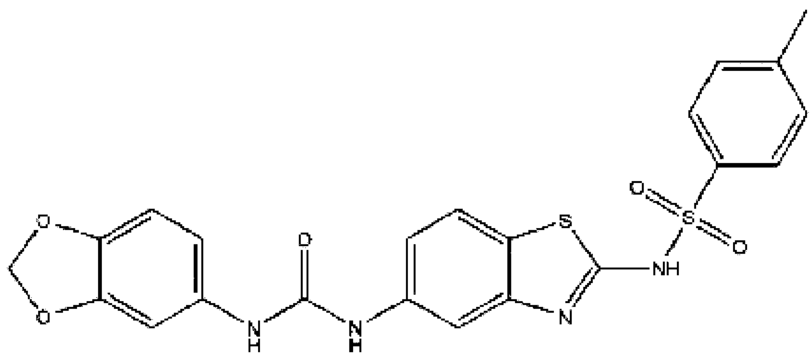

;

[Formel 1-9]

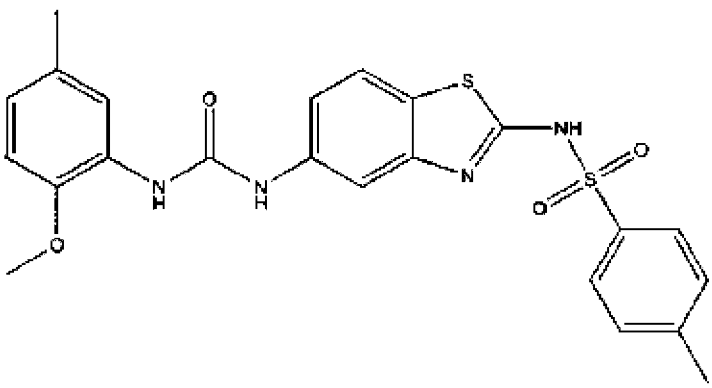

;

[Formel 1-10]

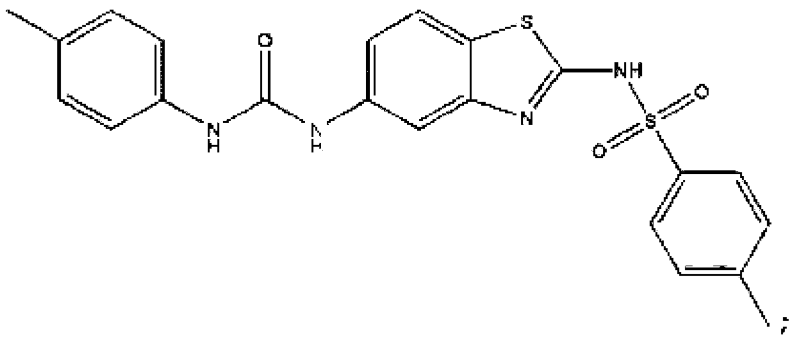

[Formel 1-11]

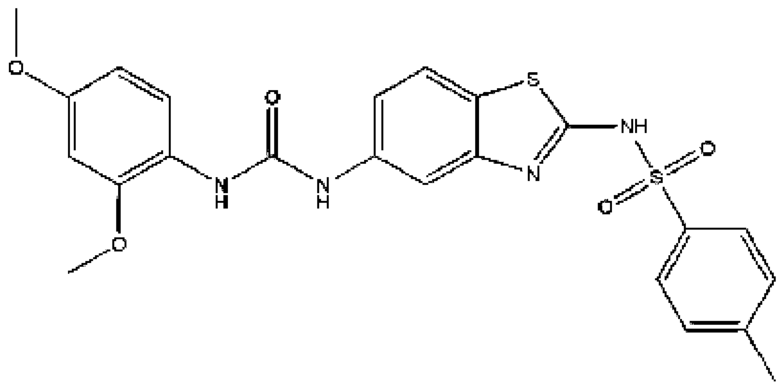

und

[Formel 1-12]

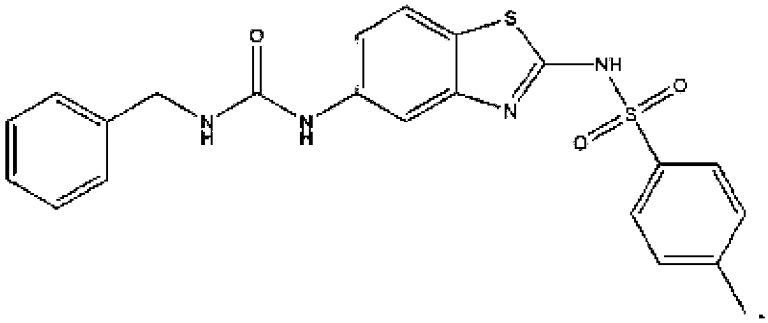

10. Zusammensetzung zur Verwendung nach einem der Ansprüche 3 bis 9, wobei die Zusammensetzung in Form einer Nahrungsmittel-Zusammensetzung vorliegt.

**Revendications**

1. Composé représenté par l'une quelconque des formules choisies dans le groupe constitué par les formules 1-1 à 1-12 suivantes, ou un sel pharmaceutiquement acceptable de celui-ci :

[Formule 1-1]

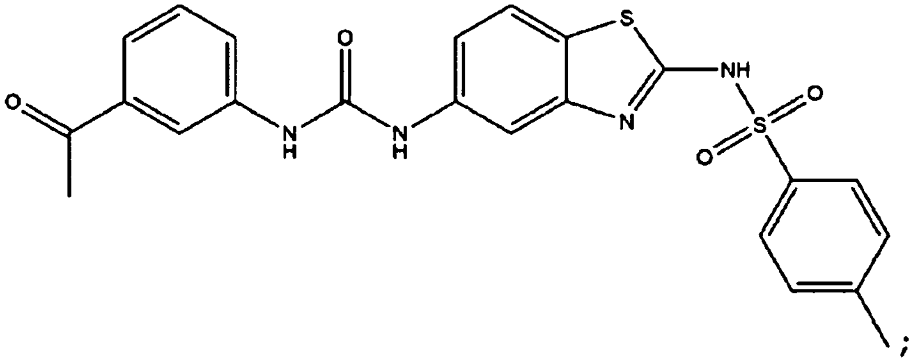

[Formule 1-2]

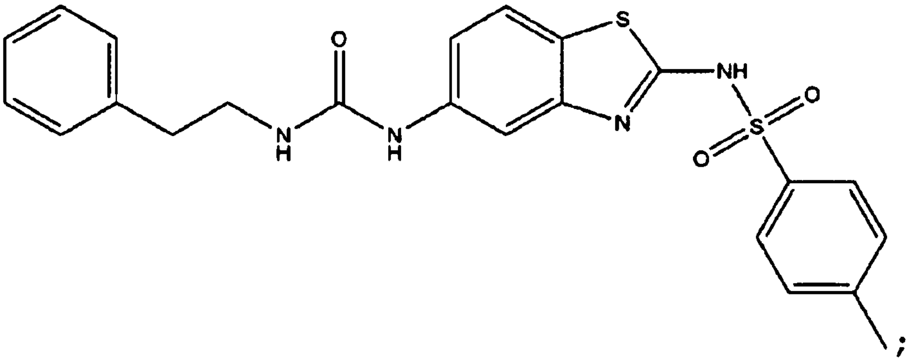

[Formule 1-3]

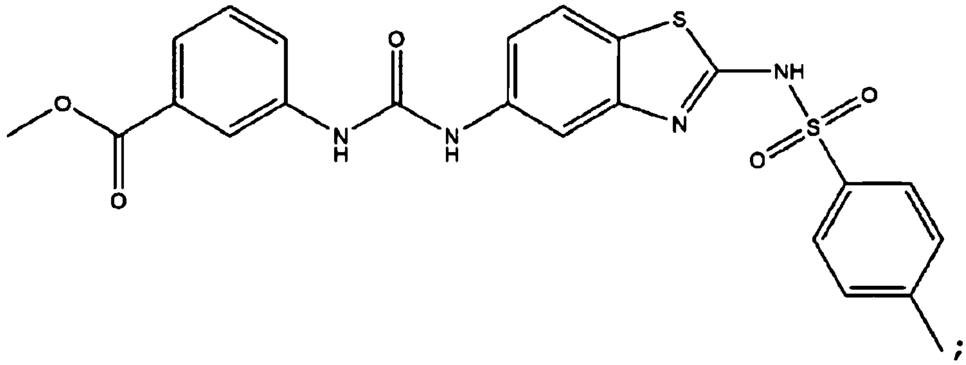

[Formule 1-4]

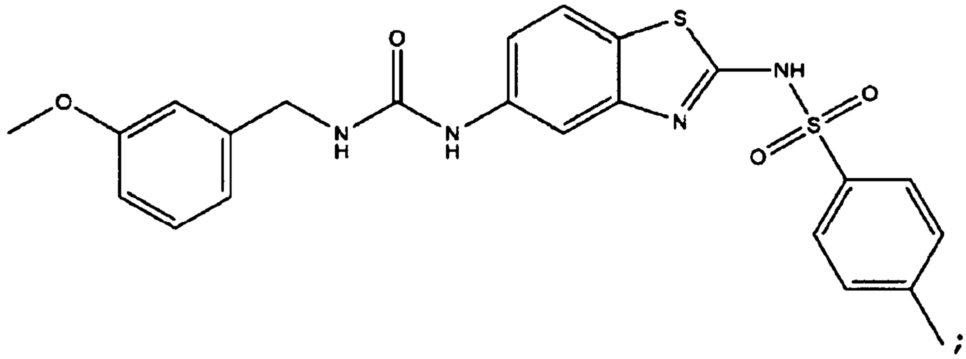

[Formule 1-5]

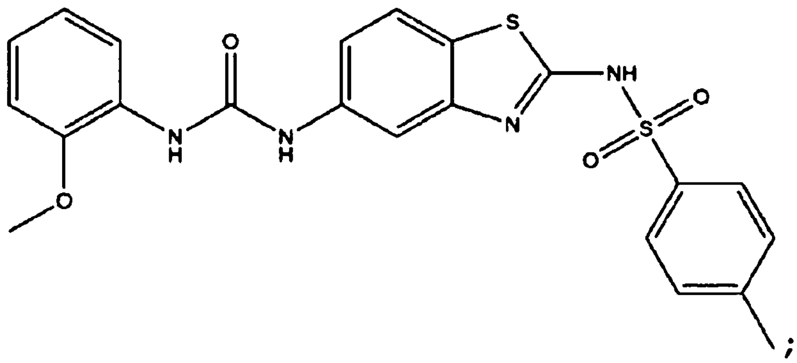

[Formule 1-6]

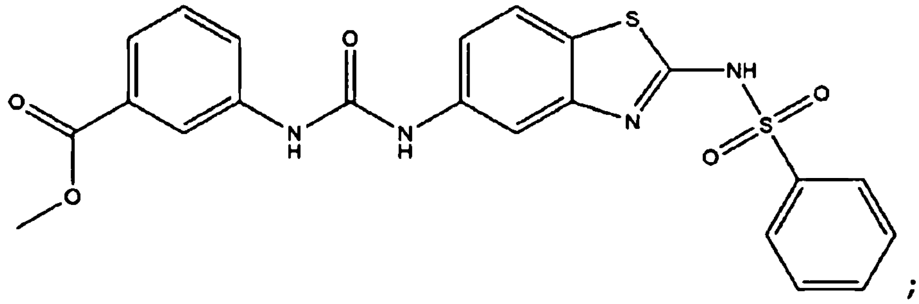

[Formule 1-7]

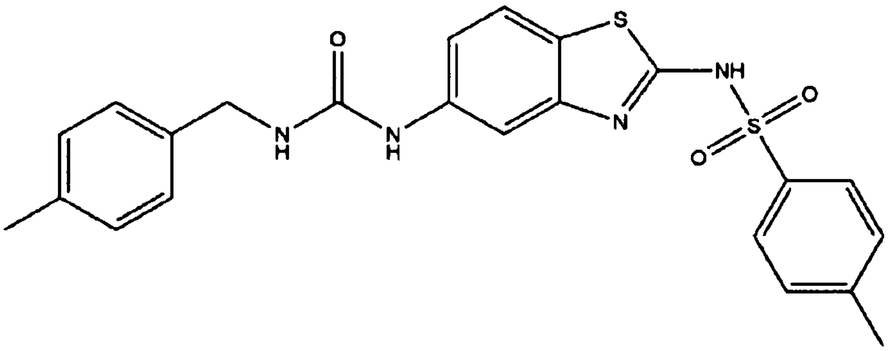

;

[Formule 1-8]

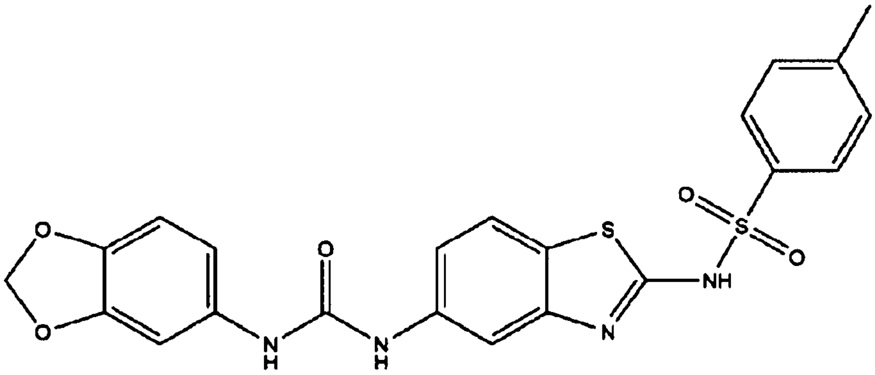

;

[Formule 1-9]

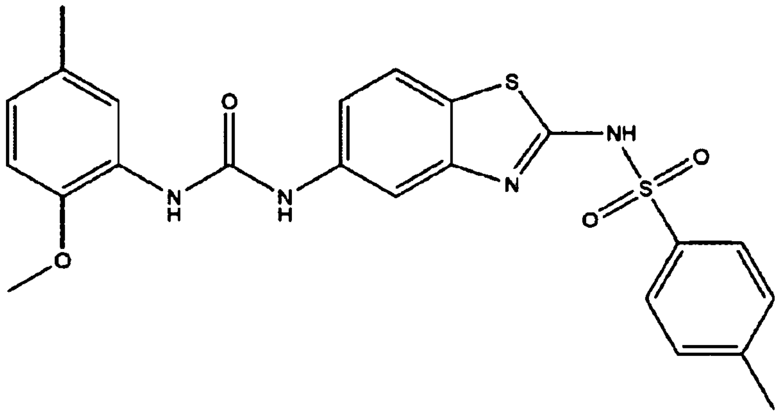

;

[Formule 1-10]

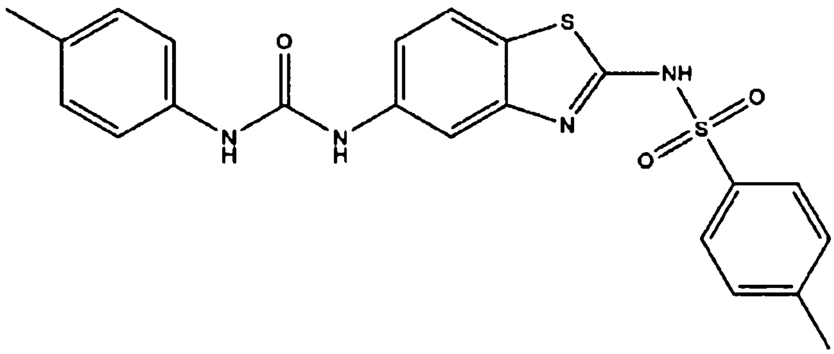

;

[Formule 1-11]

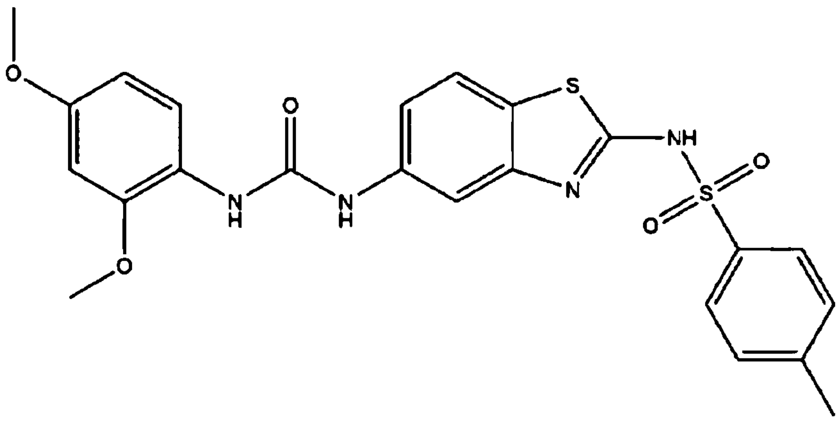

;

et

[Formule 1-12]

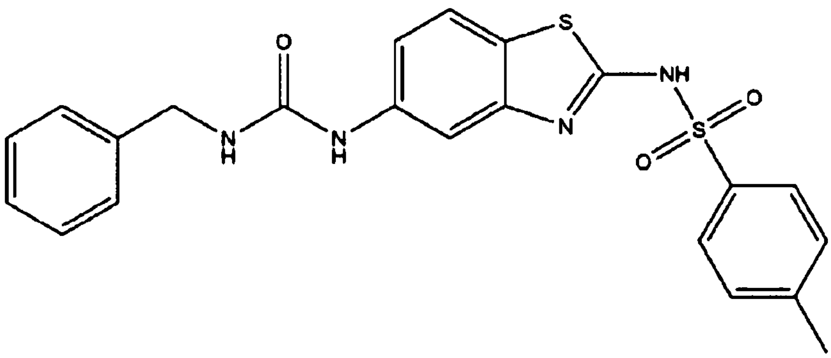

.

**2.** Composé représenté par la formule 1 suivante, ou un sel pharmaceutiquement acceptable de celui-ci, pour une utilisation dans une méthode d'inhibition de l'activité de l'inflammasome NLRP3 :

[Formule 1]

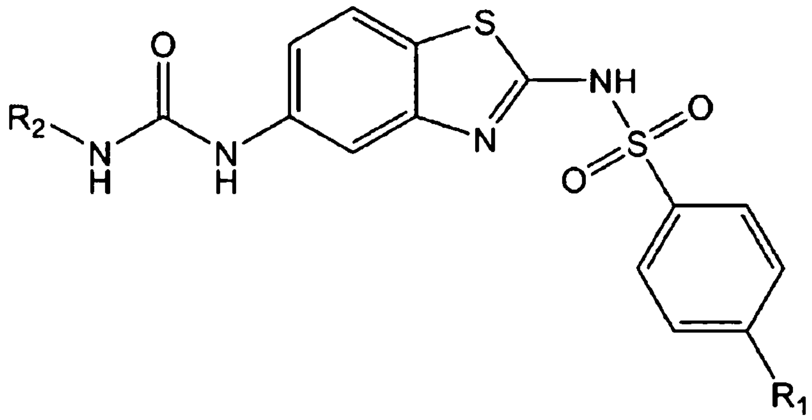

dans laquelle $R_1$ et $R_2$ sont identiques ou différents l'un de l'autre et choisis indépendamment dans le groupe constitué par un atome d'hydrogène, un atome d'halogène, un groupe hydrocarboné substitué ou non substitué, un groupe hétérogène substitué ou non substitué, un groupe carbocyclique substitué ou non substitué, un groupe hétérocyclique substitué ou non substitué, un groupe aromatique substitué ou non substitué et un groupe hétéroaromatique substitué ou non substitué.

3. Composition pour une utilisation dans une méthode de prévention ou de traitement d'une maladie inflammatoire associée à l'inflammasome NLRP3, la composition comprenant un composé représenté par la formule 1 suivante, ou un sel pharmaceutiquement acceptable de celui-ci :

[Formule 1]

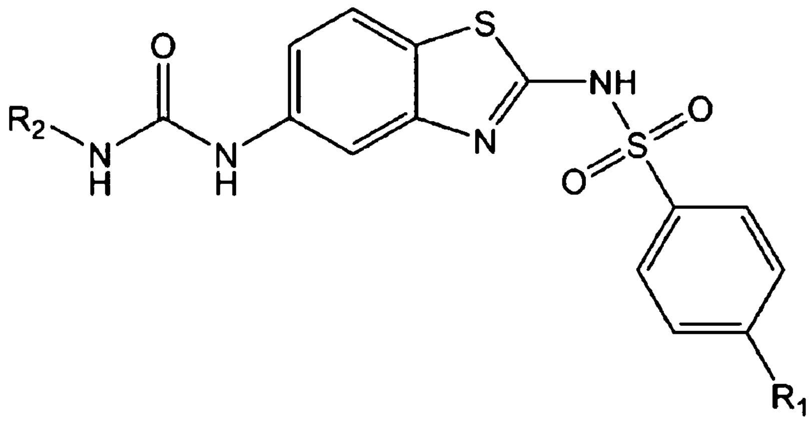

dans laquelle $R_1$ et $R_2$ sont identiques ou différents l'un de l'autre et choisis indépendamment dans le groupe constitué par un atome d'hydrogène, un atome d'halogène, un groupe hydrocarboné substitué ou non substitué, un groupe hétérogène substitué ou non substitué, un groupe carbocyclique substitué ou non substitué, un groupe hétérocyclique substitué ou non substitué, un groupe aromatique substitué ou non substitué et un groupe hétéroaromatique substitué ou non substitué.

4. Composition pour une utilisation selon la revendication 3, dans laquelle la maladie inflammatoire associée à l'inflammasome NLRP3 est une maladie métabolique, une maladie neuro-inflammatoire ou une maladie auto-inflammatoire.

5. Composition pour une utilisation selon la revendication 4, dans laquelle la maladie métabolique est l'obésité, l'hyperlipidémie, l'hypercholestérolémie, l'artériosclérose, le diabète de type 2, la stéatose hépatique non alcoolique (NAFLD) ou la stéatohépatite non alcoolique (NASH).

6. Composition pour une utilisation selon la revendication 4, dans laquelle la maladie neuro-inflammatoire est la maladie d'Alzheimer, la maladie de Parkinson, la maladie de Huntington, la maladie de Charcot, la maladie de Creutzfeldt-Jakob, la sclérose en plaques, la sclérose latérale amyotrophique, la maladie à corps de Lewy diffus, la leucoencéphalite, l'épilepsie du lobe temporal ou une lésion inflammatoire de la moelle épinière.

**7.** Composition pour une utilisation selon la revendication 4, dans laquelle la maladie auto-inflammatoire est le syndrome de Muckle-Wells (MWS), le diabète auto-immun latent de l'adulte (LADA), le syndrome familial auto-inflammatoire au froid (FCAS), le syndrome périodique associé à la cryopyrine (CAPS), le syndrome inflammatoire multisystémique à début néonatal (NOMID), le syndrome neurologique, cutané et articulaire infantile chronique (CINCA), la fièvre méditerranéenne familiale (FMF), l'arthrite juvénile idiopathique à début systémique (AJIS), l'arthrite rhumatoïde idiopathique juvénile à début systémique ou l'arthrite rhumatoïde.

**8.** Composé pour une utilisation selon la revendication 2 ou composition pour une utilisation selon la revendication 3, dans lesquels $R_1$ et $R_2$ sont des substituants choisis parmi les groupes suivants :

$R_1$ : un atome d'hydrogène ou un groupe méthyle ; et
$R_2$ :

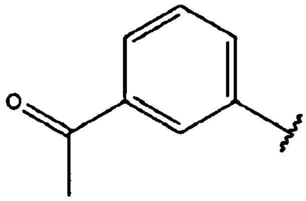 , 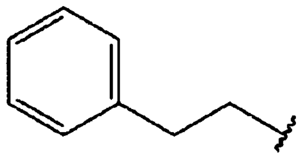 , 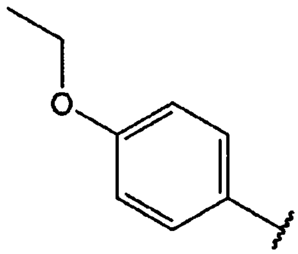 ,

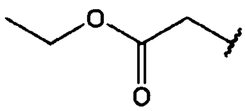 , 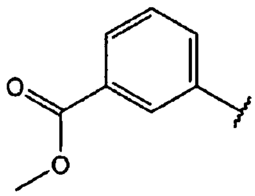 , 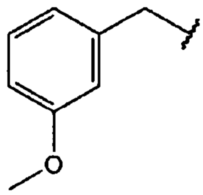 , 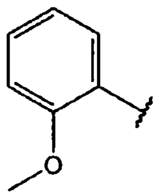 ,

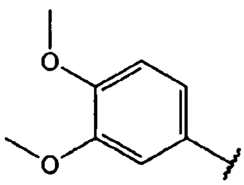 , 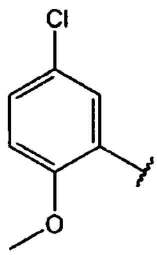 , 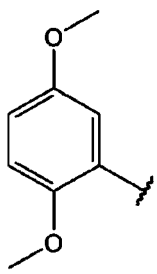 , 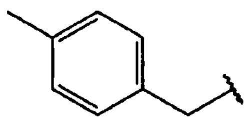 ,

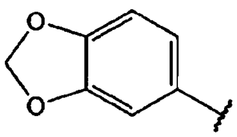 , 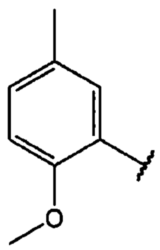 , 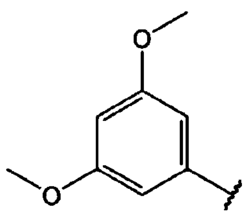 , 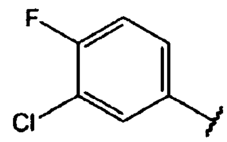 ,

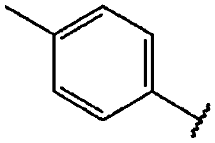 , 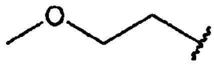 , 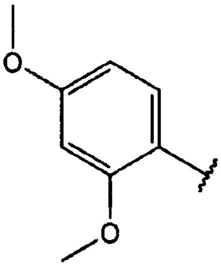 , 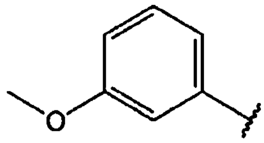 ,

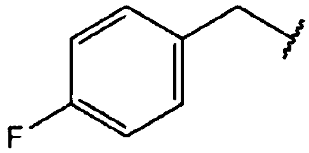 , 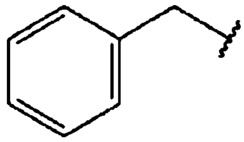

ou

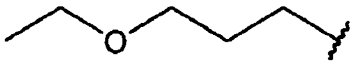 .

9. Composé pour une utilisation selon la revendication 2 ou composition pour une utilisation selon la revendication 3, dans lesquels le composé représenté par la formule 1 est un composé représenté par l'une quelconque des formules choisies dans le groupe constitué par les formules 1-1 à 1-12 suivantes :

[Formule 1-1]

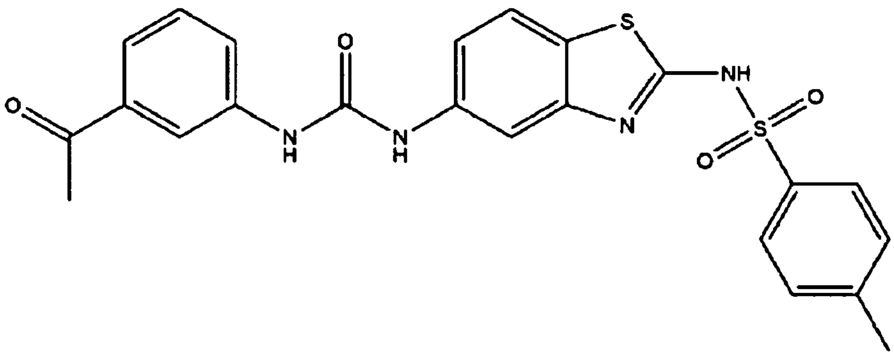 ;

[Formule 1-2]

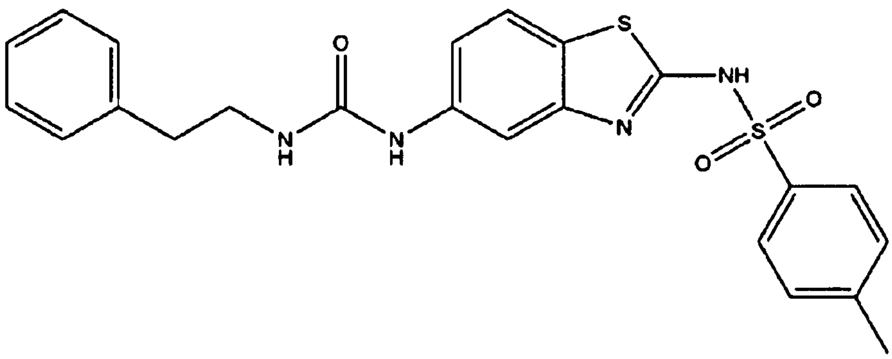 ;

[Formule 1-3]

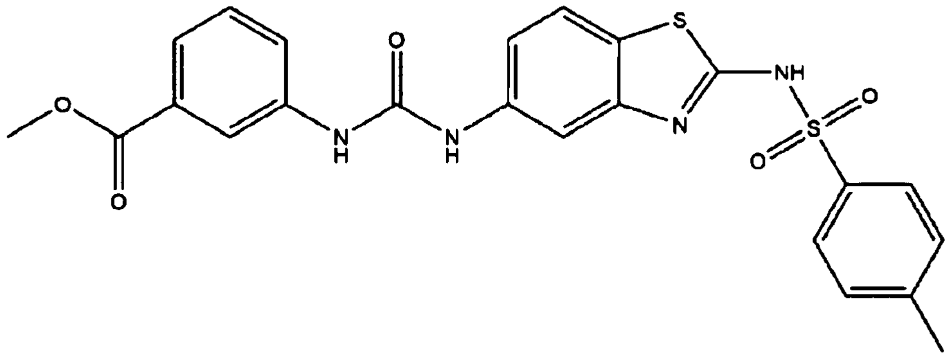

;

[Formule 1-4]

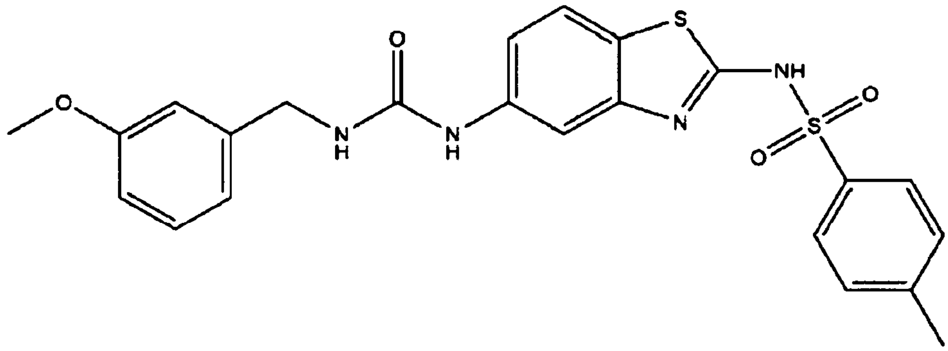

;

[Formule 1-5]

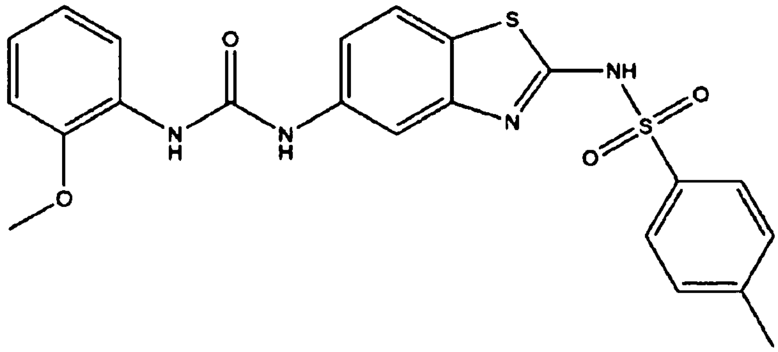

;

[Formule 1-6]

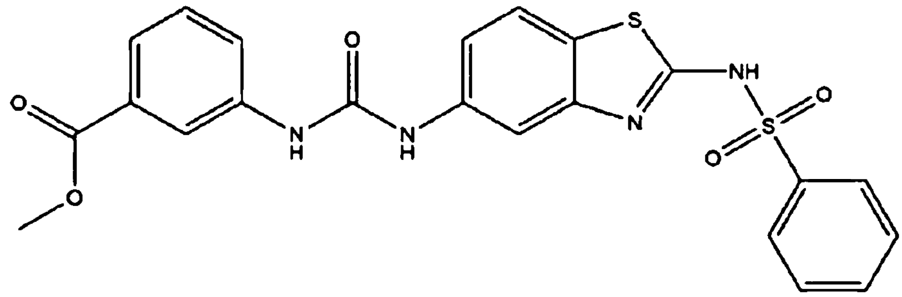

;

[Formule 1-7]

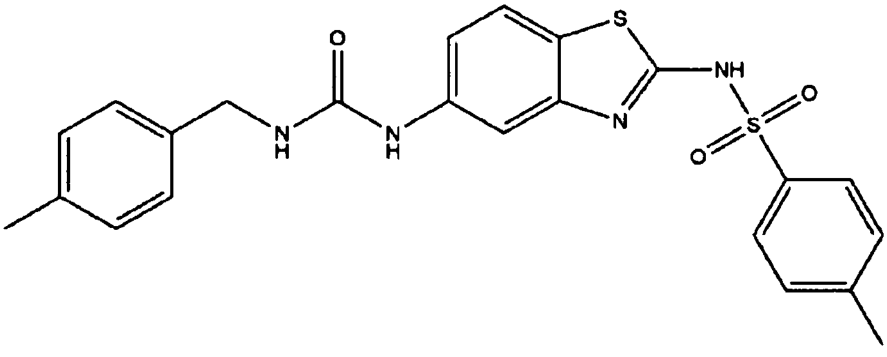

;

[Formule 1-8]

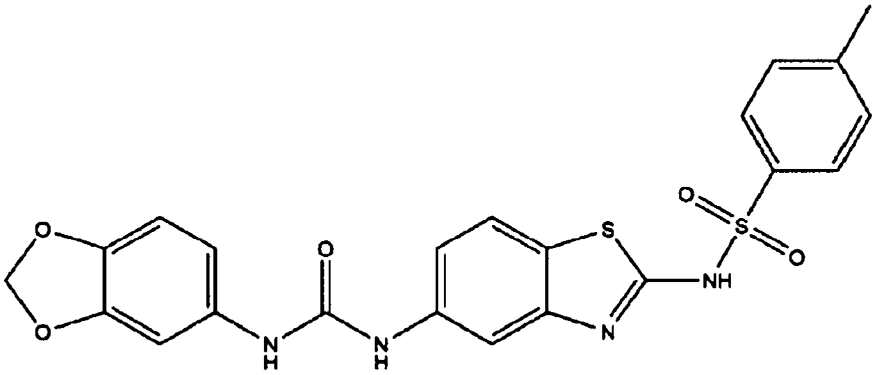

;

[Formule 1-9]

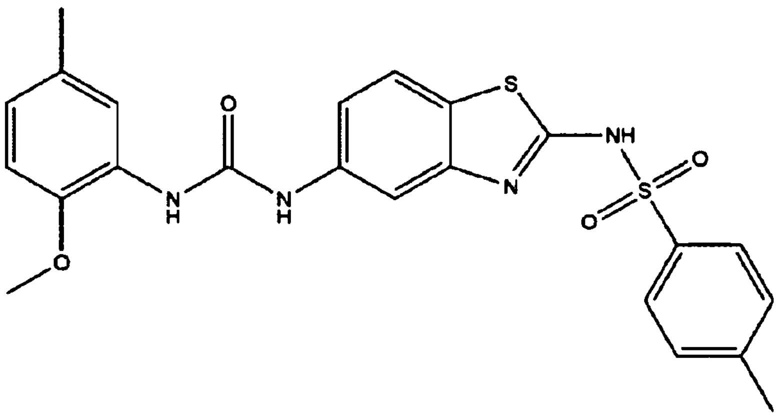

;

[Formule 1-10]

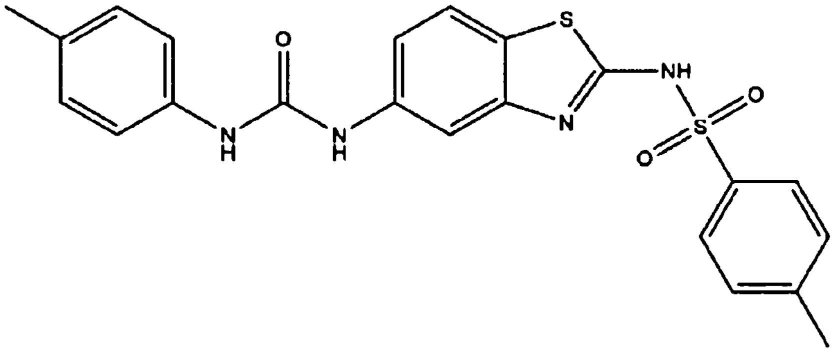

;

[Formule 1-11]

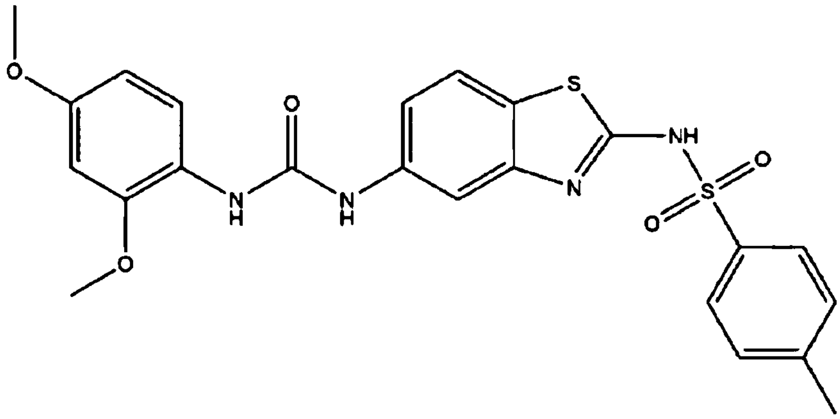

;

et

[Formule 1-12]

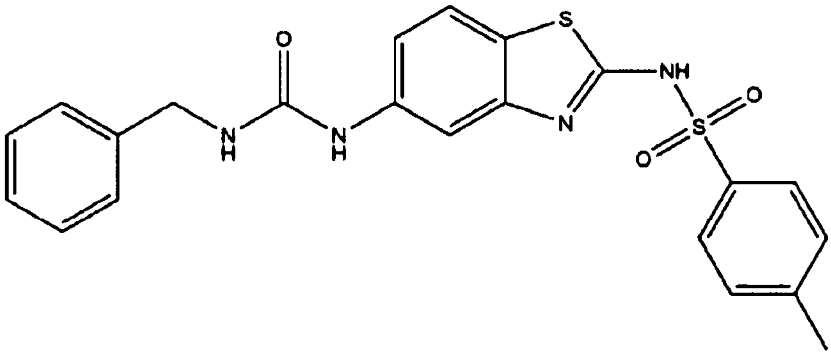

.

**10.** Composition pour une utilisation selon l'une quelconque des revendications 3 à 9, dans laquelle la composition est sous la forme d'une composition alimentaire.

FIG. 1

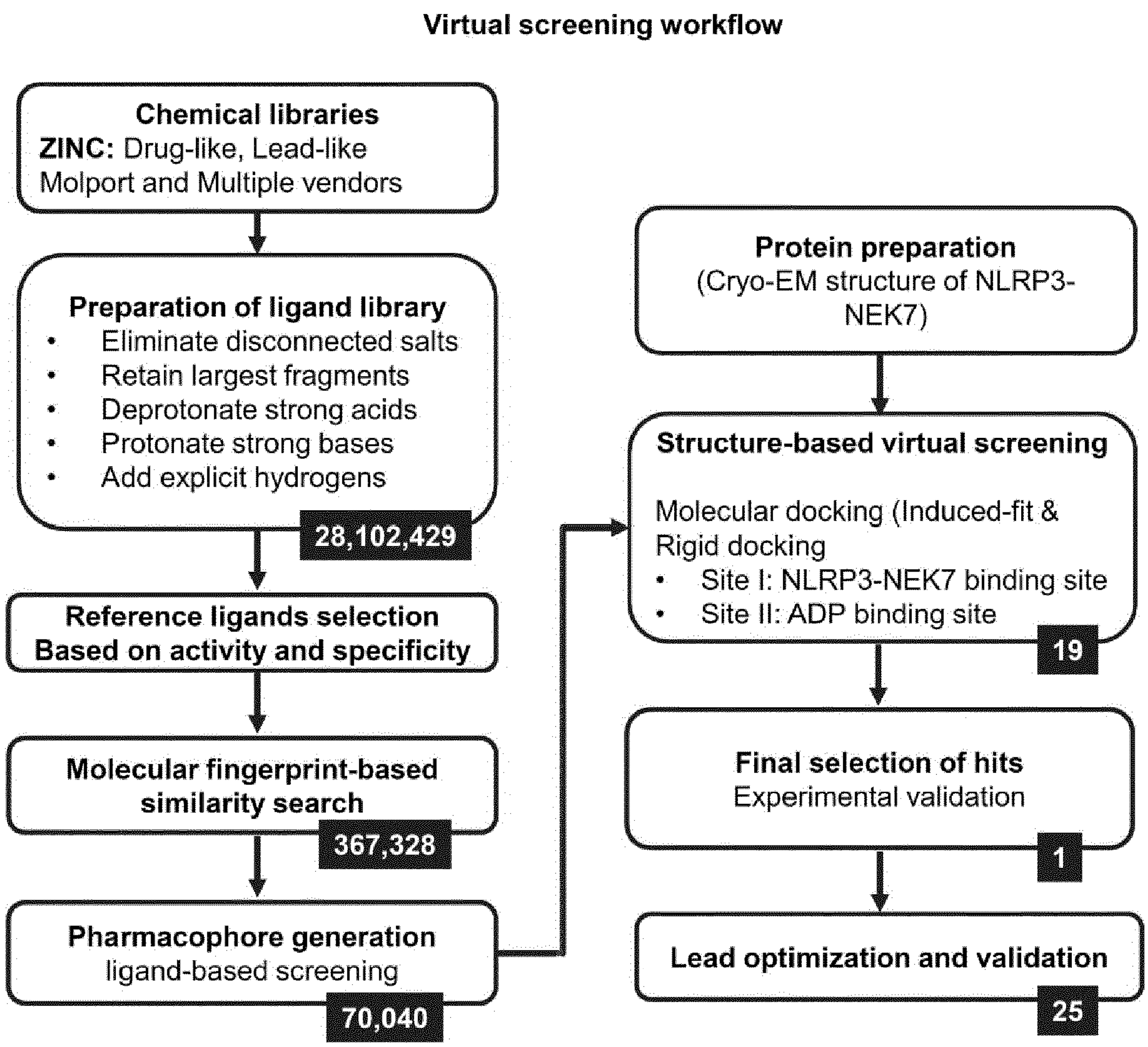
Virtual screening workflow
Chemical libraries
ZINC: Drug-like, Lead-like
Molport and Multiple vendors
Preparation of ligand library
Eliminate disconnected salts
Retain largest fragments
Deprotonate strong acids
Protonate strong bases
Add explicit hydrogens
28,102,429
Reference ligands selection
Based on activity and specificity
Molecular fingerprint-based similarity search
367,328
Pharmacophore generation
ligand-based screening
70,040
Protein preparation
(Cryo-EM structure of NLRP3-NEK7)
Structure-based virtual screening
Molecular docking (Induced-fit & Rigid docking
Site I: NLRP3-NEK7 binding site
Site II: ADP binding site
19
Final selection of hits
Experimental validation
1
Lead optimization and validation
25

FIG. 2

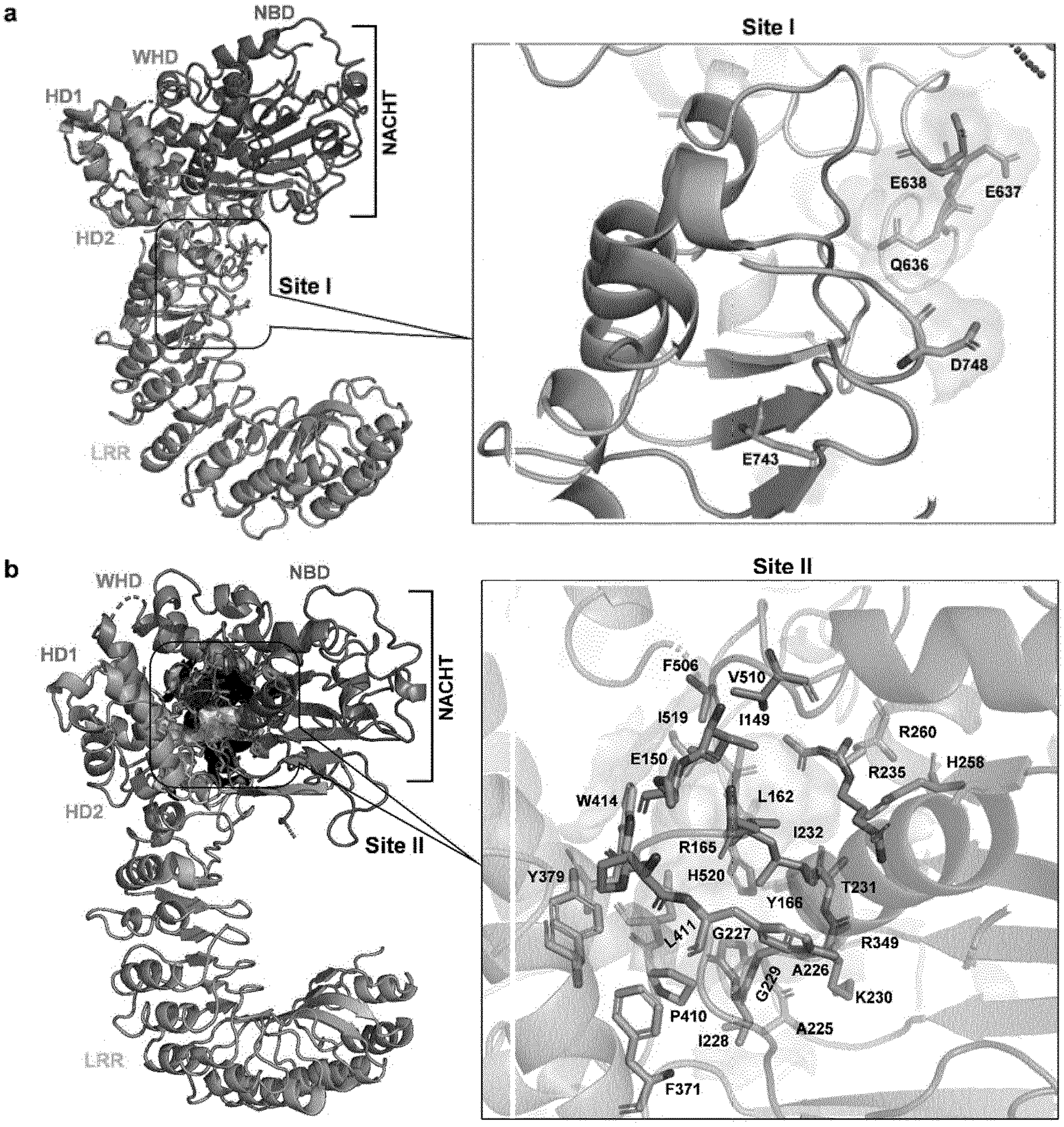
a
NBD
WHD
HD1
NACHT
HD2
Site I
LRR
Site I
E638
E637
Q636
D748
E743
b
WHD
NBD
HD1
NACHT
HD2
Site II
LRR
Site II
F506
V510
I519
I149
R260
E150
H258
R235
W414
L162
I232
R165
H520
Y379
T231
Y166
L411
G227
R349
A226
G229
K230
P410
A225
I228
F371

FIG. 3

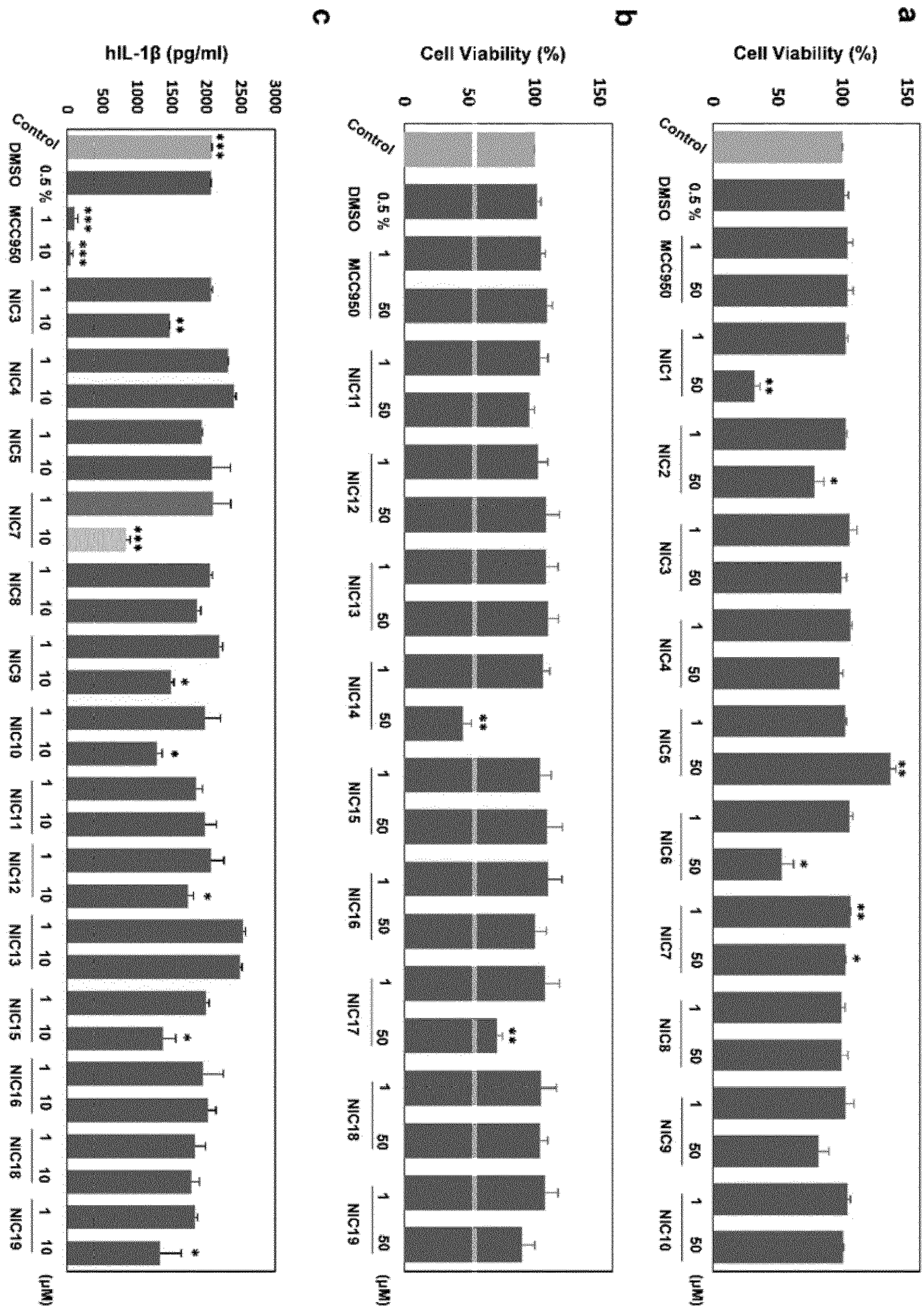
a
Cell Viability (%)
b
Cell Viability (%)
c
hIL-1β (pg/ml)
Control
DMSO 0.5 %
MCC950
NIC1
NIC2
NIC3
NIC4
NIC5
NIC6
NIC7
NIC8
NIC9
NIC10
NIC11
NIC12
NIC13
NIC14
NIC15
NIC16
NIC17
NIC18
NIC19
(μM)

FIG. 4

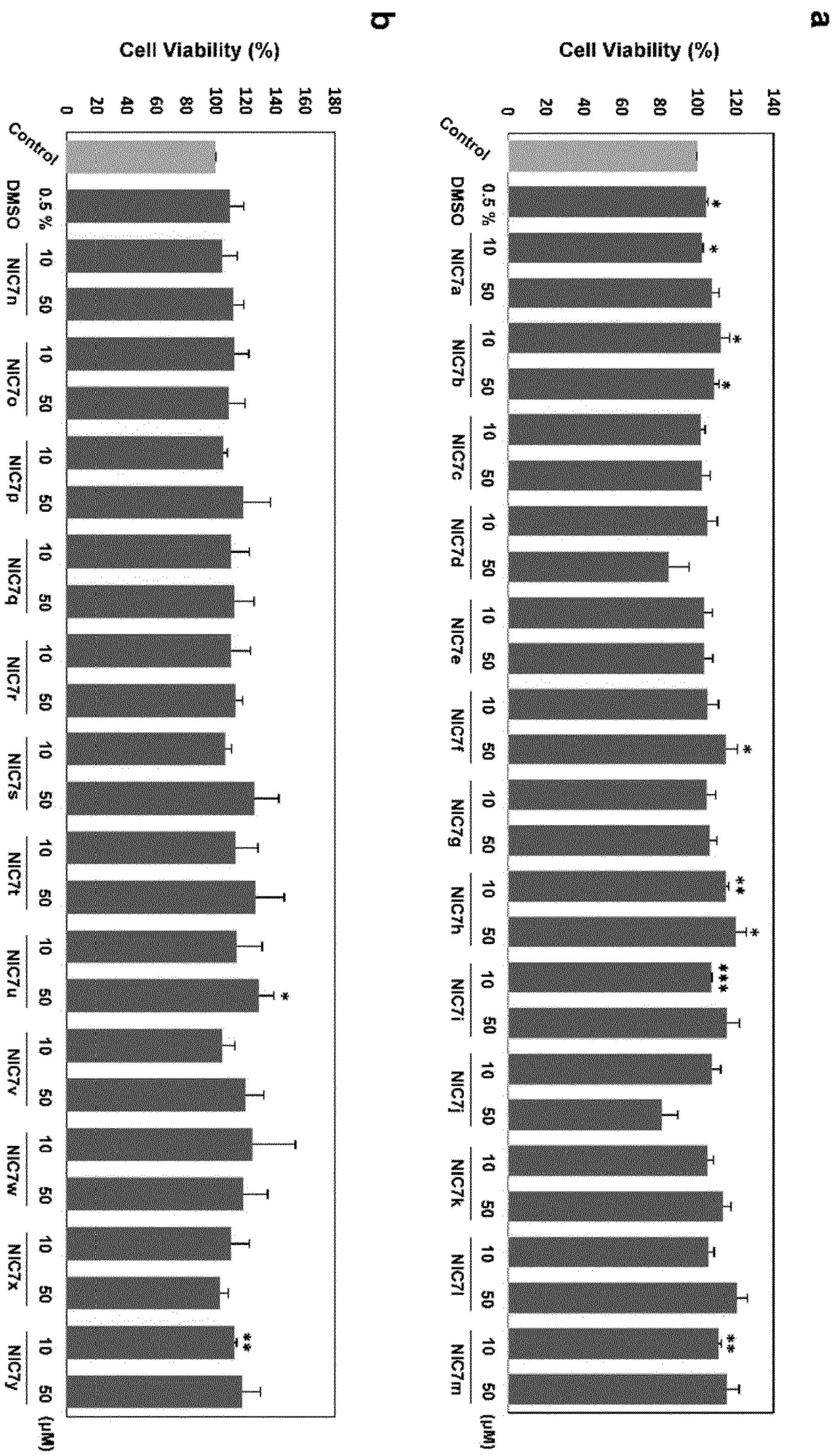
a
Cell Viability (%)
0
20
40
60
80
100
120
140
Control
DMSO
0.5 %
NIC7a
NIC7b
NIC7c
NIC7d
NIC7e
NIC7f
NIC7g
NIC7h
NIC7i
NIC7j
NIC7k
NIC7l
NIC7m
10
50
(μM)
b
Cell Viability (%)
160
180
NIC7n
NIC7o
NIC7p
NIC7q
NIC7r
NIC7s
NIC7t
NIC7u
NIC7v
NIC7w
NIC7x
NIC7y

FIG. 5

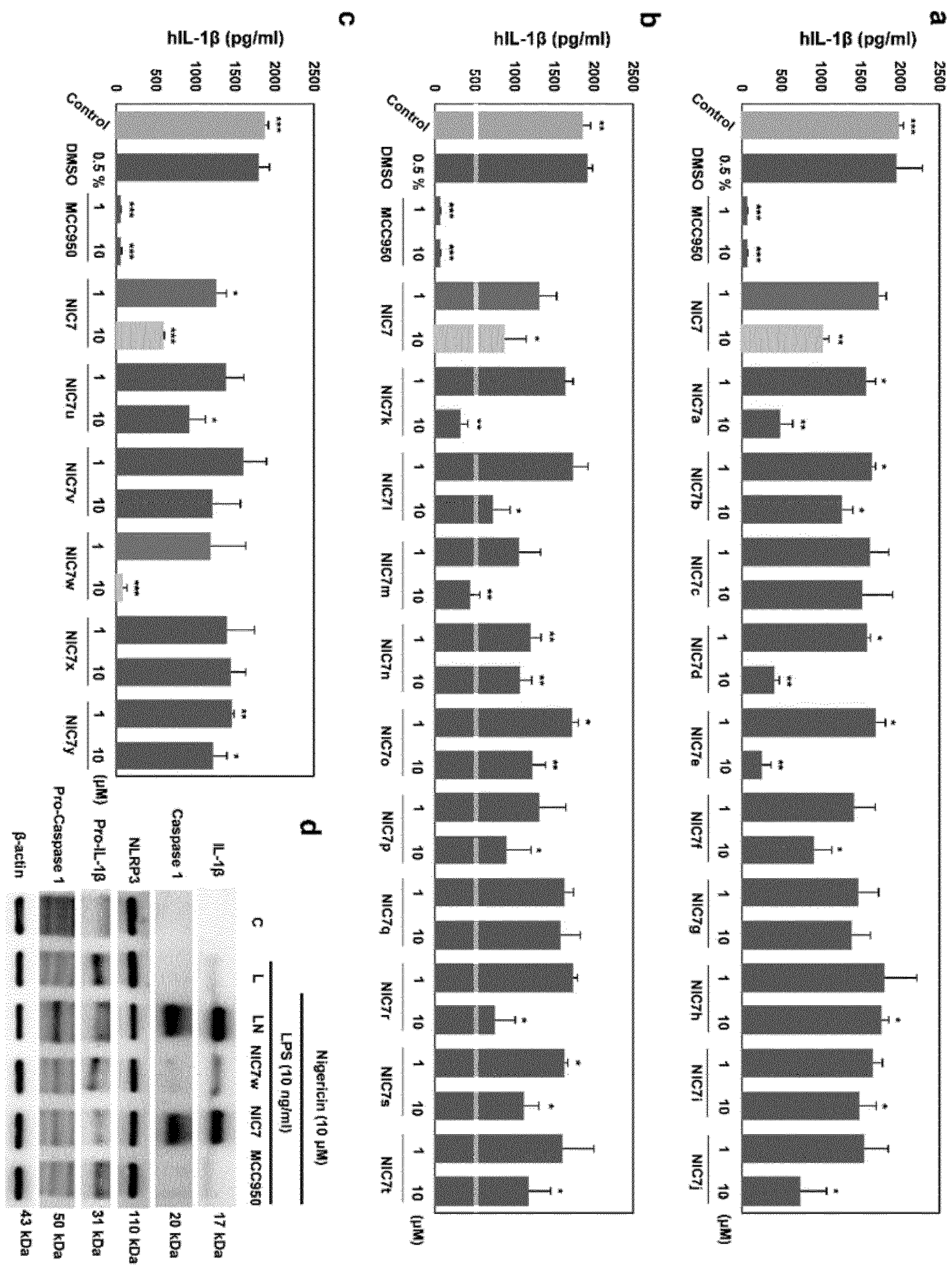
a
b
c
d
hIL-1β (pg/ml)
Control
DMSO 0.5 %
MCC950
NIC7
NIC7a
NIC7b
NIC7c
NIC7d
NIC7e
NIC7f
NIC7g
NIC7h
NIC7i
NIC7j
NIC7k
NIC7l
NIC7m
NIC7n
NIC7o
NIC7p
NIC7q
NIC7r
NIC7s
NIC7t
NIC7u
NIC7v
NIC7w
NIC7x
NIC7y
(μM)
Nigericin (10 μM)
LPS (10 ng/ml)
C
L
LN
NIC7w
NIC7
MCC950
IL-1β
Caspase 1
NLRP3
Pro-IL-1β
Pro-Caspase 1
β-actin
17 kDa
20 kDa
110 kDa
31 kDa
50 kDa
43 kDa

FIG. 6

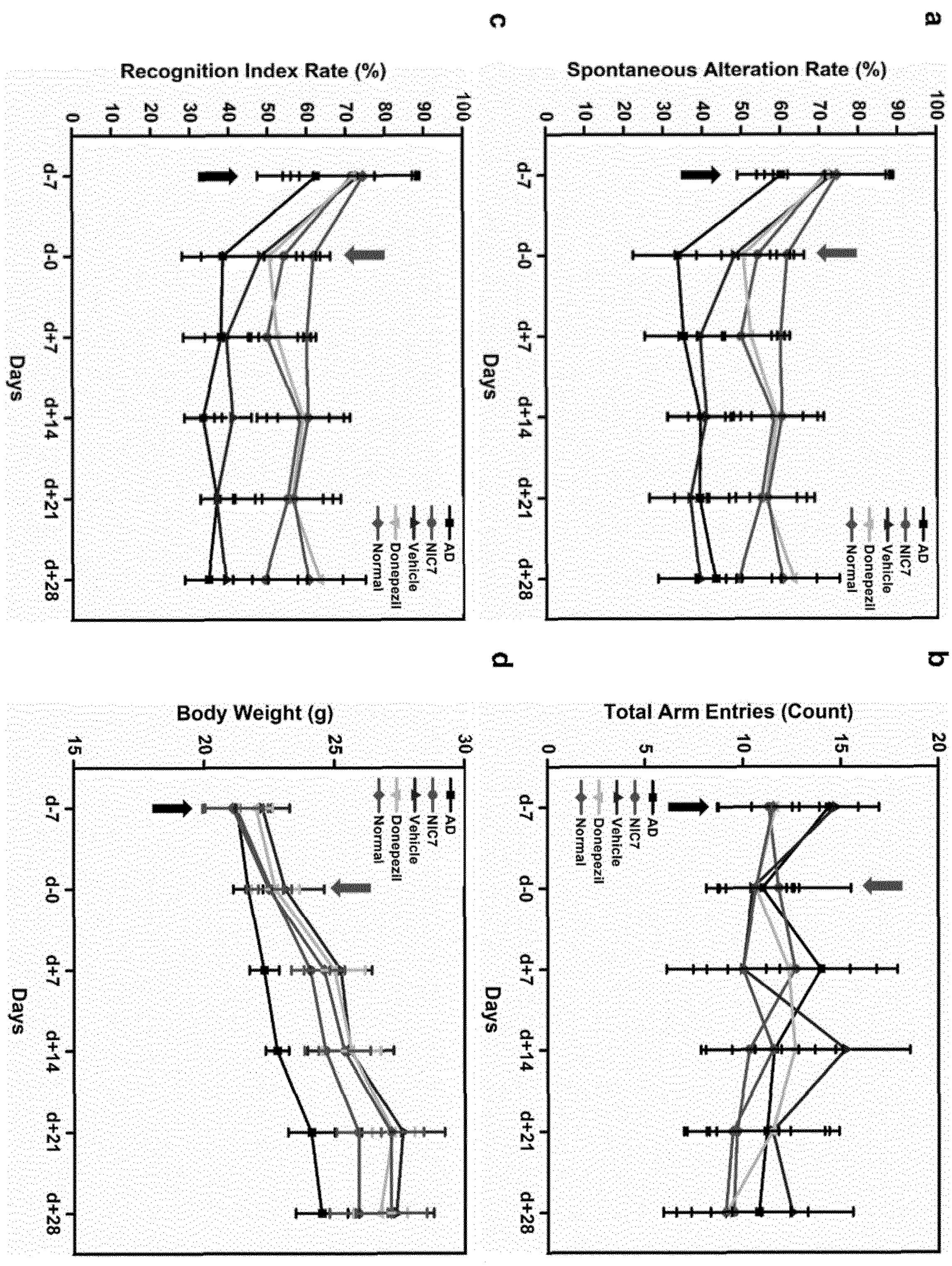
a
Spontaneous Alteration Rate (%)
b
Total Arm Entries (Count)
c
Recognition Index Rate (%)
d
Body Weight (g)
Days
d-7
d-0
d+7
d+14
d+21
d+28
AD
NIC7
Vehicle
Donepezil
Normal

FIG. 7

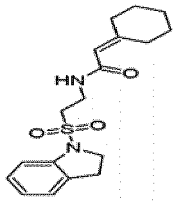

NIC3

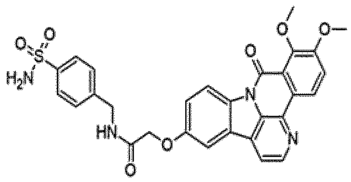

NIC4

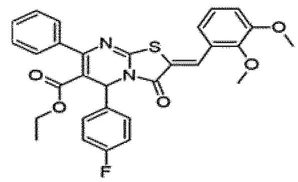

NIC5

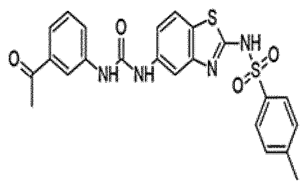

NIC7

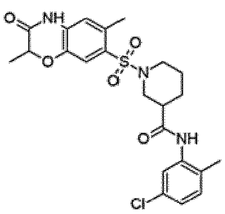

NIC9

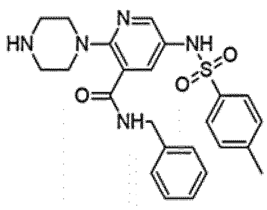

NIC10

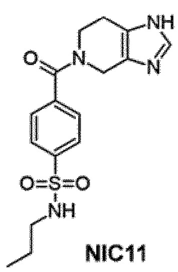

NIC11

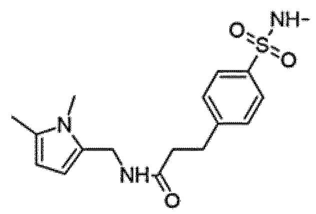

NIC12

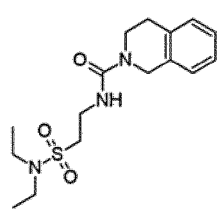

NIC13

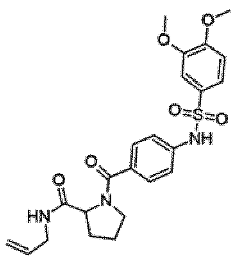

NIC15

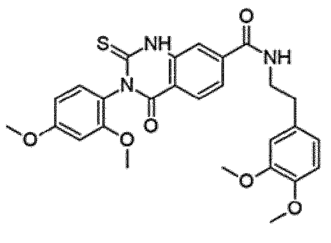

NIC16

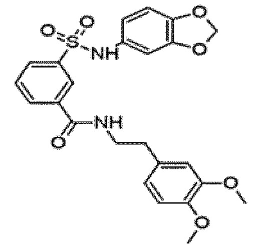

NIC18

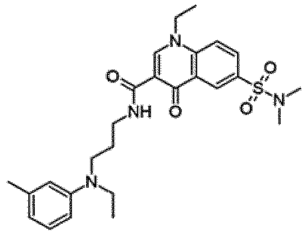

NIC19

FIG. 8

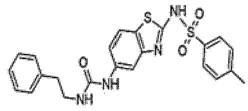

NIC7a

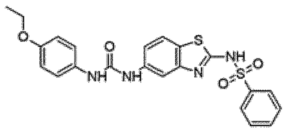

NIC7b

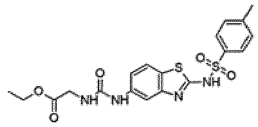

NIC7c

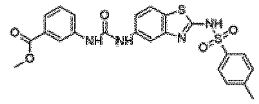

NIC7d

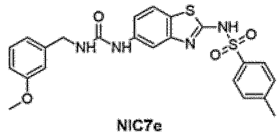

NIC7e

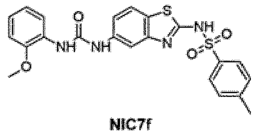

NIC7f

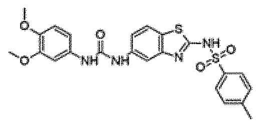

NIC7g

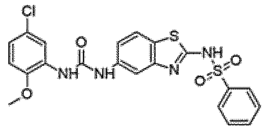

NIC7h

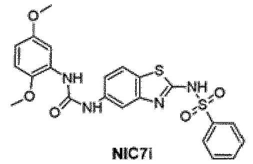

NIC7i

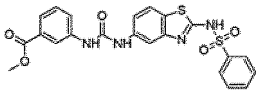

NIC7j

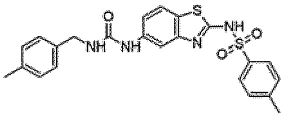

NIC7k

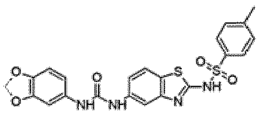

NIC7l

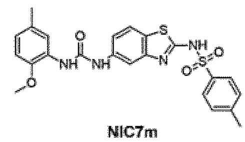

NIC7m

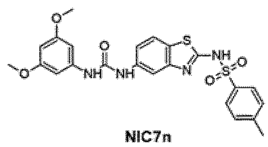

NIC7n

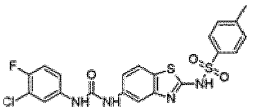

NIC7o

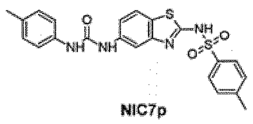

NIC7p

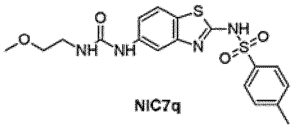

NIC7q

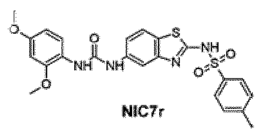

NIC7r

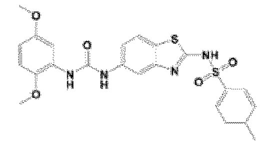

NIC7s

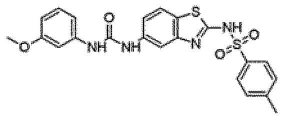

NIC7t

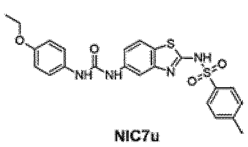

NIC7u

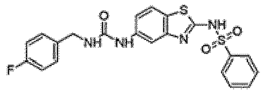

NIC7v

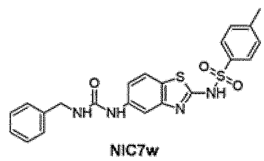

NIC7w

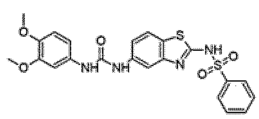

NIC7x

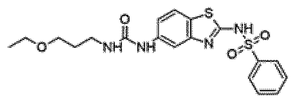

NIC7y

FIG. 9

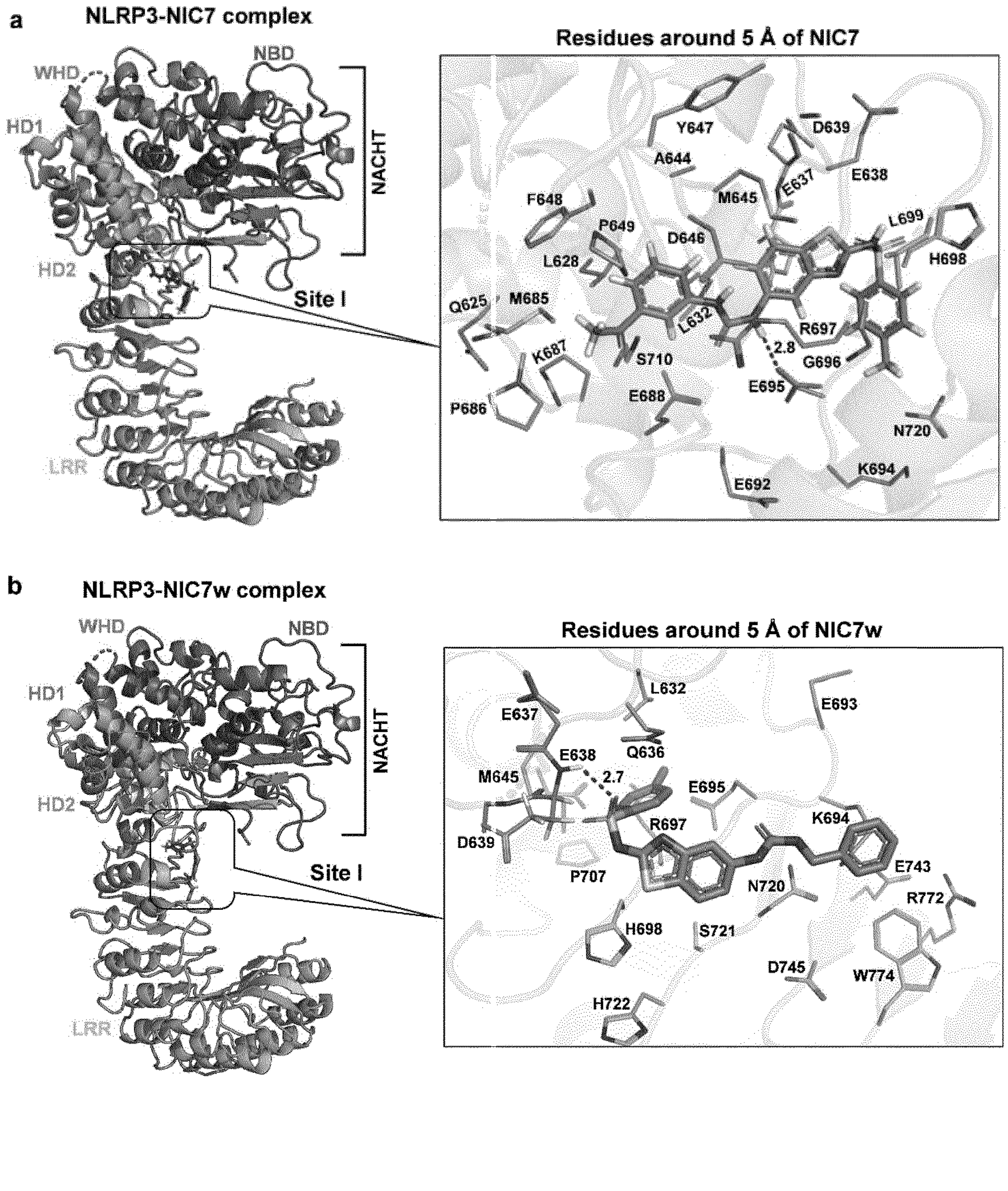
a
NLRP3-NIC7 complex
WHD
NBD
HD1
NACHT
HD2
Site I
LRR
Residues around 5 Å of NIC7
Y647
D639
A644
E638
E637
M645
F648
P649
D646
L699
L628
H698
Q625
M685
L632
R697
K687
S710
2.8
G696
E688
E695
P686
N720
K694
E692
b
NLRP3-NIC7w complex
WHD
NBD
HD1
NACHT
HD2
Site I
LRR
Residues around 5 Å of NIC7w
L632
E693
E637
E638
Q636
M645
2.7
E695
K694
R697
D639
P707
E743
N720
R772
H698
S721
D745
W774
H722

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020249667 A1
- CN 106963767 A

### Non-patent literature cited in the description


- **M. R. DE ZOETE et al.** *Cold Spring Harb Perspect Biol*, 2014, vol. 6, a016287
- **H. GUO et al.** *Nat Med*, 2015, vol. 21, 677-687
- **M. LAMKANFI** ; **V. M. DIXIT**. *Cell*, 2014, vol. 157, 1013-1022
- **K. ZHOU et al.** *J Immunol Res*, 2016, 9238290
- **K. SCHRODER** ; **J. TSCHOPP**. *Cell*, 2010, vol. 140, 821-832
- **V. SAGULENKO et al.** *Cell Death Differ*, 2013, vol. 20, 1149-1160
- **J. SHI et al.** *Nature*, 2014, vol. 514, 187-192
- **Y. HE et al.** *Nature*, 2016, vol. 530, 354-357
- **T. STROWIG et al.** *Nature*, 2012, vol. 481, 278-286
- **M. T. HENEKA et al.** *Nature*, 2013, vol. 493, 674-678
- **C. A. DINARELLO** ; **J. W. VAN DER MEER**. *Semin Immunol*, 2013, vol. 25, 469-484
- **E. ISAKOV** ; **P. WEISMAN-SHOMER** ; **M. BENHAR**. *Biochim Biophys Acta*, 2014, vol. 1840, 3153-3161
- **R. C. COLL et al.** *Nat Med*, 2015, vol. 21, 248-255
- **J. YIN et al.** *Mol Neurobiol*, 2018, vol. 55, 1977-1987
- **R. KUWAR et al.** *J Neuroinflammation*, 2019, vol. 16, 81
- **G. SLIWOSKI et al.** *Pharmacol Rev*, 2014, vol. 66, 334-395
- **H. SHARIF et al.** *Nature*, 2019, vol. 570, 338-343
- **M. SARESELLA et al.** *Mol Neurodegener*, 2016, vol. 11, 23
- **L. GAO et al.** *Inflamm Res*, 2017, vol. 66, 17-24
- **H. D. LIU et al.** *Neurochem Res*, 2013, vol. 38, 2072-2083
- **A. GUSTIN et al.** *PLoS One*, 2015, vol. 10, e0130624
- **RYAN W. GRANT** ; **VISHWA D. DIXIT**. *Front Immunol.*, 2013, vol. 4, 50
- **PETER DUEWELL et al.** *Nature*, 29 April 2010, vol. 464 (7293), 1357-61
- **EMILY L.** Goldberg and Vishwa Deep Dixit. *Immunol Rev.*, May 2015, vol. 265 (1), 63-74
- *Journal of Rheumatic Disease*, May 2018, vol. 21 (5)
- **S. SEINO**. *Diabetologia*, 2012, vol. 55, 2096-2108
- **J. J. IRWIN et al.** *J Chem Inf Model*, 2012, vol. 52, 1757-1768
- **H. WEN et al.** *Immunity*, 2013, vol. 39, 432-441
- **J. P. DE RIVERO VACCARI et al.** *J Cereb Blood Flow Metab*, 2014, vol. 34, 369-375
- **R. H. PIRZADA et al.** *Genes (Basel)*, 2020, vol. 11
- **N. J. BROADBENT et al.** *Proc Natl Acad Sci USA*, 2004, vol. 101, 14515-14520
- **L. E. CHOVAN et al.** *Rapid Commun Mass Spectrom*, 2004, vol. 18, 3105-3112
- **S. A. TESTINO, JR.** ; **G. PATONAY**. *J Pharm Biomed Anal*, 2003, vol. 30, 1459-1467